# EUROPÄISCHE PATENTSCHRIFT

(11) **EP 2 534 167 B1**
(45) Veröffentlichungstag und Bekanntmachung des Hinweises auf die Patenterteilung: **19.11.2014**
(21) Anmeldenummer: 11704431.3
(22) Anmeldetag: 08.02.2011
(51) Int. Cl.: C07J 41/00, C07J 43/00, A61P 5/34, A61K 31/567

(54) **PROGESTERONREZEPTORANTAGONISTEN**
PROGESTERONE RECEPTOR ANTAGONISTS
ANTAGONISTES DU RÉCEPTEUR DE LA PROGESTÉRONE

(30) Priorität: 10.02.2010 DE 102010007719
(43) Veröffentlichungstag der Anmeldung: 19.12.2012
(73) Patentinhaber: Bayer Intellectual Property GmbH, 40789 Monheim (DE)
(72) Erfinder: SCHWEDE, Wolfgang, 16548 Glienicke (DE); KLAR, Ulrich, 13503 Berlin (DE); MÖLLER, Carsten, 10115 Berlin (DE); ROTGERI, Andrea, 13053 Berlin (DE); BONE, Wilhelm, 13467 Berlin (DE); HUWE, Christoph, 13503 Berlin (DE)
(86) Internationale Anmeldenummer: PCT/EP2011/051780
(87) Internationale Veröffentlichungsnummer: WO 2011/098436

(56) Entgegenhaltungen:
- FUHRMANN U ET AL: "SYNTHESIS AND BIOLOGICAL ACTIVITY OF A NOVEL, HIGHLY POTENT PROGESTERONE RECEPTOR ANTAGONIST", JOURNAL OF MEDICINAL CHEMISTRY, AMERICAN CHEMICAL SOCIETY, US, Bd. 43, Nr. 26, 1. Januar 2000 (2000-01-01), Seiten 5010-5016, XP001064233, ISSN: 0022-2623, DOI: 10.1021/JM001000C in der Anmeldung erwähnt

## Beschreibung

Die Erfindung betrifft 17-Hydroxy-17-pentafluorethyl-estra-4,9(10)-dien-11-aryl-Derivate der Formel I mit Progesteron antagonisierender Wirkung und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Uterusfibroiden (Myomen, uterine Leiomyome), der Endometriose, von schweren Menstruationsblutungen, Meningiomen, hormonabhängigen Mammakarzinomen und von mit der Menopause assoziierten Beschwerden oder zur Fertilitätskontrolle und Notfallkontrazeption.

Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden. Zur Behandlung von Uterusfibroiden und der Endometriose können die erfindungsgemäßen Verbindungen auch sequentiell in Kombination mit Gestagenen verabreicht werden. In einem solchen Behandlungsregime könnten die erfindungsgemäßen Verbindungen über einen Zeitraum von 1 - 6 Monaten gegeben werden, gefolgt von einer Behandlungspause oder einer sequentiellen Behandlung mit einem Gestagen über einen Zeitraum von 2 - 6 Wochen oder gefolgt von der Behandlung mit einem oralen Kontrazeptivum (OC-Kombinationen) über den gleichen Zeitraum.

Die Wirksamkeit der erfindungsgemäßen Verbindungen als Progesteronrezeptorantagonist wurde in vitro in Transaktivierungstests und in vivo an der Ratte (Terminierung der frühen Schwangerschaft) gezeigt.

Verbindungen mit antagonistischer Wirkung am Progesteronrezeptor (kompetitive Progesteronrezeptorantagonisten) sind seit 1982 bekannt (RU 486; EP57115) und seither zahlreich beschrieben worden. Progesteronrezeptorantagonisten mit fluorierter 17α-Seitenkette wurden in WO 98/34947 und Fuhrmann et al., J. Med. Chem. 43, 5010 - 5016 (2000) veröffentlicht.

Die in WO 98/34947 beschriebenen Verbindungen mit fluorierter 17α-Seitenkette weisen im allgemeinen eine sehr starke antagonistische Aktivität am Progesteronrezeptor auf. Sehr potente und daher in WO 98/34947 bevorzugte Verbindungen sind 11β-(4-Acetylphenyl)-20,20,21,21,21-pentafluor-17-hydroxy-19-nor-17α-pregna-4,9-dien-3-on, 11β-(4-Acetylphenyl)-20,20,21,21,21-pentafluor-17-hydroxy-19-nor-17α-pregna-4-en-3-on und 6'-Acetyl-9,11β-dihydro-17β-hydroxy-17α-(1,1,2,2,2-pentafluorethyl)-4'H-naphth[3',2',1':10,9,11]ester-4-en-3-on. Diese Verbindungen werden in vivo in erheblichem Maße in verschiedene Metabolite überführt, die zum Teil starke, zum Teil geringere pharmakologische Aktivität aufweisen. Der Metabolismus tritt überwiegend am 4-Substituenten des 11-Phenylrestes auf. In WO 2008/058767 werden Verbindungen beschrieben, die zumindest zum Teil Metabolite der in WO 98/34947 beschriebenen Verbindungen sind.

Aufgabe der vorliegenden Erfindung ist es, hoch potente kompetitive Progesteronrezeptorantagonisten zur Verfügung zu stellen und damit alternative Behandlungsmöglichkeiten gynäkologischer Erkrankungen zu schaffen.

Die vorliegende Erfindung betrifft 17-Hydroxy-17-pentafluorethyl-estra-4,9(10)-dien-11-aryl-Derivate mit der allgemeinen chemischen Formel I: 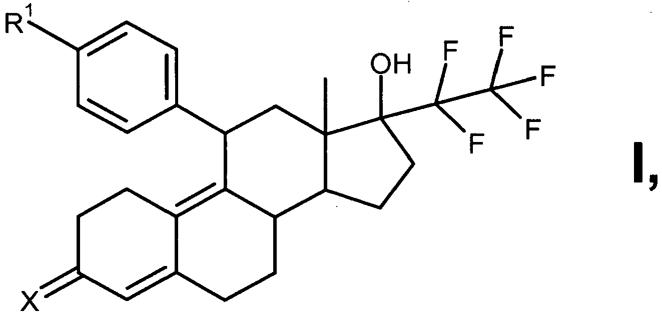 worin
- R¹: entweder für einen Rest Y oder für einen mit Y substituierten Phenylring steht,

- Y: für eine Gruppe 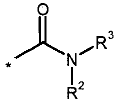 steht,
- R² und R³: gleich oder verschieden sind und für Wasserstoff,
einen gegebenenfalls -N(CH₃)₂, 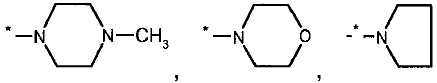 oder - NHC(O)CH₃ substituierten C₁-C₆-Alkylrest, einen 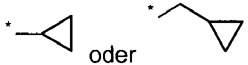 Rest
einen gegebenenfalls ein-, zwei- oder mehrfach mit Halogen (-F, -Cl, -Br, -I), -OH, -O-Alkyl, -C(O)OH, -C(O)OAlkyl, -C(O)NH₂, -C(O)NHAlkyl, -C(O)NDialkyl, -C(O)NHAryl, - C(O)NHHeteroaryl, -NH₂, -NH(C₁-C₆-Alkyl), -N(C₁-C₆-Alkyl)₂, insbesondere -N(CH₃)₂, - NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ -SO₂NHAlky oder -SO₂NDialky substituierten 6-10-gliedrigen Arylrest,
einen gegebenenfalls ein-, zwei- oder mehrfach mit den oben genannten Substituenten des 6-10-gliedrigen Arylrests substituierten 5-10-gliedrigen Heteroarylrest,
einen am Arylring gegebenenfalls ein-, zwei- oder mehrfach mit den oben genannten Substituenten des 6-10-gliedrigen Arylrests substituierten C₁-C₆-Arylalkylrest oder einen am Heteroarylring gegebenenfalls ein-, zwei- oder mehrfach mit den oben genannten Substituenten des 6-10-gliedrigen Arylrests substituierten C₁-C₆-Heteroarylalkylrest stehen
oder aber
- R² und R³: gemeinsam Bestandteil eines gegebenenfalls am Kohlenstoff Alkyl-, Carboxyl-, Alkylcarboxyl-, Alkylcarbonyl-, Aminocarbonyl-, Aryl-, insbesondere Phenyl-, Arylalkyl-, Heteroaryl-, Heteroarylalkyl-, Aminoalkyl- oder Dimethylaminoalkyl-substituierten bzw. am Stickstoff Alkyl-, Alkanoyl-, Alkylcarboxyl-, Carboxyl-, Aryl, insbesondere Phenyl-, Pyridinyl-, Pyrimidinyl-, Pyrazinyl-, Sulfonyl-, Benzoyl-, Alkylsulfonyl-, Arylsulfonyl-, Aminocarbonyl-, Dimethylaminocarbonyl-, Aminocarbonylalkyl, Alkylaminocarbonylalkyl, Arylalkyl-, insbesondere Phenylalkyl-, Heterocyclylalkyl-Heteroarylalkyl-, Aminoalkyl- oder 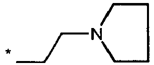 substituierten 3-10-gliedrigen Ringes sind, der gegebenenfalls Stickstoff-, Sauerstoff- oder Schwefelatome, die gegebenenfalls zum Sulfoxid oder Sulfon oxidiert sein können, enthält, wobei an den 3-10-gliedrigen Ring gegebenenfalls ein Aromat ankondensiert sein kann,
- X: ein Sauerstoffatom, NOR⁴ oder NNHSO₂R⁴ bedeutet und
- R⁴: ausgewählt ist aus der Gruppe, umfassend Wasserstoff, C₁-C₆-Alkyl und Aryl
und ihre Salze, Solvate oder Solvate der Salze, einschließlich aller Kristallmodifikationen (* symbolisiert die Anknüpfungsstelle am Grundkörper).

Die erfindungsgemäßen Verbindungen der allgemeinen Formel I können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.

Jeder der genannten Substituenten am Steroid-Grundgerüst kann sowohl in einer α- als auch in einer β-Stellung vorliegen.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen - wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen - wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldüsopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methylaminomethan und 1-Amino-2,3,4-butantriol.

Als Solvate werden im Rahmen der Erfindung solche Formen der erfindungsgemäßen Verbindungen bezeichnet, welche in festem oder flüssigem Zustand eine Adduktbildung mit Lösungsmittelmolekülen zeigen. Das Lösungsmittel kann dabei in einem stöchiometrischen oder auch unstöchiometrischen Verhältnis vorliegen. Bei stöchiometrischen Solvaten spricht man auch von Hemi-, (Semi-), Mono-, Sesqui-, Di-, Tri-, Tetra-, Penta-, usw. Solvaten. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche während ihrer Verweilzeit im Körper zu erfindungsgemäßen Verbindungen beispielsweise durch enzymatische oder hydrolytische Prozesse umgesetzt werden.

Im Rahmen der vorliegenden Erfindung haben die Substituenten, soweit nicht anders spezifiziert, die folgende Bedeutung:
Alkyl steht für eine gerad- oder verzweigtkettige Alkylgruppe mit 1 - 6 Kohlenstoffatomen, wie beispielsweise Methyl, Ethyl, Propyl, Isopropyl, n-Butyl, sec.-Butyl, Isobutyl, tert.-Butyl, n-Pentyl, Isopentyl, Neopentyl, Hexyl aber auch für Cyclopropyl oder Cyclopropylmethyl.
Aryl steht für einen mono- bis tricyclischen aromatischen, substituierten oder unsubstituierten carbocyclischen Rest, wie zum Beispiel Phenyl oder Naphthyl.

Der Arylrest kann ein-, zwei- oder mehrfach mit Halogen (-F, -Cl, -Br, -I), -OH, -O-Alkyl, - CO₂H, -CO₂-Alkyl, -C(O)NH₂, -C(O)NHAlkyl, -C(O)NDialkyl, -C(O)NHAryl, - C(O)NHHeteroaryl, -NH₂, -NH(C₁-C₆-Alkyl), -N(C₁-C₆-Alkyl)₂, insbesondere -N(CH₃)₂,-NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ -SO₂NHAlky oder -SO₂NDialkyl substituiert sein.

Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Benzofuranyl, Benzothiophenyl, Chinolinyl, Furyl, Imidazolyl, Indazolyl, Indolyl, Isochinolinyl Oxazolyl, Pyridazinyl, Pyridyl, Pyrimidyl, Pyrrolyl, Thiazolyl, Thienyl, Pyrazolyl, Isoxazolyl, Pyrazinyl, Chinolyl oder Tetrazolyl.

Der Heteroarylrest kann ein-, zwei- oder mehrfach mit Halogen (-F, -Cl, -Br, -I), -OH, -O-Alkyl, -CO₂H, -CO₂-Alkyl, -C(O)NH₂, -C(O)NHAlkyl, -C(O)NDialkyl, -C(O)NHAryl, - C(O)NHHeteroaryl, -NH₂, -NH(C₁-C₆-Alkyl), -N(C₁-C₆-Alkyl)₂, insbesondere -N(CH₃)₂,-NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ -SO₂NHAlky oder -SO₂NDialkyl substituiert sein.

Arylalkyl steht für Aralkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Aralkylreste kommen beispielsweise Benzyl, Phenylethyl, Naphthylmethyl, Naphthylethyl in Betracht.

Der Arylteil des Arylalkylrest kann ein-, zwei- oder mehrfach mit Halogen (-F, -Cl, -Br, -I), - OH, -O-Alkyl, -CO₂H, -CO₂-Alkyl, -C(O)NH₂, -C(O)NHAlkyl, -C(O)NDialkyl, -C(O)NHAryl, - C(O)NHHeteroaryl, -NH₂, -NH(C₁-C₆-Alkyl), -N(C₁-C₆-Alkyl)₂, insbesondere -N(CH₃)₂,-NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ -SO₂NHAlky oder -SO₂NDialkyl substituiert sein.

Heteroarylalkyl steht für Heteroarylalkylgruppen, wobei Heteroaryl die oben definierte Bedeutung hat und die in der Alkylkette 1-6, bevorzugt 1-4, Kohlenstoffatome enthalten können. Als Heteroarylalkylreste kommen beispielsweise Furylmethyl, Thienylethyl oder Pyridylpropyl in Betracht.
Der Heteroarylteil des Heteroarylalkylrests kann ein-, zwei- oder mehrfach mit Halogen (-F, - Cl, -Br, -I), -OH, -O-Alkyl, -CO₂H, -CO₂-Alkyl, -C(O)NH₂, -C(O)NHAlkyl, -C(O)NDialkyl, - C(O)NHAryl, -C(O)NHHeteroaryl, -NH₂, -NH(C₁-C₆-Alkyl), -N(C₁-C₆-Alkyl)₂, insbesondere - N(CH₃)₂, -NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ oder -SO₂NHalkyl, -SO₂NDialky substituiert sein.
Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Für Verbindungen, in denen R² und R³ gemeinsam Bestandteil eines gegebenenfalls substituierten Ringes sind, kann dieser Ring 3-10-gliedrig sein und neben dem vorhandenen Stickstoffatom nur Kohlenstoffatome oder aber bis zu 2 weitere Heteroatome tragen. Als weitere Heteroatome sind besonders, gegebenenfalls substituierter Stickstoff, Sauerstoff und gegebenenfalls oxidierter Schwefel zu nennen. Als Substituenten am Kohlenstoff kommen Alkyl-, Carboxyl-, Alkylcarboxyl-, Alkylcarbonyl-, Aminocarbonyl-, Aryl-, insbesondere Phenyl-, Arylalkyl-, Heteroaryl-, Heteroarylalkyl-, Aminoalkyl- oder Dimethylaminocarbonylgruppen in Betracht. Als Substituenten am Stickstoff kommen besonders Alkyl-, Alkanoyl-, Alkylcarboxyl-, Carboxyl-, Aryl-, insbesondere Phenyl-, Pyridinyl-, Pyrimidinyl-, Pyrazinyl-, Sulfonyl-, Benzyl-, Alkylsulfonyl-, Arylsulfonyl-, Aminocarbonyl-, Dimethylaminocarbonyl-, Aminocarbonylalkyl-, Alkylaminocarbonylalkyl, Arylalkyl-, insbesondere Phenylalkyl-, Heterocyclylalkyl-, Heteroarylalkyl-, Aminoalkyl oder 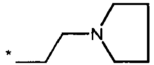 Gruppen in Frage. Schwefelatome im Ring können zum Sulfoxid oder Sulfon oxidiert sein. An den 3-10-gliedrigen Ring kann gegebenenfalls ein Aromat ankondensiert sein.

Als Ringe, die von R² und R³ gemeinsam gebildet werden sind besonders Piperidine, Piperazine, Morpholine, Diazepane, Thiomorpholine, Dioxidothiomorpholine, Tetrahydropyrrole zu nennen.

Heterocyclyl im Sinne der Erfindung ist ein nicht aromatisches mono- oder bicyclisches Ringsystem mit mindestens einem Heteroatom oder einer Heterogruppe. Als Heteroatome können Stickstoffatome, Sauerstoffatome und/oder Schwefelatome vorkommen. Als Heterogruppen können -S(O)-, -S(O)₂- vorkommen.

Heterocyclylalkyl steht für Heterocyclylalkylgruppen, wobei Heterocyclyl die oben definierte Bedeutung hat und die in der Alkylkette 1-6, bevorzugt 1-4, Kohlenstoffatome enthalten können. Als Heterocyclylalkylreste kommen beispielsweise Pyrrolidinoethyl in Betracht.

Ein monocyclischer Heterocyclylring gemäß der vorliegenden Erfindung kann 3 bis 8, bevorzugt 5 bis 8, besonders bevorzugt 5 oder 6 Ringatome aufweisen. Beispielhaft für monocyclische Heterocyclylreste mit 5 Ringatomen seien genannt: Pyrrolidinyl, Imidazolidinyl, Pyrazolidinyl, Pyrrolinyl und Tetrahydrofuranyl. Beispielhaft für monocyclische Heterocyclylreste mit 6 Ringatomen seien genannt: Piperidinyl, Piperazinyl, Morpholinyl, Tetrahydropyranyl und Thiomorpholinyl.

Ein bicyclischer Heterocyclylrest gemäß der vorliegenden Erfindung kann 5 bis 12, bevorzugt 8 bis 10 Ringatome aufweisen. Bevorzugt sind 5- bis 8-gliedrige, monocyclische gesättigte Heterocyclylreste mit bis zu zwei Heteroatomen aus der Reihe O, N und S. Besonders bevorzugt sind Morpholinyl, Piperidinyl und Pyrolidinyl.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind Kombinationen von zwei oder mehreren der oben genannten Vorzugsbereiche.

Besonders bevorzugt sind ebenfalls folgende unter die allgemeiner Formel I fallende Verbindungen der allgemeinen Formel V: 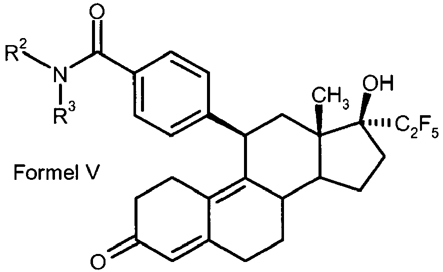 worin
- R²: Wasserstoff, C₁-C₄-Alkyl (insbesondere Methyl, Ethyl, Cyclopropyl oder Cyclopropylmethyl), -(CH₂)ₖ-N(CH₃)₂ mit k = 2 oder 3, -CH₂-CH₂-NH-CO-CH₃ und-(CH₂)ₖ-R⁵ mit k = 2 oder 3 und 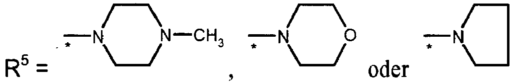 und
- R³: Wasserstoff oder C₁-C₄-Alkyl (insbesondere Methyl oder Ethyl) bedeutet
und ihre Salze, Solvate oder Solvate der Salze, einschließlich aller Kristallmodifikationen, insbesondere die Verbindungen:
4-[((11β,17β)-17-Hydroxy-3-oxo-17-(pentafluorethyl)estra-4,9-dien-11-yl]benzamid (Bsp. 1),
4-[((11β,17β)-17-Hydroxy-3-oxo-17-(pentafluorethyl)estra-4,9-dien-11-yl]-N,N-dimethylbenzamid (Bsp. 2),
N-[2-(Dimethylamino)ethyl]-4-[(11β,17β)-17-hydroxy-3-oxo-17-(pentafluorethyl)estra-4,9-dien-11-yl]benzamid (Bsp. 7),
N-[3-(Dimethylamino)propyl]-4-[(11β,17β)-17-hydroxy-3-oxo-17-(pentafluorethyl)estra-4,9-dien-11-yl]benzamid (Bsp. 8),
N-[2-(Dimethylamino)ethyl]-4-[(11β,17β)-17-hydroxy-3-oxo-17-(pentafluorethyl)estra-4,9-dien-11-yl]-N-methylbenzamid (Bsp. 9),
4-[((11β,17β)-17-Hydroxy-3-oxo-17-(pentafluorethyl)estra-4,9-dien-11-yl]-N-[3-(morpholin-4-yl)propyl]benzamid (Bsp. 13),
N-Ethyl-4-[(11β,17β)-17-hydroxy-3-oxo-17-(pentafluorethyl)estra-4,9-dien-11-yl]benzamid (Bsp. 19),
N,N-Diethyl-4-[(11β,17β)-17-hydroxy-3-oxo-17-(pentafluorethyl)estra-4,9-dien-11-yl]benzamid (Bsp. 27),
N-(2-Acetamidoethyl)-4-[(11β,17β)-17-hydroxy-3-oxo-17-(pentafluorethyl)estra-4,9-dien-11-yl]benzamid (Bsp. 37),
N-Cyclopropyl-4-[(11β,17β)-17-hydroxy-3-oxo-17-(pentafluorethyl)estra-4,9-dien-11-yl]benzamid (Bsp. 42),
4-[((11β,17β)-17-Hydroxy-3-oxo-17-(pentafluorethyl)estra-4,9-dien-11-yl]-N-[2-(pyrrolidin-1-yl)ethyl]benzamid (Bsp. 54),
N-(Cyclopropylmethyl)-4-[(11β,17β)-17-hydroxy-3-oxo-17-(pentafluorethyl)estra-4,9-dien-11-yl]benzamid (Bsp. 59) oder
4-[((11β,17β)-17-Hydroxy-3-oxo-17-(pentafluorethyl)estra-4,9-dien-11-yl]-N-[2-(4-methylpiperazin-1-yl)ethyl]benzamid (Bsp. 62).

Besonders bevorzugt sind ebenfalls folgende unter die allgemeine Formel I fallende Verbindungen der allgemeinen Formel VI: 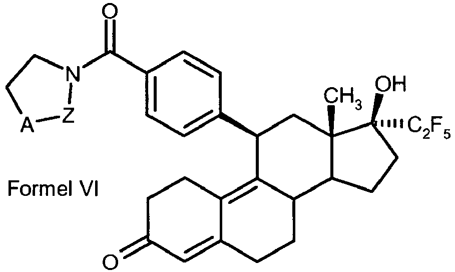 worin
- Z: -CH₂- oder -CH₂-CH₂-,
- A: Sauerstoff, -CHR⁶- oder -NR⁷- und
- R⁶, R⁷: Wasserstoff,
C₁-C₄-Alkyl,
einen gegebenenfalls ein-, zwei- oder mehrfach mit Halogen (-F, -Cl, -Br, -I), -OH, - O-Alkyl, -CO₂H, -CO₂-Alkyl, -C(O)NH₂, -C(O)NHAlkyl, -C(O)NDialkyl, -C(O)NHAryl, -C(O)NHHeteroaryl, -NH₂, -NH(C₁-C₆-Alkyl), -N(C₁-C₆-Alkyl)₂, insbesondere-N(CH₃)₂, -NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ -SO₂NHAlky oder -SO₂NDialky substituierten-(CH₂)_{I}-Arylrest mit I = 0, 1 oder 2,
einen gegebenenfalls ein-, zwei- oder mehrfach mit Halogen (-F, -Cl, -Br, -I), -OH, - O-Alkyl, -CO₂H, -CO₂-Alkyl, -C(O)NH₂, -C(O)NHAlkyl, -C(O)NDialkyl, -C(O)NHAryl, -C(O)NHHeteroaryl, -NH₂, -NH(C₁-C₆-Alkyl), -N(C₁-C₆-Alkyl)₂, insbesondere-N(CH₃)₂, -NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂ -SO₂NHAlky oder -SO₂NDialky substituierten-(CH₂)_{I}-Heteroarylrest mit bis zu zwei Heteroatomen und I = 0, 1 oder 2 oder
-COR⁸ und
- R⁸: -OH, -(C₁-C₄-Alkyl), -Aryl, -O-C₁-C₄-Alkyl, -NH-(C₁-C₄-Alkyl), -N(CH₃)₂, -SO₂-(C₁-C₄-Alkyl) bedeuten und ihre Salze, Solvate oder Solvate der Salze, einschließlich aller Kristallmodifikationen.

Ebenfalls besonders bevorzugt sind die unter die Formel VI fallende Verbindungen wobei:
- R⁶: Wasserstoff, Phenyl, -CO₂H, -CO₂CH₃ und
- R⁷: Wasserstoff, -CH₃, -(CH₂)ₗ-Phenyl mit I = 0, 1 oder 2, 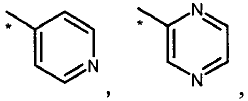 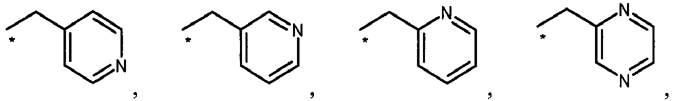 -CH₂-CH₂-N(CH₃)₂, 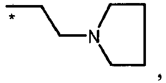 -CO-CH₃, -CO-Phenyl, -CO₂CH₃, -CO₂C(CH₃)₃ -CO-NH-CH₃, -CO-N(CH₃)₂; -SO₂-CH₃, -CH₂CO-NH-CH₃ bedeutet,
insbesondere die Verbindungen:
(11β,17β)-17-Hydroxy-11-{4-[(4-methylpiperazin-1-yl)carbonyl]phenyl}-17-(pentafluorethyl)estra-4,9-dien-3-on (Bsp. 3),
tert-Butyl-4-{4-[(11β,17β)-17-hydroxy-3-oxo-17-(pentafluorethyl)estra-4,9-dien-11-yl]benzoyl}piperazin-1-carboxylat (Bsp. 4),
(11β,17β)-17-Hydroxy-17-(pentafluorethyl)-11-[4-(piperidin-1-ylcarbonyl)phenyl]estra-4,9-dien-3-on (Bsp. 5),
Methyl-1-{4-[(11β,17β)-17-hydroxy-3-oxo-17-(pentafluorethyl)estra-4,9-dien-11-yl]benzoyl}piperidin-4-carboxylat (Bsp. 6),
(11β,17β)-17-Hydroxy-17-(pentafluorethyl)-11-[4-(pyrrolidin-1-ylcarbonyl)phenyl]estra-4,9-dien-3-on (Bsp. 15)
(11β,17β)-17-Hydroxy-11-[4-(morpholin-4-ylcarbonyl)phenyl]-17-(pentafluorethyl)estra-4,9-dien-3-on (Bsp. 29),
(11β,17β)-17-Hydroxy-17-(pentafluorethyl)-11-{4-[(4-phenylpiperazin-1-yl)carbonyl]phenyl}estra-4,9-dien-3-on (Bsp. 30),
(11β,17β)-11-{4-[(4-Benzylpiperazin-1-yl)carbonyl]phenyl}-17-hydroxy-17-(pentafluorethyl)estra-4,9-dien-3-on (Bsp. 40),
(11β,17β)-17-Hydroxy-17-(pentafluorethyl)-11-(4-{[4-(2-phenylethyl)piperazin-1-yl]carbonyl}phenyl)estra-4,9-dien-3-on (Bsp. 44),
(11β,17β)-17-Hydroxy-17-(pentafluorethyl)-11-(4-{[4-(pyridin-4-yl)piperazin-1-yl]carbonyl}phenyl)estra-4,9-dien-3-on (Bsp. 45),
(11β,17β)-17-Hydroxy-17-(pentafluorethyl)-11-(4-{[4-(pyrazin-2-yl)piperazin-1-yl]carbonyl}phenyl)estra-4,9-dien-3-on (Bsp. 46),
(11β,17β)-11-[4-({4-[2-(Dimethylamino)ethyl]piperazin-1-yl}carbonyl)phenyl]-17-hydroxy-17-(pentafluorethyl)estra-4,9-dien-3-on (Bsp. 47),
(11β,17β)-17-Hydroxy-17-(pentafluorethyl)-11-[4-({4-[2-(pyrrolidin-1-yl)ethyl]piperazin-1-yl}carbonyl)phenyl]estra-4,9-dien-3-on (Bsp. 48),
(11β,17β)-17-Hydroxy-17-(pentafluorethyl)-11-(4-{[4-(pyridin-4-ylmethyl)piperazin-1-yl]carbonyl}phenyl)estra-4,9-dien-3-on (Bsp. 49),
(11β,17β)-17-Hydroxy-17-(pentafluorethyl)-11-{4-[(4-phenylpiperidin-1-yl)carbonyl]phenyl}estra-4,9-dien-3-on (Bsp. 63),
(11β,17β)-11-{4-[(4-Benzoylpiperazin-1-yl)carbonyl]phenyl}-17-hydroxy-17-(pentafluorethyl)estra-4,9-dien-3-on (Bsp. 65),
4-{4-[(11β,17β)-17-Hydroxy-3-oxo-17-(pentafluorethyl)estra-4,9-dien-11-yl]benzoyl}-N,N-dimethylpiperazin-1-carboxamid (Bsp. 66),
(11β,17β)-17-Hydroxy-11-(4-{[4-(methylsulfonyl)piperazin-1-yl]carbonyl}phenyl)-17-(pentafluorethyl)estra-4,9-dien-3-on (Bsp. 67),
(11β,17β)-17-Hydroxy-17-(pentafluorethyl)-11-(4-{[4-(pyridin-2-ylmethyl)piperazin-1-yl]carbonyl}phenyl)estra-4,9-dien-3-on (Bsp. 68),
(11β,17β)-17-Hydroxy-17-(pentafluorethyl)-11-(4-{[4-(pyridin-3-ylmethyl)piperazin-1-yl]carbonyl}phenyl)estra-4,9-dien-3-on (Bsp. 69),
Methyl-4-{4-[(11β,17β)-17-hydroxy-3-oxo-17-(pentafluorethyl)estra-4,9-dien-11-yl]benzoyl}piperazin-1-carboxylat (Bsp. 70),
2-((4-{4-[(11β,17β)-17-Hydroxy-3-oxo-17-(pentafluorethyl)estra-4,9-dien-11-yl]benzoyl}piperazin-1-yl)-N-methylacetamid (Bsp. 71),
(11β,17β)-17-Hydroxy-17-(pentafluorethyl)-11-[4-(piperazin-1-ylcarbonyl)phenyl]estra-4,9-dien-3-on (Bsp. 73) und
(11β,17β)-11-{4-[(4-Acetylpiperazin-1-yl)carbonyl]phenyl}-17-hydroxy-17-(pentafluorethyl)estra-4,9-dien-3-on (Bsp. 74).
1-{4-[(11β,17β)-17-Hydroxy-3-oxo-17-(pentafluorethyl)estra-4,9-dien-11-yl]benzoyl}piperidin-4-carbonsäure (Bsp. 75).

Besonders bevorzugt sind ebenfalls folgende unter die allgemeine Formel I fallende Verbindungen der allgemeinen Formel VII: 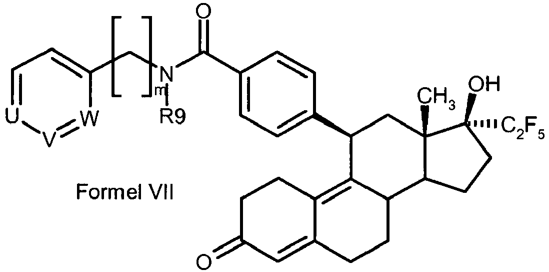 worin
- m: 0, 1 oder 2,
- R⁹: Wasserstoff oder -C₁-C₄-Alkyl, insbesondere Methyl oder Ethyl,
- U, V und W: unabhängig voneinander -CH=, -CR¹⁰= oder -N= bedeuten und -CR¹⁰= oder -N= unabhängig von der Postition im aromatischen Ring jedoch höchstens einmal vorkommen und
- R¹⁰: -O-(C₁-C₄-Alkyl), Halogen, -COR¹¹ mit R¹¹ =-OH, -NH₂ oder -O-(C₁-C₄-Alkyl), -SO₂-NH₂; -NH-CO-(C₁-C₄-Alkyl), oder -CO-NH-Aryl bedeutet.

Ebenfalls besonders bevorzugt sind die unter die Formel VI fallende Verbindungen wobei:
- R⁹: Wasserstoff, Methyl oder Ethyl und
- R¹⁰: -O-CH₃, -Cl, -CO₂H, -CO₂CH₃, -CO-NH₂, -SO₂-NH₂; -NH-CO-CH₃ oder-CO-NH-Phenyl bedeutet
und ihre Salze, Solvate oder Solvate der Salze, einschließlich aller Kristallmodifikationen,
insbesondere die Verbindungen:
Methyl-2-({4-[(11β,17β)-17-hydroxy-3-oxo-17-(pentafluorethyl)estra-4,9-dien-11-yl]benzoyl}amino)benzoat (Bsp. 10)
4-[((11β,17β)-17-Hydroxy-3-oxo-17-(pentafluorethyl)estra-4,9-dien-11-yl]-N-(pyridin-2-yl)benzamid (Bsp. 11)
4-[((11β,17β)-17-Hydroxy-3-oxo-17-(pentafluorethyl)estra-4,9-dien-11-yl]-N-(pyridin-3-yl)benzamid (Bsp. 12)
4-[((11β,17β)-17-Hydroxy-3-oxo-17-(pentafluorethyl)estra-4,9-dien-11-yl]-N-(pyridin-4-yl)benzamid (Bsp. 14)
4-[((11β,17β)-17-Hydroxy-3-oxo-17-(pentafluorethyl)estra-4,9-dien-11-yl]-N-(2-phenylethyl)benzamid (Bsp. 16)
4-[((11β,17β)-17-Hydroxy-3-oxo-17-(pentafluorethyl)estra-4,9-dien-11-yl]-N-(4-sulfamoylbenzyl)benzamid (Bsp. 18)
4-[((11β,17β)-17-Hydroxy-3-oxo-17-(pentafluorethyl)estra-4,9-dien-11-yl]-N-(pyridin-2-ylmethyl)benzamid (Bsp. 20)
4-[((11β,17β)-17-Hydroxy-3-oxo-17-(pentafluorethyl)estra-4,9-dien-11-yl]-N-(pyridin-4-ylmethyl)benzamid (Bsp. 21)
4-[((11β,17β)-17-Hydroxy-3-oxo-17-(pentafluorethyl)estra-4,9-dien-11-yl]-N-phenylbenzamid (Bsp. 22)
4-[((11β,17β)-17-Hydroxy-3-oxo-17-(pentafluorethyl)estra-4,9-dien-11-yl]-N-(2-methoxyphenyl)benzamid (Bsp. 23)
4-[((11β,17β)-17-Hydroxy-3-oxo-17-(pentafluorethyl)estra-4,9-dien-11-yl]-N-(3-methoxyphenyl)benzamid (Bsp. 24)
4-[((11β,17β)-17-Hydroxy-3-oxo-17-(pentafluorethyl)estra-4,9-dien-11-yl]-N-(4-methoxyphenyl)benzamid (Bsp. 25)
N-(4-Chlorphenyl)-4-[(11β,17β)-17-hydroxy-3-oxo-17-(pentafluorethyl)estra-4,9-dien-11-yl]benzamid (Bsp. 26)
4-[((11β,17β)-17-Hydroxy-3-oxo-17-(pentafluorethyl)estra-4,9-dien-11-yl]-N-methyl-N-phenylbenzamid (Bsp. 28)
4-[((11β,17β)-17-Hydroxy-3-oxo-17-(pentafluorethyl)estra-4,9-dien-11-yl]-N-methyl-N-(pyridin-2-ylmethyl)benzamid (Bsp. 31)
4-[((11β,17β)-17-Hydroxy-3-oxo-17-(pentafluorethyl)estra-4,9-dien-11-yl]-N-methyl-N-(pyridin-3-ylmethyl)benzamid (Bsp. 32)
4-[((11β,17β)-17-Hydroxy-3-oxo-17-(pentafluorethyl)estra-4,9-dien-11-yl]-N-methyl-N-(pyridin-4-ylmethyl)benzamid (Bsp. 33)
N-Benzyl-4-[(11β,17β)-17-hydroxy-3-oxo-17-(pentafluorethyl)estra-4,9-dien-11-yl]benzamid (Bsp. 34)
4-[((11β,17β)-17-Hydroxy-3-oxo-17-(pentafluorethyl)estra-4,9-dien-11-yl]-N-(4-methoxybenzyl)benzamid (Bsp. 35)
N-(4-Chlorbenzyl)-4-[(11β,17β)-17-hydroxy-3-oxo-17-(pentafluorethyl)estra-4,9-dien-11-yl]benzamid (Bsp. 36)
Methyl-4-({4-[(11β,17β)-17-hydroxy-3-oxo-17-(pentafluorethyl)estra-4,9-dien-11-yl]benzoyl}amino)benzoate (Bsp. 38)
N-(4-Carbamoylphenyl)-4-[(11β,17β)-17-hydroxy-3-oxo-17-(pentafluorethyl)estra-4,9-dien-11-yl]benzamid (Bsp. 41)
4-[((11β,17β)-17-Hydroxy-3-oxo-17-(pentafluorethyl)estra-4,9-dien-11-yl]-N-methyl-N-(2-phenylethyl)benzamid (Bsp. 43)
4-[((11β,17β)-17-Hydroxy-3-oxo-17-(pentafluorethyl)estra-4,9-dien-11-yl]-N-methyl-N-[2-(pyridin-2-yl)ethyl]benzamid (Bsp. 50)
N-Benzyl-4-[(11β,17β)-17-hydroxy-3-oxo-17-(pentafluorethyl)estra-4,9-dien-11-yl]-N-methylbenzamid (Bsp. 51)
N-(4-Acetamidophenyl)-4-[(11β,17β)-17-hydroxy-3-oxo-17-(pentafluorethyl)estra-4,9-dien-11-yl]benzamid (Bsp. 52)
4-[((11β,17β)-17-Hydroxy-3-oxo-17-(pentafluorethyl)estra-4,9-dien-11-yl]-N-(3-methoxybenzyl)benzamid (Bsp. 53)
4-[((11β,17β)-17-Hydroxy-3-oxo-17-(pentafluorethyl)estra-4,9-dien-11-yl]-N-[2-(pyridin-3-yl)ethyl]benzamid (Bsp. 55)
N-(3-Chlorbenzyl)-4-[(11β,17β)-17-hydroxy-3-oxo-17-(pentafluorethyl)estra-4,9-dien-11-yl]benzamid (Bsp. 56)
Methyl-3-({4-[(11β,17β)-17-hydroxy-3-oxo-17-(pentafluorethyl)estra-4,9-dien-11-yl]benzoyl}amino)benzoate (Bsp. 57)
4-[((11β,17β)-17-Hydroxy-3-oxo-17-(pentafluorethyl)estra-4,9-dien-11-yl]-N-methyl-N-[2-(pyridin-4-yl)ethyl]benzamid (Bsp. 58)
4-[((11β,17β)-17-Hydroxy-3-oxo-17-(pentafluorethyl)estra-4,9-dien-11-yl]-N-[4-(phenylcarbamoyl)phenyl]benzamid (Bsp. 60)
4-[((11β,17β)-17-Hydroxy-3-oxo-17-(pentafluorethyl)estra-4,9-dien-11-yl]-N-[2-(pyridin-2-yl)ethyl]benzamid (Bsp. 61)
4-[((11β,17β)-17-Hydroxy-3-oxo-17-(pentafluorethyl)estra-4,9-dien-11-yl]-N-[2-(pyridin-4-yl)ethyl]benzamid (Bsp. 64)
4-[((11β,17β)-17-Hydroxy-3-oxo-17-(pentafluorethyl)estra-4,9-dien-11-yl]-N-methyl-N-[2-(pyridin-3-yl)ethyl]benzamid (Bsp. 72)
2-({4-[(11β,17β)-17-Hydroxy-3-oxo-17-(pentafluorethyl)estra-4,9-dien-11-yl]benzoyl}amino)benzoesäure (Bsp. 76)
3-({4-[(11β,17β)-17-Hydroxy-3-oxo-17-(pentafluorethyl)estra-4,9-dien-11-yl]benzoyl}amino)benzoesäure (Bsp. 77)
4-({4-[(11β,17β)-17-Hydroxy-3-oxo-17-(pentafluorethyl)estra-4,9-dien-11-yl]benzoyl}amino)benzoesäure (Bsp. 78)

Ebenfalls besonders bevorzugt sind die Verbindungen:
4-[((11β,17β)-17-Hydroxy-3-oxo-17-(pentafluorethyl)estra-4,9-dien-11-yl]-N-(2-phenylpropan-2-yl)benzamid (Bsp. 17),
(11β,17β)-11-[4-(3,4-Dihydroisochinolin-2(1H)-ylcarbonyl)phenyl]-17-hydroxy-17-(pentafluorethyl)estra-4,9-dien-3-on (Bsp. 39).

Es wurde gefunden, dass die erfindungsgemäßen Verbindungen bzw. Derivate eine gute, das Progesteron antagonisierende Wirkung aufweisen. In mehreren klinischen Studien wurde gefunden, dass die Behandlung mit Progestronrezeptorantagonisten (Mifepriston, Asoprisnil, Proellex) zu einer signifikanten Schrumpfung von Uterusfibroiden und einer signifikanten Reduktion der mit diesen Uterusfibroiden assoziierten Symptome führen kann. Ferner wurde in klinischen Studien gezeigt, dass unter einer Behandlung mit den genannten Progesteronrezeptorantagonisten auch die von Endometriose verursachten Symptome (insbesondere Schmerzen) deutlich reduziert werden können.

Die Verbindungen der allgemeinen Formel I sowie deren physiologisch verträglichen und pharmazeutisch akzeptablen Salze können nach dem Fachmann bekannten Verfahren formuliert werden, wobei orale, einmal täglich zu verabreichende Dosisformen bevorzugt sind.

Die Verbindungen der allgemeinen Formel I sowie deren physiologisch verträglichen und pharmazeutisch akzeptablen Salze können nach dem Fachmann bekannten Verfahren formuliert werden, wobei orale, einmal täglich zu verabreichende Dosisformen bevorzugt sind.

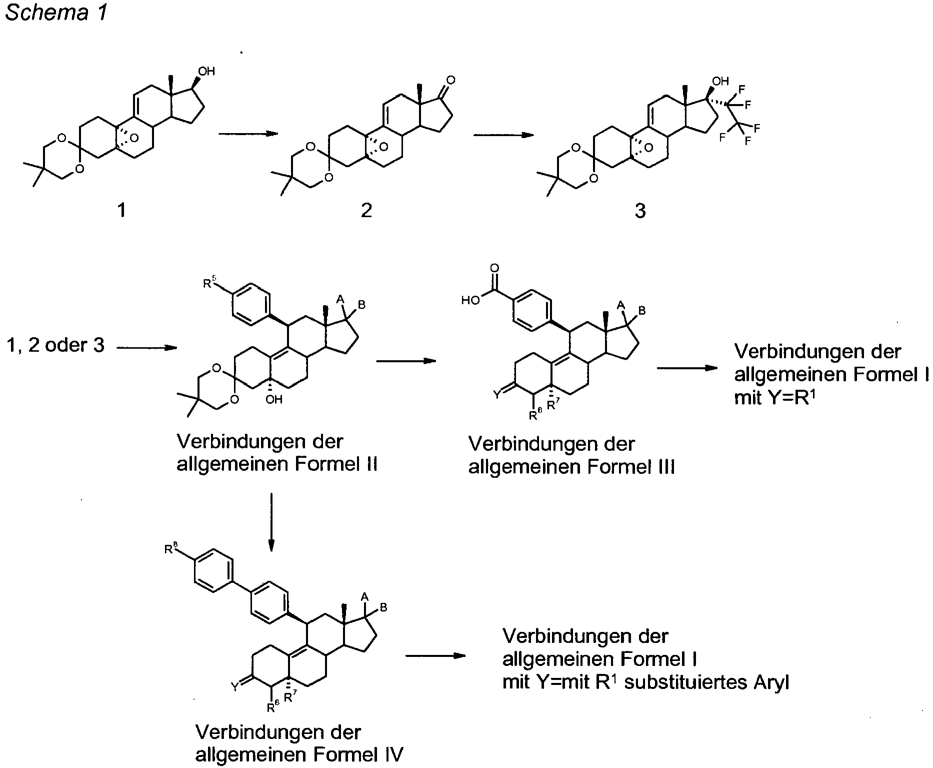

Eine Übersicht zur Herstellung von Verbindungen der allgemeinen Formel I ist in Schema 1 gezeigt. Die Darstellung der Verbindungen mit der allgemeinen chemischen Formel I erfolgt ausgehend von (5'R, 8'S, 10'R, 13'S, 14'S, 17'S)-5,5,13'-Trimethyl-1',2',7',8',12',13',14',15',16',17'-decahydro-6'H-spiro[1,3-dioxan-2,3'-[5,10]epoxycyclopenta-[a]phenanthren]-17'-ol (Verbindung 1, Schema 1) (Herstellung siehe Tetrahedron Lett. 26, 2069-2072 (1985) in Analogie zu den in WO 98/34947 und in WO 2008/058767 beschriebenen Verfahren. Nach Oxidation der Hydroxygruppe in Position 17 des Steroidgerüstes (Verbindung 2, Schema 1) erfolgt die Einführung der 17a-Pentafluorethylseitenkette an die entsprechenden 17-Ketoverbindungen gemäß den in WO 98/34947 und in WO 2008/058767 beschriebenen Verfahren (Verbindung 3, Schema 1). Die Einführung des 11β-Phenylsubstituenten erfolgt über konjugierte Addition von Arylgrignard- oder Aryllithiumreagenzien unter Kupfer-Katalyse. Generell können die Verbindungen 1, 2 oder 3 als Ausgangsmaterial zur Einführung des 11β-Phenylsubstituenten genutzt werden. Durch Einführung des 11β-Phenylsubstituenten werden Verbindungen der allgemeinen Formel II erhalten, in denen R⁵ alle bereits für R¹ genannten Bedeutungen haben kann und zusätzlich ein geschützter Aldehyd, eine Carboxy-, Alkylcarboxy-, Arylcarboxy, Aralkylcarboxy, Hydroxymethyl-, Alkoxymethyl., Benzyloxymethyl-, Alkanoyloxymethyl-, Silyloxymethyl-, Hydroxy-, C₁-C₆-Alkoxy-, Benzyloxy-, C₁-C₆-Alkanoyloxy-, Benzoyloxy-, Silyloxyl-, Alkoxyalkyloxygruppe ein Cl, Br, I oder eine Gruppierung CₘFₘ₊₁SO₃ mit m=1-4 sein kann und A bzw. B entweder für eine Carbonylgruppe stehen oder für eine 17β-OH/17α-H Gruppierung oder für eine 17β-OH/17α-C2F5 Gruppierung steht. Aus Verbindungen der allgemeinen Formel II können dann über mehrere Stufen die Verbindungen der allgemeinen Formel I erhalten werden. Hierzu werden funktionelle Gruppen gegebenenfalls weiter modifiziert. Hervorzuheben hierbei ist die Herstellung von Verbindungen der allgemeinen Formel I aus Verbindungen der allgemeinen Formel III durch dem Fachmann bekannte Methoden der Amidbildung. Für die Amidbildung kommen Methoden in Frage, bei denen die Carboxygruppe direkt durch Umsetzung mit einem Amin unter zu Hilfenahme von bekannten Reagenzien wie 2-(1H-Benzotriazol-1-yl)-1,1,3,3-tetramethyluronium-tetrafluoroborat (TBTU), 1-Hydroxy-benzotriazol (HOBT) oder 2-Propanphosphonsäureanhydrid in ein Amid überführt wird oder aber zunächst aus der freien Carbonsäure z.B. ein Säurechlorid gebildet wird, welches dann durch Reaktion mit den entsprechenden Aminen in ein Amid überführt wird. Verbindungen der allgemeinen Formel III werden aus Verbindungen der Formel II durch Oxidation von Aldehyden, die durch Spaltung vorhandener Schutzgruppen erhalten werden hergestellt oder durch ein- oder zweistufige Oxidation der Hydroxymethylverbindungen, die ebenfalls durch Abpaltung evtl vorhandener Alkoholschutzgruppen freigesetzt werden. Ausgehend von Verbindungen der allgemeinen Formel II, in denen R⁵ für einen geschützten Aldehyd steht, wird hierzu nach dem Fachmann bekannten Methoden die Aldehydschutzgruppe gespalten. Die Spaltung der Aldehydschutzgruppe kann unter Reaktionsbedingungen erfolgen, unter denen auch die Ketalschutzgruppe an Position 3 des Steroidgerüstes zum Keton gespalten wird, aber auch unter mild sauren Bedingungen unter denen die Schutzgruppe an der Position 3 erhalten bleibt. Ausgehend von Verbindungen der allgemeinen Formel II, in denen R⁵ für eine Gruppe geschütze Hydroxymethylgruppe steht, wird die Alkoholschutzgruppe gespalten, so dass eine freie Hydroxymethylgruppe ensteht. Alternativ hierzu kann die Amidbildung aber auch ausgehend von den Alkylcarboxy- oder Arylcarboxyverbindungen durch Umsetzung mit den entsprechenden Aminen in Gegenwart von Trimethylaluminium oder vergleichbaren Lewis-Säuren erfolgen. Verbindungen, in denen Y die Bedeutung eines mit R¹ substituierten Arylrestes hat (allgemeine Formel IV), werden entweder direkt durch konjugierte Addition des Diarylgrignard- oder Diaryllithiumreagenzes unter Kupfer-Katalyse oder aber z.B. über Palladium-katalysierte Kupplungsreaktionen an die entsprechend funktionalisierten 11β-Phenylderivate, z.B. Phenyltriflate oder Phenylnonaflate hergestellt. Bei der Einführung des zweiten Aromaten z.B. über Palladium-katalysierte Kupplungsreaktionen kann R⁸ bereits die Bedeutung von R¹ haben oder aber in der bereits vorher beschriebenen Weise z.B. aus Carboxygruppen oder Alkyl- bzw. Arylcarboxygruppen hergestellt werden. Zur Herstellung von Verbindungen der allgemeinen Formel I werden schließlich noch ggf vorhandene Schutzgruppen gespalten sowie die Reste R⁵ oder R⁸ weiter modifiziert. Für diese Modifikationen sind Oxidationen oder Reduktionen, Veresterungen, Verseifungen, Alkylierung, Acylierungen von freien Valenzen in R⁵ oder R⁸ zu nennen.

Als Ketalschutz- oder Acetalschutzgruppen sind beispielsweise die Ethylendioxy- oder die 2,2-Dimethylpropylen-1,2-dioxygruppe zu nennen. Hydroxygruppen werden beispielsweise in Form von Methoxymethyl, Methoxyethyl-, Tetrahydropyranyl-, Benzyl-, oder Silylethern geschützt.

Bei der Spaltung des 3-Ketals zur 3-Ketogruppe des Steroidgerüstes wird eine gegebenenfalls noch vorhandene 5α-Hydroxygruppe elimininiert, so dass Verbindungen der allgemeinen Formel I entstehen.

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung, in die einzelnen Verbindungen aufgetrennt werden. Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die resultierenden Verbindungen der Formel (I) werden gegebenenfalls mit den entsprechenden (i) Lösungsmitteln und/oder (ii) Basen oder Säuren zu ihren Solvaten, Salzen und/oder Solvaten der Salze umsetzt.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die erfindungsgemäßen Verbindungen zeigen ein nicht vorhersehbares, wertvolles pharmakologisches, pharmakokinetisches und pharmakodynamisches Wirkprofil.

Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen lässt sich durch ihre Wirkung als Progesteronrezeptorantagonisten, also ihre antagonisierende Wirkung am Progesteronrezeptor erklären.

Weiterer Gegenstand der vorliegenden Erfindung ist der Einsatz der erfindungsgemäßen Verbindungen zur Behandlung und/oder Prophylaxe von Erkrankungen basierend auf hormonabhängigen hyperproliferativen Prozessen, vorzugsweise von gynäkologischen Krankheiten, insbesondere von Uterusfibroiden, der Endometriose oder von hormonabhängigen Mammakarzinomen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von Uterusfibroiden, der Endometriose und von hormonabhängigen Mammakarzinomen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung von 0,1-100 mg der erfindungsgemäßen Verbindungen pro Tag und Patientin bei der Behandlung von Uterusfibroiden oder der Endometriose und für die kontrazeptive Anwendung bzw. von 0,1-500 mg der erfindungsgemäßen Verbindungen pro Tag und Patientin bei Tumor-Erkrankungen (z.B. Menginiome oder hormonabhängige Tumore wie z.B. das Mammakarzinom) und bei der Notfallkontrazeption.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika.

Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.

In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84tägige Gabe des Progesteronrezeptorantagonisten gefolgt von der 14tägigen Gabe des Gestagens.

Zur Behandlung von mit der Menopause assoziierten Beschwerden kommt eine gleichzeitige oder sequentielle Verabreichung der erfindungsgemäßen Verbindungen z.B. mit SERMs, SERDs und Östrogenen in Betracht.

SERMs (Selective Estrogen Receptor Modulators) sind solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen.

Selektive Estrogenrezeptordestabilisatoren (SERD) sind Arzneistoffe, die den Estrogenrezeptor vollständig antagonisieren (,reine Antiöstrogene' ohne östrogene Wirkkomponente) und zu einem Abbau des Rezeptors führen (beispielsweise Fulvestrant, ZK-703 und ZK-253 (Hoffmann J et al., J Natl Cancer Inst 2004, 96:210-218) sowie in WO 98/007740, WO 99/33855 und WO 03/045972 beschriebene Verbindungen.

Antiöstrogene sind Verbindungen die den Estrogenrezeptor vollständig antagonisieren, beispielsweise Fulvestrant.

Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole.

Kinaseinhibitoren sind Enzyme, die einen Phosphatrest von ATP auf andere Substrate, dort insbesondere auf Hydroxygruppen, übertragen, z.B. Sorafenib (Nexavar) oder Imatinib (Gleevec).

Angiogenesehemmer, z.B. Avastin, reduzieren bzw. blockieren die Gefäßversorgung und damit die Durchblutung eines Tumors.

Zytostatika, z.B. cis-Platin, Taxol, Taxotere sind natürliche oder synthetische Substanzen, die das Zellwachstum bzw. die Zellteilung hemmen.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.

Die erfindungsgemäßen Verbindungen können systemisch und/oder lokal wirken. Zu diesem Zweck können sie auf geeignete Weise, wie z.B. oral, intrauterin[är], intravaginal, parenteral, pulmonal, nasal, sublingual, lingual, buccal, rectal, dermal, transdermal, conjunctival, otisch oder als Implantat bzw. Stent appliziert werden.

Intrauterin[är] bedeutet dabei insbesondere die Applikation mittels IUS (intrauterine system) oder IUD (intrauterine device). Die intravaginale Applikation kann u.a. mittels IVR/VRS (Intra-Vaginalring/Vaginalringsystem) erfolgen.

Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nonsilikon- und/oder Silikonpolymere, insbesondere auch siloxanebasierte Elastomere (vergl. WO 01/47490, insbesondere Seite 7, Zeile 19 - Seite 15, Zeile 15), enthalten.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die folgenden Beispiele dienen der Erläuterung der Erfindung ohne diese in irgend einer Weise zu beschränken.

### Beispiel 1:

### 4-[(11β,17β)-17-Hydroxy-3-oxo-17-(pentafluorethyl)estra-4,9-dien-11-yl]benzamid

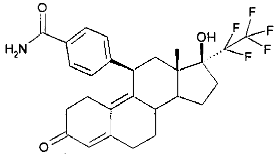

### a) (5'R,8'S,10'R,13'S,14'S,17'S)-5,5,13'-Trimethyl-17'-(pentafluorethyl)-1',2',7',8',12',13',14',15',16',17'-decahydro-6'H-spiro[1,3-dioxan-2,3'-[5,10]epoxycyclopenta[a]phenanthren]-17'-ol

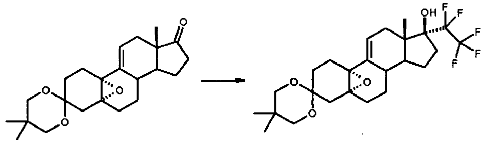

Zu 116 g kondensiertem Pentafluoriodethan in 500 ml absolutem Toluol wurden bei -70°C 50 g (5'R,8'S,10'R,13'S,14'S)-5,5,13'-Trimethyl-1',2',6',7',8',12',13',14',15',16'-decahydro-17'H-spiro[1,3-dioxan-2,3'-[5,10]epoxycyclopenta[a]phenanthren]-17'-on (Herstellung siehe Tetrahedron Lett. 26, 2069-2072 (1985) gegeben. Dazu gab man 290 ml einer 1,5 molaren Lösung von Methyllithium-Lithiumbromid-Komplex in Diethylether bei gleicher Temperatur. Anschließend wurde eine Stunde bei 0°C nachgerührt. Dann wurde das Reaktionsgemisch auf gesättigte wässrige Ammoniumchloridlösung gegeben und mit Ethylacetat extrahiert. Die organische Phase wurde mit gesättigter wässriger Natriumchloridlösung gewaschen, über Natriumsulfat getrocknet und im Vakuum eingeengt. Das Rohprodukt wurde in 200 ml Aceton aufgelöst und mit 450 ml Wasser versetzt. Das ausgefallene Produkt wurde abfiltriert und im Vakuum getrocknet.
Ausbeute 61,6 g
¹H-NMR (400 MHz, CDCl₃): δ= 6,04 brd (1 H); 3,60 d (1 H); 3,35-3,50 m (3H); 2,51 dbr (1 H); 1,06 s (3H); 0,93 s (3H); 0,85 s (3H).

### 1b) (5R,8S,11R,13S,14S,17S)-11-[4-({[tert-Butyl(dimethyl)silyl]oxy}methyl)phenyl]-5,5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

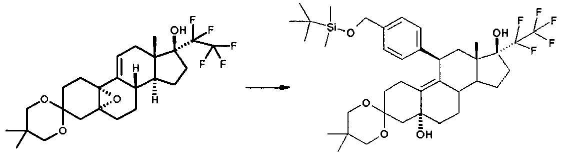

1,23 g Magnesium-Späne wurden in 5 ml Tetrahydrofuran suspendiert und unter Rühren mit 50 µl Dibromethan versetzt. Zur Suspension wurde eine Lösung von 15,29 g [(4-Brombenzyl)oxy]-(tert-butyl)dimethylsilan in 40 ml Tetrahydrofuran mit einer solchen Geschwindigket addiert, dass die Innentemperatur nicht über 60°C anstieg. Danach wurde für eine Stunde bei 23°C nachgerührt. Anschließend wurde die entstandene Lösung auf 0 °C gekühlt. Man addierte 151 mg Kupfer (I) chlorid und ließ weitere 15 Minuten bei 0°C nachrühren. Danach wurde eine Lösung von 5 g der unter Beispiel 1a) beschriebenen Substanz in 50 ml Tetrahydrofuran addiert. Dann ließ man das Reaktionsgemisch unter Rühren über ca. 3 Stunden auf 23°C kommen und rührte anschließend bei dieser Temperatur 10 Stunden nach. Anschließend wurde gesättigte wässrige Ammoniumchlorid-Lösung zum Reaktionsgemisch unter externer Kühlung addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 5,62 g der Titelverbindung.
¹H-NMR (300 MHz, CDCl₃): δ= 7,10-7,22 m (4H); 4,70 s (2H); 4,43 s (1H); 4,30 dbr (1H); 3,40-3,56 m (4H); 1,06 s (3H); 0,91 s (9H); 0,86 s (3H); 0,50 s (3H); 0,08 s (6H).

### c) (5R,8S,11R,13S,14S,17S)-11-[4-(Hydroxymethyl)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

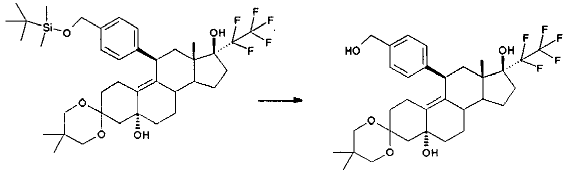

2,99 g der unter Beispiel 1b) beschriebenen Verbindung wurden in 25 ml Tetrahydrofuran gelöst. Man addierte 6,3 ml einer 1 molaren Lösung von Tetrabutylammoniumfluorid in Tetrahydrofuran und ließ danach 2,5 Stunden bei 23°C nachrühren. Anschließend wurde das Reaktionsgemisch auf gesättigte Natriumhydrogencarbonatlösung gegossen. Man extrahierte mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 2,5 g der Titelverbindung.
¹H-NMR (400 MHz, CDCl₃): δ= 7,18-7,30 m (4H); 4,66 d (2H); 4,42 s (1 H); 4,30 dbr (1 H); 3,40-3,58 m (4H); 1,03 s (3H); 0,87 s (3H); 0,51 s (3H).

### d) 4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]benzaldehyd

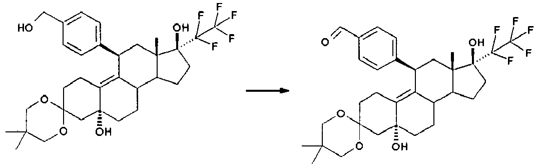

Zu einer Lösung von 2,51 g der unter 1c) beschriebenen Verbindung in 30 ml Dichlormethan wurden 735 mg N-Methylmorpholin-N-Oxid, 58 mg Tetrabutylammoniumperuthenat und etwas Molsieb (4 A) addiert. Man ließ 3,5 Stunden bei 23°C nachrühren. Anschließend wurde das Reaktionsgemisch über etwas Kieselgel filtriert und im Vakuum eingeengt. Das erhaltene Rohprodukt wurde durch Chromatographie an Kieselgel aufgereinigt. Man erhielt 2,35 g der Titelverbindung.
¹H-NMR (400 MHz, CDCl₃): ö= 9,95 s (1 H); 7,79 d (2H); 7,40 d (2H); 4,44 s (1 H); 4,39 dbr (1H); 3,40-3,60 m (4H); 1,04 s (3H); 0,87 s (3H); 0,50 s (3H).

### e) 4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]benzoesäure

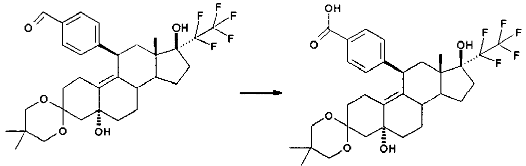

1,09 g der unter 1d) beschriebenen Verbindung wurden mit 66 ml tert.Butanol und einer Mischung von 11 ml 2-Methyl-2-buten und 50 ml Tetrahydrofuran versetzt. Es wurde auf 0°C gekühlt. Dann addierte man unter starkem Rühren 13,9 ml Wasser, 1,02 g Natriumchlorit und 755 mg Natriumhydrogenphosphat Monohydrat. Man ließ 3,5 Stunden bei 0°C nachrühren. Anschließend wurde das Reaktionsgemisch vorsichtig auf gesättigte Natriumthiosulfatlösung gegossen. Man extrahierte mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 839 mg der Titelverbindung.
¹H-NMR (300 MHz, CDCl₃): δ= 8,00 d (2H); 7,32 d (2H); 4,40 dbr (1H); 3,40-3,60 m (4H); 1,05 s (3H); 0,86 s (3H); 0,50 s (3H).

### f) Methyl-4-[(5R,8S,11 R,13S,14S,17S)-5,17-dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]benzoat

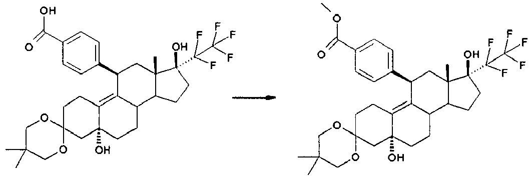

833 mg der unter 1e) beschriebenen Verbindung wurden in einer Mischung aus 17 ml Tetrahydrofuran und 3,6 ml Methanol gelöst. Zu dieser Lösung wurden 773 µl einer 2 molaren Lösung von (Trimethylsilyl)diazomethan in Diethylether getropft. Man ließ 1,5 Stunden bei 23°C nachrühren. Danach wurde das Reaktionsgemisch im Vakuum eingeengt. Das erhaltene Rohprodukt wurde durch Chromatographie an Kieselgel aufgereinigt. Man erhielt 720 mg der Titelverbindung.
¹H-NMR (300 MHz, CDCl₃): δ= 7,93 d (2H); 7,29 d (2H); 4,43 s (1 H); 4,36 dbr (1 H); 3,90 s (3H); 3,35-3,60 m (4H); 1,06 s (3H); 0,86 s (3H); 0,51 s (3H).

### g) 4-[(5R,11R,13S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]benzamid

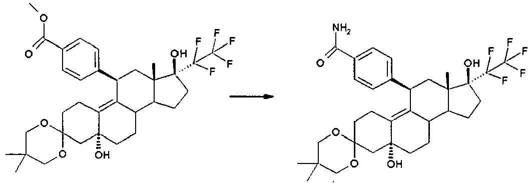

554 mg der unter 1f) beschriebenen Verbindung wurden mit 9 ml einer 7 molaren methanolischen Ammoniaklösung versetzt. Das Reaktionsgemisch wurde in einem Bombenrohr 4 Tage bei 85°C gerührt. Nach dem Abkühlen wurde im Vakuum eingeengt. Das erhaltene Rohprodukt wurde durch Chromatographie an Kieselgel aufgereinigt. Man erhielt 270 mg der Titelverbindung.
¹H-NMR (300 MHz, CDCl₃): 5= 7,70 d (2H); 7,30 d (2H); 6,02 sbr 1H); 5,61 sbr (1 H); 4,43 s (1H); 4,36 dbr (1H); 3,40-3,62 m (4H); 1,03 s (3H); 9,87 s (3H); 0,50 s (3H).

### h) 4-[(11β,17β)-17-Hydroxy-3-oxo-17-(pentafluorethyl)estra-4,9-dien-11-yl]benzamid

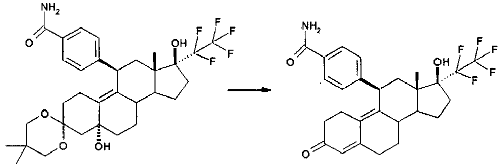

260 mg der unter 1g) beschriebenen Verbindung wurden in 6 ml Methanol gelöst. Man addierte 190 µl halbkonzentrierte Schwefelsäure und ließ 3 Stunden bei 23°C nachrühren. Danach wurde das Reaktionsgemisch auf gesättigte wässrige Natriumhydrogencarbonatlösung gegossen. Man extrahierte mehrfach mit Ethylacetat. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 186 mg der Titelverbindung.
¹H-NMR (400 MHz, CDCl₃): ö= 7,73 d (2H); 7,28 d (2H); 6,10 sbr (1 H); 5,80 sbr (1 H); 5,64 sbr (1 H); 4,48 dbr (1 H); 0,56 s (3H).

### Beispiel 2:

### 4-[(11β,17β)-17-Hydroxy-3-oxo-17-(pentafluorethyl)estra-4,9-dien-11-yl]-N,N-dimethylbenzamid

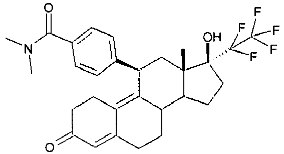

### a) (5R,8S,11R,13S,14S,17S)-11-[4-(Dimethoxymethyl)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

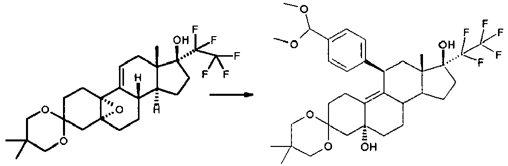

1,48 g Magnesium-Späne wurden in 5 ml Tetrahydrofuran suspendiert und unter Rühren mit 50 µl Dibromethan versetzt. Zur Suspension wurde bei 40°C eine Lösung von 10,18 ml 1-Brom-4-(dimethoxymethyl)benzol in 70 ml Tetrahydrofuran addiert. Danach wurde für eine Stunde bei 50°C nachgerührt. Anschließend wurde die entstandene Lösung auf 0 °C gekühlt. Man addierte 40 mg Kupfer (I) chlorid und ließ weitere 15 Minuten bei 0°C nachrühren. Danach wurde eine Lösung von 5 g der unter Beispiel 1a) beschriebenen Substanz in 50 ml Tetrahydrofuran addiert. Dann ließ man das Reaktionsgemisch unter Rühren über ca. 3 Stunden auf 23°C kommen und rührte anschließend bei dieser Temperatur 10 Stunden nach. Anschließend wurde gesättigte wässrige Ammoniumchlorid-Lösung zum Reaktionsgemisch unter externer Kühlung addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 6,4 g der Titelverbindung.
¹H-NMR (400 MHz, CDCl₃): δ= 7,32 d (2H); 7,21 d (2H); 5,36 s (1 H); 4,43 s (1 H); 4,32 dbr (1 H); 3,39-3,58 m (4H); 3,31 s (6H); 1,03 s (3H); 0,86 s (3H); 0,51 s (3H).

### b) 4-[(11β,17β)-17-Hydroxy-3-oxo-17-(pentafluorethyl)estra-4,9-dien-11-yl]benzaldehyd

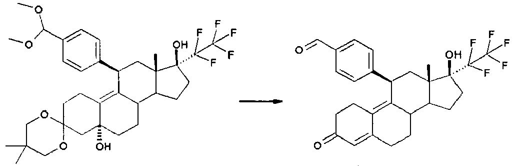

3,5 g der unter 2a) beschriebenen Verbindung wurden in 55 ml 70%iger Essigsäure gelöst. Man ließ 16 Stunden bei 30°C nachrühren. Anschließend wurde das Reaktionsgemisch auf Wasser gegossen. Man ließ weitere 5 Stunden nachrühren. Danach wurde filtriert. Der Rückstand wurde mit Wasser gewaschen, getrocknet und durch Chromatographie an Kieselgel gereinigt. Man erhielt 2,2 g der Titelverbindung.
¹H-NMR (300 MHz, CDCl₃): δ= 9,99 s (1H); 7,80 d (2H); 7,37 d (2H); 5,80 sbr (1H); 4,51 dbr (1 H); 0,58 s (3H).

### c) 4-[(11β,17β)-17-Hydroxy-3-oxo-17-(pentafluorethyl)estra-4,9-dien-11-yl]benzoesäure

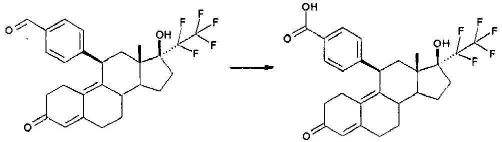

Zu einer Lösung von 1,2 g der unter Beispiel 2b) beschriebenen Verbindung in 40 ml Aceton wurden bei -15°C 910 µl 8N Chromschwefelsäure (Jones-Reagenz) addiert. Man ließ 4,5 Stunden bei 0°C nachrühren und goss dann das Reaktionsgemisch auf 300 ml eiskalte gesättigte Natriumchloridlösung. Man ließ 12 Stunden nachrühren und filtrierte anschließend das ausgefallene Reaktionsprodukt ab. Das Rohprodukt wurde durch Chromatographie an Kieselgel aufgereinigt. Man erhielt 989 mg der Titelverbindung.
¹H-NMR (400 MHz, CDCl₃): δ= 8,00 d (2H); 7,30 d (2H); 5,80 sbr (1 H); 4,50 dbr (1 H); 0,59 s (3H).

### d) 4-[(11β,17β)-17-Hydroxy-3-oxo-17-(pentafluorethyl)estra-4,9-dien-11-yl]-N,N-dimethylbenzamid

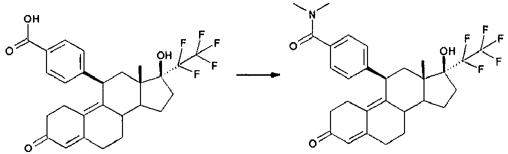

Zu einer Lösung von 60 mg der unter Beispiel 2c) beschriebenen Verbindung in 2 ml N,N-Dimethylformamid wurden 20 µl Triethylamin, 6,9 µl Dimethylamin und 43 mg 2-(1H-Benzotriazol-1-yl)-1,1,3,3-tetramethyluronium-tetrafluoroborat (TBTU) addiert. Man ließ das Reaktionsgemisch 16 Stunden bei 23°C nachrühren und verdünnte dann mit 50 ml Ethylacetat. Die Lösung wurde mit gesättigter Natriumhydrogencarbonatlösung sowie gesättigter Natriumchloridlösung gewaschen und über Natriumsulfat getrocknet. Das Rohprodukt wurde durch Chromatographie an Kieselgel aufgereinigt. Man erhielt 48 mg der Titelverbindung.
¹H-NMR (300 MHz, CDCl₃): ö= 7,39 d (2H); 7,25 d (2H); 5,83 sbr (1 H); 4,51 dbr (1 H); 3,12 sbr (3H); 3,03 sbr (3H); 0,60 s (3H).

Die Beispiele 3-10 wurden in Analogie zu Beispiel 2d aus der unter 2c) beschriebenen Verbindung und dem jeweiligen Amin synthetisiert:

| Bsp. | Struktur | Name | ¹H-NMR |
|---|---|---|---|
| 3 | 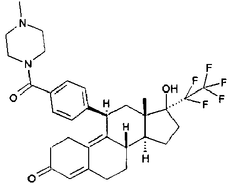 | (11β,17β)-17-Hydroxy-11-{4-[(4-methylpiperazin-1-yl)carbonyl]phenyl}-17-(pentafluorethyl)estra-4,9-dien-3-on | ¹H-NMR (300 MHz, CDCl₃): δ= 7,32 d (2H); 7,21 d (2H); 5,79 sbr (1H); 4,46 dbr (1H); 3,78 m (2H); 3,43 m (2H); 2,30 s (3H); 0,56 s (3H). |
| 4 | 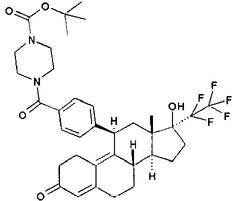 | tert-Butyl-4-{4-[(11β,17β)-17-hydroxy-3-oxo-17-(pentafluorethyl)estra-4,9-dien-11-yl]benzoyl}piperazin-1-carboxylat | ¹H-NMR (300 MHz, CDCl₃): δ= 7,38 d (2H); 7,26 d (2H); 5,84 sbr (1H); 4,50 dbr (1H); 3,35-3,85 m (8H); 1,52 s (9H); 0,61 s (3H). |
| 5 | 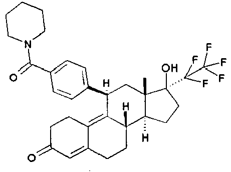 | (11β,17β)-17-Hydroxy-17-(pentafluorethyl)-11-[4-(piperidin-1-ylcarbonyl)phenyl]estra-4,9-dien-3-on | ¹H-NMR (300 MHz, CDCl₃): δ= 7,35 d (2H); 7,25 d (2H); 5,84 sbr (1H); 4,50 dbr (1H); 3,73 m (2H); 3,39 m (2H); 0,61 s (3H). |
| 6 | 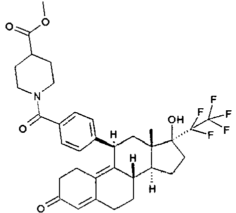 | Methyl-1-{4-[(11β,17β)-17-hydroxy-3-oxo-17-(pentafluorethyl)estra-4,9-dien-11-yl]benzoyl}piperidin-4-carboxylat | ¹H-NMR (300 MHz, CDCl₃): δ= 7,31 d (2H); 7,21 d (2H); 5,79 sbr (1H); 4,46 dbr (1H); 3,70 s (3H); 0,58 s (3H). |
| 7 | 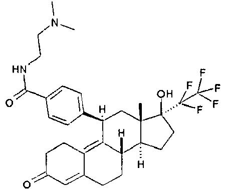 | N-[2-(Dimethylamino)ethyl]-4-[(11β,17β)-17-hydroxy-3-oxo-17-(pentafluorethyl)estra-4,9-dien-11-yl]benzamid | ¹H-NMR (400 MHz, CDCl₃): δ= 7,74 d (2H); 7,28 d (2H); 7,09 t (1H); 5,79 sbr (1H); 4,48 dbr (1H); 3,51 m (2H); 2,29 s (6H); 0,59 s (3H). |
| 8 | 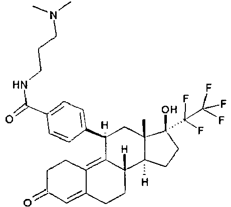 | N-[3-(Dimethylamino)propyl]-4-[(11β,17β)-17-hydroxy-3-oxo-17-(pentafluorethyl)estra-4,9-dien-11-yl]benzamid | ¹H-NMR (300 MHz, CDCl₃): δ= 8,36 t (1H); 7,70 d (2H); 7,27 d (2H); 5,79 sbr (1H); 4,48 dbr (1H); 3,54 m (2H); 2,33 s (6H); 0,58 s (3H). |
| 9 | 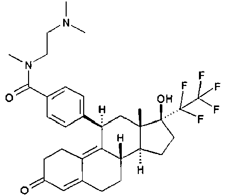 | N-[2-(Dimethylamino)ethyl]-4-[(11β,17β)-17-hydroxy-3-oxo-17-(pentafluorethyl)estra-4,9-dien-11-yl]-N-methylbenzamid | ¹H-NMR (300 MHz, CDCl₃): δ= 7,34 d (2H); 7,20 d (2H); 5,79 sbr (1H); 4,45 dbr (1H); 3,05 m (3H); 2,30 m (6H); 0,55 s (3H). |
| 10 | 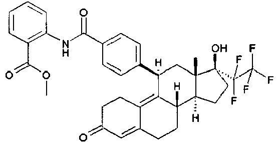 | Methyl-2-({4-[(11β,17β)-17-hydroxy-3-oxo-17-(pentafluorethyl)estra-4,9-dien-11-yl]benzoyl}amino)benzoat | ¹H-NMR (400 MHz, CDCl₃): 5= 8,92 d (1H); 8,09 d (1H); 7,99 d (2H); 7,61 tbr (1H); 7,33 d (2H); 7,12 tbr (1H); 5,80 sbr (1H); 4,52 dbr (1H); 3,97 s (3H); 0,60 s (3H). |

Die Beispiele 11-72 wurden gemäß der folgenden Vorschrift durch Parallelsynthese hergestellt:
Die Carbonsäure (0.4 mL einer 0.5 M Suspension in DMF, 0.2 mmol, 1 eq.) wurde unter Schutzgas bei 20 °C vorgelegt, unter Rühren nacheinander das Amin (0.5 mL einer 0.5 M Lösung in DMF, 0.25 mmol, 1.25 eq.) und Diisopropylethylamin (0.175 mL, 1 mmol, 5 eq.) zugegeben, auf 0 °C abgekühlt und schließlich 2-Propanphosphonsäureanhydrid (0.23 mL einer 50%igen Lösung in DMF, 0.39 mmol,1.95 eq.) zugegeben. Nach weiteren 10 min. bei 0 °C wurde auf 20 °C erwärmt und 10 h bei 20 °C gerührt. Zur Aufarbeitung wurde das Reaktionsgemisch mit Methanol (3 mL) verdünnt, überführt und eingeengt. Der Rückstand wurde in 1 mL DMSO und 1 mL Acetonitril/Wasser 9:1 gelöst, ein Niederschlag ggf. abfiltriert, die Lösung durch HPLC gereinigt und per HPLC-MS analysiert (Waters HPLC 1525µ Binary HPLC, Micromass ZQ, MUX UV 2488, Wellenlänge 210-350 nm, Säule Waters XBridge C18 3,5 µM, 4,6x50 mm, Gradient: Gradient 1-99% Acetonitril in 0.1% Trifluoressigsäure/Wasser, Flußrate 2 mL/min., Laufzeit 8 min.).

| Bsp. | Struktur | Name | HPLC-MS (MH⁺, RT) |
|---|---|---|---|
| 11 | 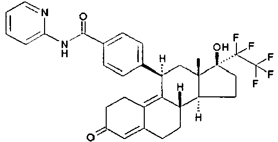 | 4-[(11β,17β)-17-Hydroxy-3-oxo-17-(pentafluorethyl)estra-4,9-dien-11-yl]-N-(pyridin-2-yl)benzamid | 587, 4.25 min. |
| 12 | 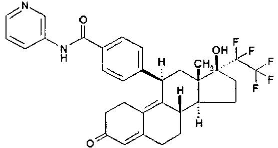 | 4-[(11β,17β)-17-Hydroxy-3-oxo-17-(pentafluorethyl)estra-4,9-dien-11-yl]-N-(pyridin-3-yl)benzamid | 587, 3.71 min. |
| 13 | 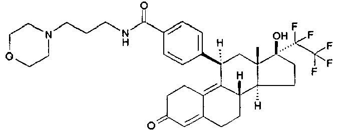 | 4-[(11β,17β)-17-Hydroxy-3-oxo-17-(pentafluorethyl)estra-4,9-dien-11-yl]-N-[3-(morpholin-4-yl)propyl]benzamid | 637,3.15 min. |
| 14 | 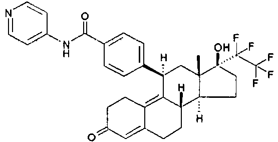 | 4-[(11β,17β)-17-Hydroxy-3-oxo-17-(pentafluorethyl)estra-4,9-dien-11-yl]-N-(pyridin-4-yl)benzamid | 587, 3.31 min. |
| 15 | 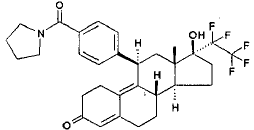 | (11β,17β)-17-Hydroxy-17-(pentafluorethyl)-11-[4-(pyrrolidin-1-ylcarbonyl)phenyl]estra-4,9-dien-3-on | 564, 4.20 min. |
| 16 | 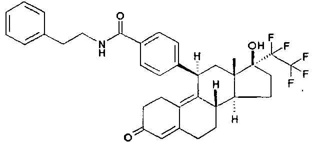 | 4-[(11β,17β)-17-Hydroxy-3-oxo-17-(pentafluorethyl)estra-4,9-dien-11-yl]-N-(2-phenylethyl)benzamid | 614,4.56 min. |
| 17 | 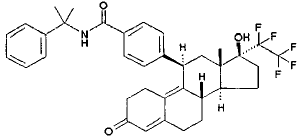 | 4-[(11β,17β)-17-Hydroxy-3-oxo-17-(pentafluorethyl)estra-4,9-dien-11-yl]-N-(2-phenylpropan-2-yl)benzamid | 628, 4.75 min. |
| 18 | 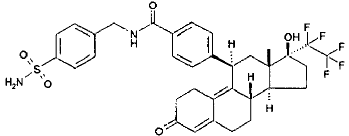 | 4-[(11β,17β)-17-Hydroxy-3-oxo-17-(pentafluorethyl)estra-4,9-dien-11-yl]-N-(4-sulfamoylbenzyl)benzamid | 679, 4.07 min. |
| 19 | 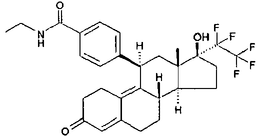 | N-Ethyl-4-[(11β,17β)-17-hydroxy-3-oxo-17-(pentafluorethyl)estra-4,9-dien-11-yl]benzamid | 538, 4.08 min. |
| 20 | 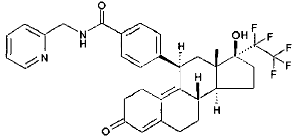 | 4-[(11β,17β)-17-Hydroxy-3-oxo-17-(pentafluorethyl)estra-4,9-dien-11-yl]-N-(pyridin-2-ylmethyl)benzamid | 601, 3.68 min. |
| 21 | 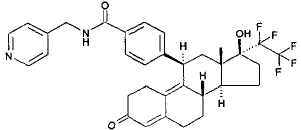 | 4-[(11β,17β)-17-Hydroxy-3-oxo-17-(pentafluorethyl)estra-4,9-dien-11-yl]-N-(pyridin-4-ylmethyl)benzamid | 601, 3.30 min. |
| 22 | 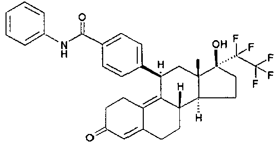 | 4-[(11β,17β)-17-Hydroxy-3-oxo-17-(pentafluorethyl)estra-4,9-dien-11-yl]-N-phenylbenzamid | 586, 4.57 min. |
| 23 | 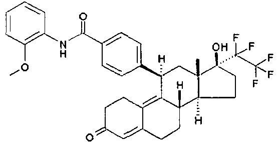 | 4-[(11β,17β)-17-Hydroxy-3-oxo-17-(pentafluorethyl)estra-4,9-dien-11-yl]-N-(2-methoxyphenyl)benzamid | 616,4.70 min. |
| 24 | 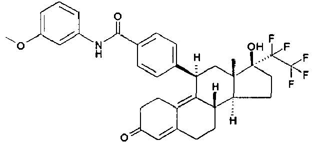 | 4-[(11β,17β)-17-Hydroxy-3-oxo-17-(pentafluorethyl)estra-4,9-dien-11-yl]-N-(3-methoxyphenyl)benzamid | 616,4.58 min. |
| 25 | 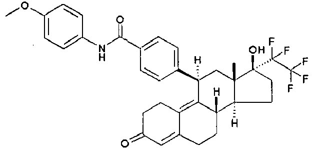 | 4-[(11β,17β)-17-Hydroxy-3-oxo-17-(pentafluorethyl)estra-4,9-dien-11-yl]-N-(4-methoxyphenyl)benzamid | 616,4.53 min. |
| 26 | 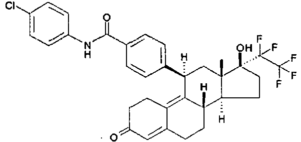 | N-(4-Chlorphenyl)-4-[(11β,17β)-17-hydroxy-3-oxo-17-(pentafluorethyl)estra-4,9-dien-11-yl]benzamid | 620, 4.78 min. |
| 27 | 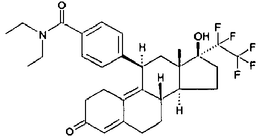 | N,N-Diethyl-4-[(11β,17β)-17-hydroxy-3-oxo-17-(pentafluorethyl)estra-4,9-dien-11-yl]benzamid | 566, 4.38 min. |
| 28 | 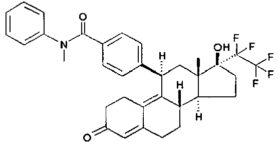 | 4-[(11β,17β)-17-Hydroxy-3-oxo-17-(pentafluorethyl)estra-4,9-dien-11-yl]-N-methyl-N-phenylbenzamid | 600, 4.62 min. |
| 29 | 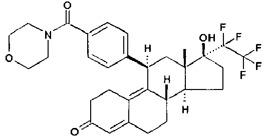 | (11β,17β)-17-Hydroxy-11-[4-(morpholin-4-ylcarbonyl)phenyl]-17-(pentafluorethyl)estra-4,9-dien-3-on | 580, 4.03 min. |
| 30 | 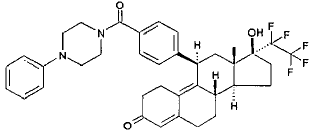 | (11β,17β)-17-Hydroxy-17-(pentafluorethyl)-11-{4-[(4-phenylpiperazin-1-yl)carbonyl]phenyl}estra-4,9-dien-3-on | 655, 4.71 min. |
| 31 | 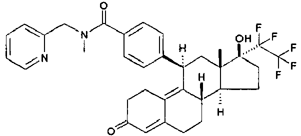 | 4-[(11β,17β)-17-Hydroxy-3-oxo-17-(pentafluorethyl)estra-4,9-dien-11-yl]-N-methyl-N-(pyridin-2-ylmethyl)benzamid | 615,4.00 min. |
| 32 | 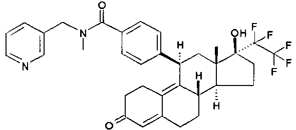 | 4-[(11β,17β)-17-Hydroxy-3-oxo-17-(pentafluorethyl)estra-4,9-dien-11-yl]-N-methyl-N-(pyridin-3-ylmethyl)benzamid | 615,3.57 min. |
| 33 | 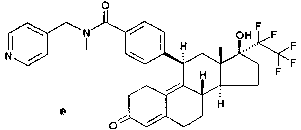 | 4-[(11β,17β)-17-Hydroxy-3-oxo-17-(pentafluorethyl)estra-4,9-dien-11-yl]-N-methyl-N-(pyridin-4-ylmethyl)benzamid | 615,3.35 min. |
| 34 | 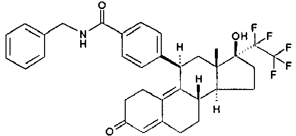 | N-Benzyl-4-[(11β,17β)-17-hydroxy-3-oxo-17-(pentafluorethyl)estra-4,9-dien-11-yl]benzamid | 600, 4.49 min. |
| 35 | 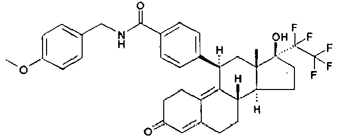 | 4-[(11β,17β)-17-Hydroxy-3-oxo-17-(pentafluorethyl)estra-4,9-dien-11-yl]-N-(4-methoxybenzyl)benzamid | 630, 4.45 min. |
| 36 | 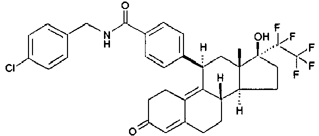 | N-(4-Chlorbenzyl)-4-[(11β,17β)-17-hydroxy-3-oxo-17-(pentafluorethyl)estra-4,9-dien-11-yl]benzamid | 634, 4.63 min. |
| 37 | 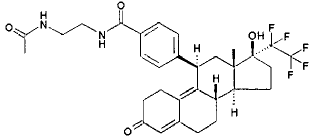 | N-(2-Acetamidoethyl)-4-[(11β,17β)-17-hydroxy-3-oxo-17-(pentafluorethyl)estra-4,9-dien-11-yl]benzamid | 595,3.77 min. |
| 38 | 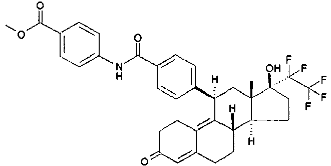 | Methyl-4-({4-[(11β,17β)-17-hydroxy-3-oxo-17-(pentafluorethyl)estra-4,9-dien-11-yl]benzoyl}amino)benzoat | 644, 4.58 min. |
| 39 | 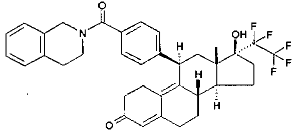 | (11β,17β)-11-[4-(3,4-Dihydroisochinolin-2(1H)-ylcarbonyl)phenyl]-17-hydroxy-17-(pentafluorethyl)estra-4,9-dien-3-on | 626,4.71 min. |
| 40 | 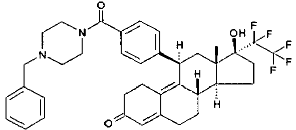 | (11β,17β)-11-{4-[(4-Benzylpiperazin-1-yl)carbonyl]phenyl}-17-hydroxy-17-(pentafluorethyl)estra-4,9-dien-3-on | 669, 3.38 min. |
| 41 | 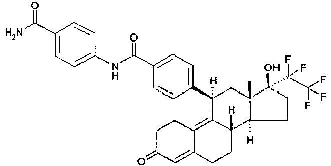 | N-(4-Carbamoylphenyl)-4-[(11β,17β)-17-hydroxy-3-oxo-17-(pentafluorethyl)estra-4,9-dien-11-yl]benzamid | 629, 4.05 min. |
| 42 | 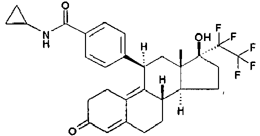 | N-Cyclopropyl-4-[(11β,17β)-17-hydroxy-3-oxo-17-(pentafluorethyl)estra-4,9-dien-11-yl]benzamid | 550,4.13 min. |
| 43 | 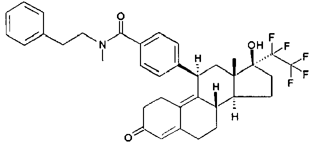 | 4-[(11β,17β)-17-Hydroxy-3-oxo-17-(pentafluorethyl)estra-4,9-dien-11-yl]-N-methyl-N-(2-phenylethyl)benzamid | 628, 4.70 min. |
| 44 | 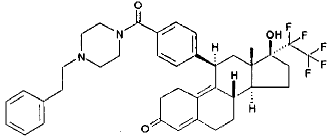 | (11β,17β)-17-Hydroxy-17-(pentafluorethyl)-11-(4-{[4-(2-phenylethyl)piperazin-1-yl]carbonyl}phenyl)estra-4,9-dien-3-on | 683, 3.40 min. |
| 45 | 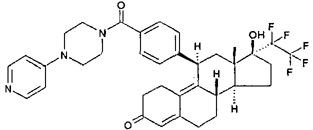 | (11β,17β)-17-Hydroxy-17-(pentafluorethyl)-11-(4-{[4-(pyridin-4-yl)piperazin-1-yl]carbonyl}phenyl)estra-4,9-dien-3-on | 656, 3.25 min. |
| 46 | 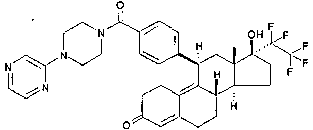 | (11β,17β)-17-Hydroxy-17-(pentafluorethyl)-11-(4-{[4-(pyrazin-2-yl)piperazin-1-yl]carbonyl}phenyl)estra-4,9-dien-3-on | 657,4.12 min. |
| 47 | 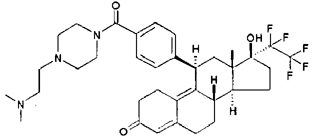 | (11β,17β)-11-[4-({4-[2-(Dimethylamino)ethyl]piperazin-1-yl}carbonyl)phenyl]-17-hydroxy-17-(pentafluorethyl)estra-4,9-dien-3-on | 650,3.11 min. |
| 48 | 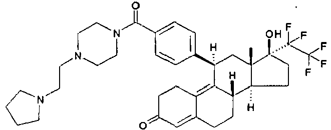 | (11β,17β)-17-Hydroxy-17-(pentafluorethyl)-11-[4-({4-[2-(pyrrolidin-1-yl)ethyl]piperazin-1-yl}carbonyl)phenyl]estra-4,9-dien-3-on | 676,3.17 min. |
| 49 | 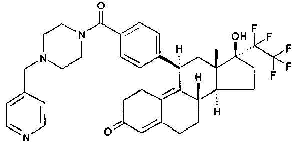 | (11β,17β)-17-Hydroxy-17-(pentafluorethyl)-11-(4-{[4-(pyridin-4-ylmethyl)piperazin-1-yl]carbonyl}phenyl)estra-4,9-dien-3-on | 670, 3.21 min. |
| 50 | 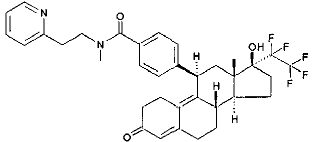 | 4-[(11β,17β)-17-Hydroxy-3-oxo-17-(pentafluorethyl)estra-4,9-dien-11-yl]-N-methyl-N-[2-(pyridin-2-yl)ethyl]benzamid | 629, 3.45 min. |
| 51 | 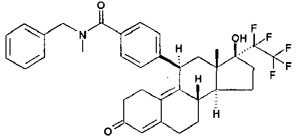 | N-Benzyl-4-[(11β,17β)-17-hydroxy-3-oxo-17-(pentafluorethyl)estra-4,9-dien-1-yl]-N-methylbenzamid | 614,4.65 min. |
| 52 | 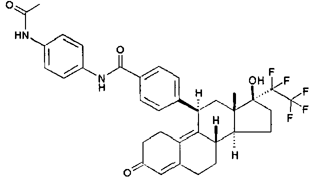 | N-(4-Acetamidophenyl)-4-[(11β,17β)-17-hydroxy-3-oxo-17-(pentafluorethyl)estra-4,9-dien-11-yl]benzamid | 643,4.15 min. |
| 53 | 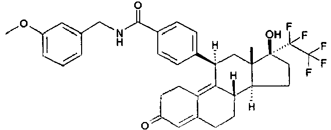 | 4-[(11β,17β)-17-Hydroxy-3-oxo-17-(pentafluorethyl)estra-4,9-dien-11-yl]-N-(3-methoxybenzyl)benzamid | 630, 4.47 min. |
| 54 | 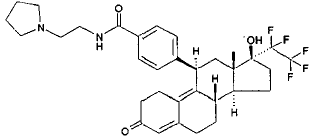 | 4-[(11β,17β)-17-Hydroxy-3-oxo-17-(pentafluorethyl)estra-4,9-dien-11-yl]-N-[2-(pyrrolidin-1-yl)ethyl]benzamid | 607, 4.75 min. |
| 55 | 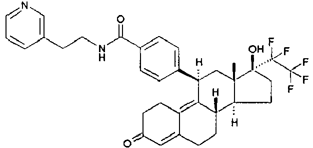 | 4-[(11β,17β)-17-Hydroxy-3-oxo-17-(pentafluorethyl)estra-4,9-dien-11-yl]-N-[2-(pyridin-3-yl)ethyl]benzamid | 615,3.30 min. |
| 56 | 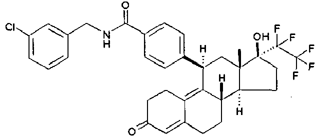 | N-(3-Chlorbenzyl)-4-[(11β,17β)-17-hydroxy-3-oxo-17-(pentafluorethyl)estra-4,9-dien-11-yl]benzamid | 634, 4.70 min. |
| 57 | 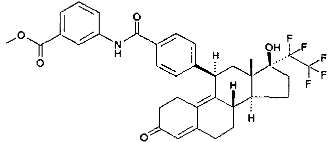 | Methyl-3-({4-[(11β,17β)-17-hydroxy-3-oxo-17-(pentafluorethyl)estra-4,9-dien-11-yl]benzoyl}amino)benzoat | 644, 4.60 min. |
| 58 | 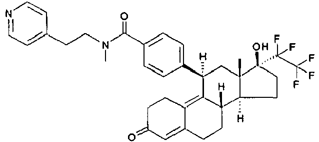 | 4-[(11β,17β)-17-Hydroxy-3-oxo-17-(pentafluorethyl)estra-4,9-dien-11-yl]-N-methyl-N-[2-(pyridin-4-yl)ethyl]benzamid | 629, 3.28 min. |
| 59 | 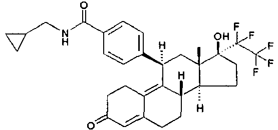 | N-(Cyclopropylmethyl)-4-[(11β,17β)-17-hydroxy-3-oxo-17-(pentafluorethyl)estra-4,9-dien-11-yl]benzamid | 564, 4.35 min. |
| 60 | 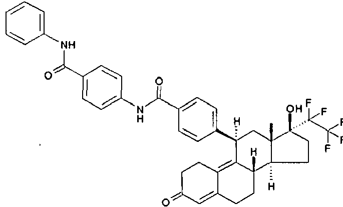 | 4-[(11β,17β)-17-Hydroxy-3-oxo-17-(pentafluorethyl)estra-4,9-dien-11-yl]-N-[4-(phenylcarbamoyl)phenyl]benzamid | 705, 4.50 min. |
| 61 | 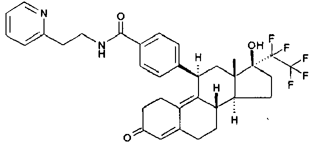 | 4-[(11β,17β)-17-Hydroxy-3-oxo-17-(pentafluorethyl)estra-4,9-dien-11-yl]-N-[2-(pyridin-2-yl)ethyl]benzamid | 615, 3.37 min. |
| 62 | 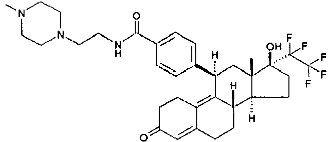 | 4-[(11β,17β)-17-Hydroxy-3-oxo-17-(pentafluorethyl)estra-4,9-dien-11-yl]-N-[2-(4-methylpiperazin-1-yl)ethyl]benzamid | 636, 3.08 min. |
| 63 | 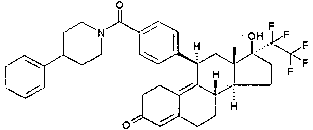 | (11β,17β)-17-Hydroxy-17-(pentafluorethyl)-11-{4-[(4-phenylpiperidin-1-yl)carbonyl]phenyl}estra-4,9-dien-3-on | 654, 4.87 min. |
| 64 | 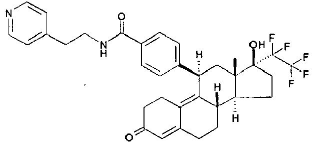 | 4-[(11β,17β)-17-Hydroxy-3-oxo-17-(pentafluorethyl)estra-4,9-dien-11-yl]-N-[2-(pyridin-4-yl)ethyl]benzamid | 615,3.23 min. |
| 65 | 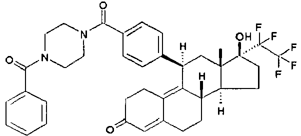 | (11β,17β)-11-{4-[(4-Benzoylpiperazin-1-yl)carbonyl]phenyl}-17-hydroxy-17-(pentafluorethyl)estra-4,9-dien-3-on | 683, 4.28 min. |
| 66 | 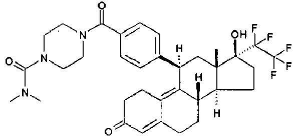 | 4-{4-[(11β,17β)-17-Hydroxy-3-oxo-17-(pentafluorethyl)estra-4,9-dien-11-yl]benzoyl}-N,N-dimethylpiperazin-1-carboxamid | 650, 3.93 min. |
| 67 | 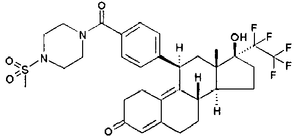 | (11β,17β)-17-Hydroxy-11-(4-{[4-(methylsulfonyl)piperazin-1-yl]carbonyl}phenyl)-17-(pentafluorethyl)estra-4,9-dien-3-on | 657,4.10 min. |
| 68 | 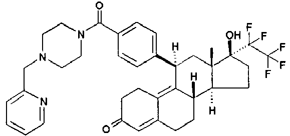 | (11β,17β)-17-Hydroxy-17-(pentafluorethyl)-11-(4-{[4-(pyridin-2-ylmethyl)piperazin-1-yl]carbonyl}phenyl)estra-4,9-dien-3-on | 670, 3.20 min. |
| 69 | 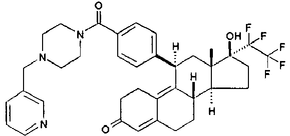 | (11β,17β)-17-Hydroxy-17-(pentafluorethyl)-11-(4-{[4-(pyridin-3-ylmethyl)piperazin-1-yl]carbonyl}phenyl)estra-4,9-dien-3-on | 670,3.17 min. |
| 70 | 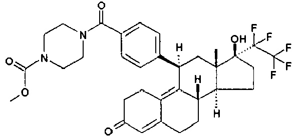 | Methyl-4-{4-[(11β,17β)-17-hydroxy-3-oxo-17-(pentafluorethyl)estra-4,9-dien-11-yl]benzoyl}piperazin-1-carboxylat | 637,4.08 min. |
| 71 | 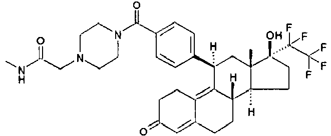 | 2-(4-{4-[(11β,17β)-17-Hydroxy-3-oxo-17-(pentafluorethyl)estra-4,9-dien-11-yl]benzoyl}piperazin-1-yl)-N-methylacetamid | 650, 3.23 min. |
| 72 | 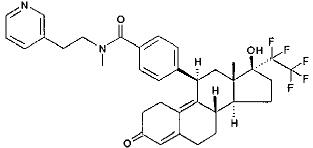 | 4-[(11β,17β)-17-Hydroxy-3-oxo-17-(pentafluorethyl)estra-4,9-dien-11-yl]-N-methyl-N-[2-(pyridin-3-yl)ethyl]benzamid | 629, 3.48 min. |

### Beispiel 73:

### (11β,17β)-17-Hydroxy-17-(pentafluorethyl)-11-[4-(piperazin-1-ylcarbonyl)phenyl]estra-4,9-dien-3-on

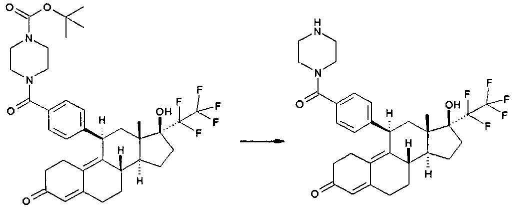

Zu einer Lösung von 166 mg der unter Beispiel 4) beschriebenen Verbindung in 5 ml Dichlormethan wurden 0,47 ml Trifluoressigsäure addiert. Man ließ 90 Minuten bei 23°C nachrühren und goss danach das Reaktionsgemisch auf eiskalte gesättigte Natriumhydrogencarbonatlösung. Danach extrahierte man mehrfach mit Dichlormethan. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 56 mg der Titelverbindung.
¹H-NMR (300 MHz, CDCl₃): δ= 7,37 d (2H); 7,28 d (2H); 5,83 sbr (1H); 4,50 dbr (1 H); 3,76 m (2H); 3,49 m (2H); 2,96 m (4H); 0,59 s (3H).

### Beispiel 74:

### (11β,17β)-11-{4-[(4-Acetylpiperazin-1-yl)carbonyl]phenyl}-17-hydroxy-17-(pentafluorethyl)estra-4,9-dien-3-on

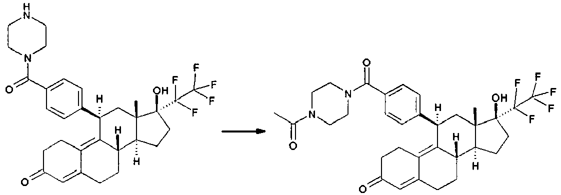

Zu einer Lösung von 70 mg der unter Beispiel 73) beschriebenen Verbindung in 2,5 ml Dichlormethan wurden 42 µl Triethylamin und 14 µl Essigsäureanhydrid addiert. Man ließ 90 Minuten bei 23°C nachrühren und goss danach das Reaktionsgemisch auf eiskalte gesättigte Natriumhydrogencarbonatlösung. Danach extrahierte man mehrfach mit Dichlormethan. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 35 mg der Titelverbindung.
¹H-NMR (300 MHz, CDCl₃): δ= 7,34 d (2H); 7,24 d (2H); 5,79 sbr (1 H); 4,46 dbr (1 H); 3,35-3,85 m (8H); 2,12 s (3H); 0,58 s (3H).

### Beispiel 75:

### 1-{4-[(11β,17β)-17-Hydroxy-3-oxo-17-(pentafluorethyl)estra-4,9-dien-11-yl]benzoyl}piperidin-4-carbonsäure

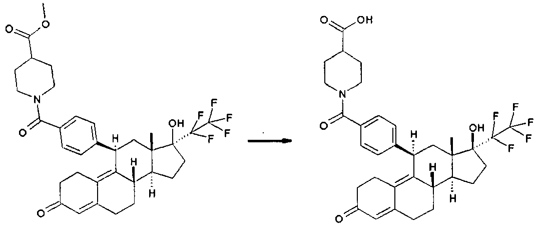

Zu einer Lösung von 225 mg der unter Beispiel 6) beschriebenen Verbindung in 5 ml Methanol wurden 0,53 ml einer 2 N Natriumhydroxidlösung addiert. Man ließ 2,5 Stunden bei 23°C nachrühren und goss danach das Reaktionsgemisch auf 60 ml Eiswasser. Danach wurde mit 2,5 ml 2N Salzsäure angesäuert und 16 Stunden nachgerührt. Der entstandene Niederschlag wurde abgesaugt und mit Wasser gewaschen. Das Rohprodukt wurde durch Chromatographie an Kieselgel aufgereinigt. Man erhielt 139 mg der Titelverbindung.
¹H-NMR (400 MHz, CDCl₃): δ= 7,28 d (2H); 7,20 d (2H); 5,76 sbr (1 H); 4,43 dbr (1 H); 0,51 s (3H).

### Beispiel 76:

### 2-({4-[(11β,17β)-17-Hydroxy-3-oxo-17-(pentafluorethyl)estra-4,9-dien-11-yl]benzoyl}amino)benzoesäure

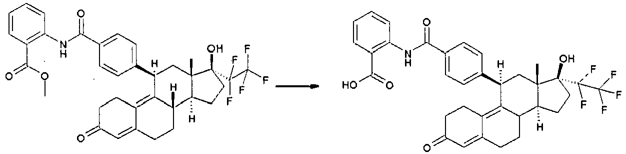

Zu einer Lösung von 43 mg der unter Beispiel 10) beschriebenen Verbindung in 2 ml Tetrahydrofuran wurde eine Lösung von 16 mg Lithiumhydroxid in 466 µl Wasser addiert. Man ließ 19 Stunden bei 23°C nachrühren. Danach wurde das Reaktionsgemisch mit 1ml Wasser verdünnt und dann mit 0,4 ml einer 2N Salzsäure angesäuert. Man extrahierte mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 14 mg der Titelverbindung.
¹H-NMR (400 MHz, CDCl₃): δ= 8,93 d (1H); 8,13 d (1H); 7,92 d (2H); 7,65 tbr (1H); 7,27 d (2H); 7,15 tbr (1 H); 5,87 sbr (1 H); 4,45 dbr (1 H); 0,50 s (3H).

### Beispiel 77:

### 3-({4-[(11β,17β)-17-Hydroxy-3-oxo-17-(pentafluorethyl)estra-4,9-dien-11-yl]benzoyl}amino)benzoesäure

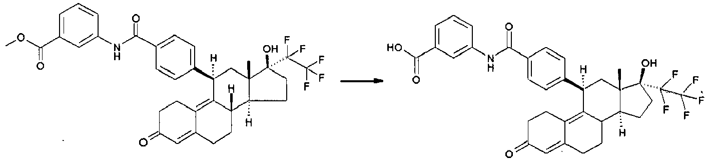

Analog zu Beispiel 76 wurden aus 115 mg der unter Beispiel 57 beschriebenen Verbindung durch Umsetzung mit Lithiumhydroxid in einem Gemisch aus Tetrahydrofuran und Wasser 46 mg der Titelverbindung erhalten.
¹H-NMR (300 MHz, CDCl₃): δ= 8,19 m (2H); 7,78-7,90 m (3H); 7,47 tbr (1H); 7,28 m (2H); 5,79 sbr (1 H); 4,48 dbr (1 H); 0,57 s (3H).

### Beispiel 78:

### 4-({4-[(11β,17β)-17-Hydroxy-3-oxo-17-(pentafluorethyl)estra-4,9-dien-11-yl]benzoyl}amino)benzoesäure

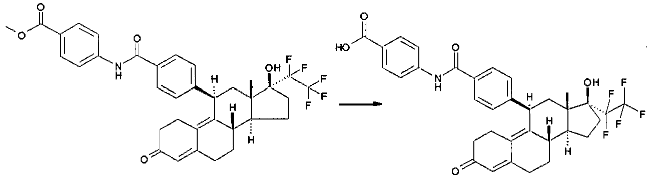

Analog zu Beispiel 76 wurden aus 92 mg der unter Beispiel 38 beschriebenen Verbindung durch Umsetzung mit Lithiumhydroxid in einem Gemisch aus Tetrahydrofuran und Wasser 42 mg der Titelverbindung erhalten.
¹H-NMR (400 MHz, CDCl₃): δ= 8,25 s (1 H); 8,06 d (2H); 7,80 d (2H); 7,71 d (2H); 7,30 d (2H); 5,79 sbr (1 H); 4,50 dbr (1 H); 0,57 s (3H).

### Beispiel 79:

### 4'-[(11β,17β)-17-Hydroxy-3-oxo-17-(pentafluorethyl)estra-4,9-dien-11-yl]biphenyl-4-carboxamid

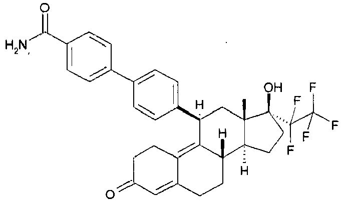

### a) (5R,8S,11R,13S,14S,17S)-11-[4-(Benzyloxy)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

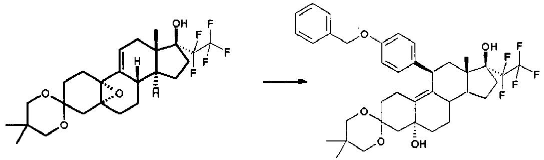

2,47 g Magnesium-Späne wurden 5 ml Tetrahydrofuran suspendiert und unter Rühren mit 50 µl Dibromethan versetzt. Zur Suspension wurde eine Lösung von 26,7 g 1-Brom-4-(phenylmethoxy)benzol in 115 ml Tetrahydrofuran bei 65 °C langsam gegeben. Die entstandene Lösung wurde auf 0 °C gekühlt. Dazu wurden 301 mg Kupfer (I) chlorid gegeben. Man rührte 10 Minuten bei 0°C nach und addierte dann langsam eine Lösung von 10 g der unter Beispiel 1a) beschriebenen Substanz in 70 ml Tetrahydrofuran. Man ließ das Reaktionsgemisch unter Rühren über ca. 3 Stunden auf 23°C kommen und rührte anschließend bei dieser Temperatur 10 Stunden nach. Anschließend wurde gesättigte wässrige Ammoniumchlorid-Lösung zum Reaktionsgemisch unter externer Kühlung addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel und anschließende Kristallisation aus einem Gemisch von Dichlormethan und Diisopropylether aufgereinigt. Man erhielt 9,7 g der Titelverbindung.
¹H-NMR (400 MHz, CDCl₃): δ= 7,30-7,50 m (5H); 7,12 d (2H); 6,88 d (2H); 5,02 s (2H); 4,43 s (1 H); 4,28 dbr (1 H); 3,50-3,60 m (3H); 3,42 d (1 H); 1,06 s (3H); 0,87 s (3H); 0,56 s (3H).

### b) (5R,8S,11R,13S,14S,17S)-11-[4-(Benzyloxy)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

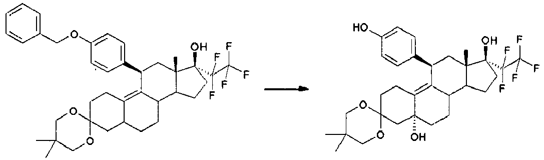

Zu einer Lösung von 9,72 g der unter 79a) beschriebenen Verbindung in 100 ml Methanol wurden 5,53 g Ammoniumformiat und 972 mg Palladium auf Aktivkohle (10%ig) addiert. Man rührte 2 Stunden bei 23°C nach und filtrierte dann über Celite®. Das Filtrat wurde im Vakuum eingeengt. Man erhielt 8,5 g Rohprodukt, welches ohne Reinigung in die Folgestufe eingesetzt wurde.
¹H-NMR (300 MHz, CDCl₃): δ= 7,05 d (2H); 6,70 d (2H); 4,43 sbr (1 H); 4,27 dbr (1 H); 3,50-3,58 m (3H); 3,41 sbr (1H); 1,94 s (3H); 0,86 s (3H); 0,54 s (3H).

### c) 4-[(5R,8S,11R,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]phenyl-1,1,2,2,3,3,4,4,4-nonafluorbutan-1-sulfonat

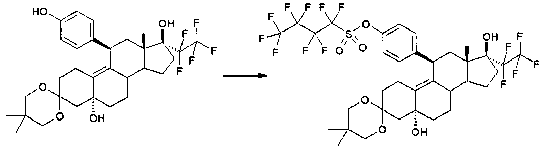

Zu einer Lösung von 9,16 g der unter 79b) beschriebenen Verbindung in 100 ml absolutem Tetrahydrofuran wurden bei 0°C 14,64 ml einer 1,6 molaren Lösung von n-Butyllithium in Hexan addiert. Man ließ 30 Minuten bei 0°C nachrühren und addierte dann langsam 5,62 ml Perfluorbutan-1-sulfonsäurefluorid. Anschließend ließ man weitere 1,5 Stunden bei 0°C nachrühren. Danach wurde das Reaktionsgemisch auf eine Mischung von 300 ml gesättigter Natriumhydrogencarbonatlösung und 90 ml 2 N Natronlauge gegossen. Es wurde 45 Minuten gerührt und dann mehrfach mit Ethylacetat extrahiert. Die vereinigten organischen Phasen wurden mit gesättigter Natriumchloridlösung gewaschen und über Natriumsulfat getrocknet. Das erhaltene Rohprodukt wurde durch Chromatographie an Kieselgel aufgereinigt. Man erhielt 10,1 g der Titelverbindung.
¹H-NMR (400 MHz, CDCl₃): δ= 7,28 d (2H); 7,18 d (2H); 4,42 s (1 H); 4,34 dbr (1 H); 3,50-3,58 m (3H); 3,42 d (1H); 1,05 s (3H); 0,86 s (3H); 0,50 s (3H).

### d) Methyl-4'-[(5R,8S,13S,14S,17S)-5,17-dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]biphenyl-4-carboxylat

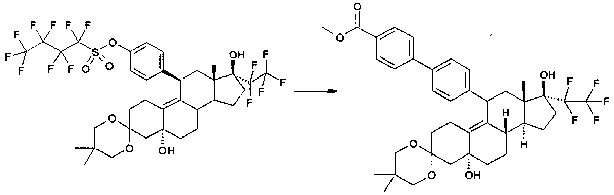

Eine Lösung von 10 g der unter 79c) beschriebenen Verbindung in einem Gemisch aus 100 ml Toluol und 50 ml Ethanol wurde mit 16,7 ml 2 N wässriger Natriumcarbonatlösung, 1,09 g Lithiumchlorid, 2,12 g 4-Methoxycarbonylphenyl-boronsäure und 1,6 g Tetrakis(triphenylphosphin)palladium(0) versetzt. Man kochte 2 Stunden unter Rückfluss, ließ dann auf Raumtemperatur abkühlen und addierte Wasser und Ethylacetat zu dem Reaktionsgemisch. Anschließend wurden die Phasen getrennt und die wässrige Phase wurde mit Ethylacetat extrahiert. Die vereinigten organischen Phasen wurden mit gesättigter Natriumchloridlösung gewaschen und über Natriumsulfat getrocknet. Das erhaltene Rohprodukt wurde durch Chromatographie an Kieselgel und anschließendem Ausrühren in Diisopropylether aufgereinigt. Man erhielt 5,8 g der Titelverbindung.
¹H-NMR (300 MHz, CDCl₃): δ= 8,08 d (2H); 7,66 d (2H); 7,53 d (2H); 7,30 d (2H); 4,45 s (1 H); 4,38 dbr (1 H); 3,95 s (3H); 3,40-3,60 m (4H); 1,04 s (3H); 0,85 s (3H); 0,57 s (3H).

### e) 4'-[(5R,8S,13S,14S,17S)-5,17-Dihydroxy-5',5',13-trimethyl-17-(pentafluorethyl)-1,2,4,5,6,7,8,11,12,13,14,15,16,17-tetradecahydrospiro[cyclopenta[a]phenanthren-3,2'-[1,3]dioxan]-11-yl]biphenyl-4-carboxamid

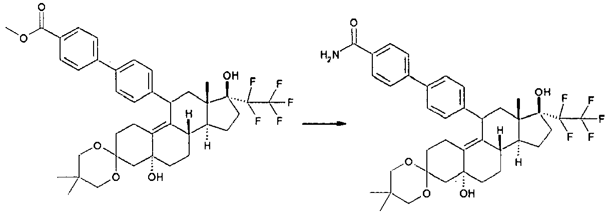

Analog zu Beispiel 1g) wurden aus 755 mg der unter 79e) beschriebenen Verbindung durch Umsetzung mit 11 ml einer 7 molaren methanolischen Ammoniaklösung im Bombenrohr bei 85°C über 4 Tage 395 mg der Titelverbindung erhalten.
¹H-NMR (400 MHz, CDCl₃): δ= 7,87 d (2H); 7,64 d (2H); 7,50 d (2H); 7,31 d (2H); 6,12 sbr (1 H); 5,68 sbr (1 H); 4,45 s (1 H); 4,39 dbr (1 H); 3,40-3,60 m (4H); 1,07 s (3H); 0,87 s (3H); 0,58 s (3H).

### f) 4'-[(11β,17β)-17-Hydroxy-3-oxo-17-(pentafluorethyl)estra-4,9-dien-11-yl]biphenyl-4-carboxamid

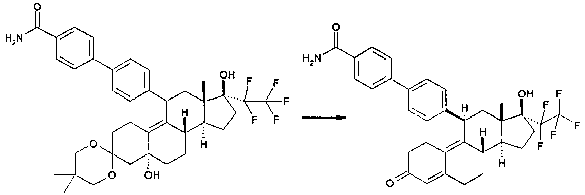

Analog zu Beispiel 2b) wurden aus 390 mg der unter 79e) beschriebenen Verbindung mit 9 ml 70%iger Essigsäure bei 35°C über 16 Stunden 301 mg der Titelverbindung erhalten.
¹H-NMR (300 MHz, CDCl₃): δ= 7,88 d (2H); 7,64 d (2H); 7,52 d (2H); 7,30 d (2H); 6,17 sbr (1 H); 6,01 sbr (1 H); 5,80 sbr (1 H); 4,50 dbr (1 H); 0,67 s (3H).

### Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MTV-LUC Reporter Konstrukt

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0.01 nmol/l, 0.1 nmol/l, 1 nmol/l, 10 nmol/l, 100 nmol/l und 1 µmol/l) inkubiert, um die agonistische Wirksamkeit zu bestimmen. Als Positivkontrolle der Reportergeninduktion wurden die Zellen mit dem synthetischen Gestagen Promegeston (0.01 nmol/l, 0.1 nmol/l, 1 nmol/l, 10 nmol/l, 100 nmol/l and 1 µmol/l) behandelt. Zur Bestimmung der antagonistischen Aktivität wurden die Zellen mit 0.1 nmol/l Promegeston und zusätzlich mit steigenden Mengen der jeweiligen Testverbindung (0.01 nmol/l, 0.1 nmol/l, 1 nmol/l, 10 nmol/l, 100 nmol/l and 1 µmol/l) behandelt. Die Aktivität des Reportergens LUC (LUC = Luciferase) wurde in den Zelllysaten bestimmt und als RLU (relative light units) gemessen. Alle Meßwerte werden angegeben als % Wirksamkeit und als EC₅₀ bzw. IC₅₀ Konzentrationen.

### a) agonistische Aktivität:

Keine der genannten Testverbindungen zeigt eine agonistische Aktivität.

### b) antagonistische Aktivitat:

Alle genannten Verbindungen zeigen eine 100%ige antagonistische Wirksamkeit.
Die antagonistische Wirkstärke der Verbindungen ist in Tabelle 1 zusammengefasst.

**Tabelle 1: Antagonistische Wirkstärke der Verbindungen**

| Bsp. | PR-A IC₅₀ [nM] | PR-B IC₅₀ [nM] | Bsp. | PR-A IC₅₀ [nM] | PR-B IC₅₀ [nM] | Bsp. | PR-A IC₅₀ [nM] | PR-B IC₅₀ [nM] |
|---|---|---|---|---|---|---|---|---|
| 1 | 0.9 | 0.9 | 29 | 39 | n.d. | 57 | 0.8 | n.d. |
| 2 | 2.3 | 1.2 | 30 | 7.2 | n.d. | 58 | 5.7 | n.d. |
| 3 | 24 | 44 | 31 | 10.8 | n.d. | 59 | 6.9 | n.d. |
| 4 | 0.62 | 1.6 | 32 | 375 | n.d. | 60 | 0.39 | n.d. |
| 5 | 4.7 | 5 | 33 | 6.98 | n.d. | 61 | 1.6 | n.d. |
| 6 | 9.1 | 11 | 34 | 14.4 | n.d. | 62 | 94 | n.d. |
| 7 | 54 | 30 | 35 | 34 | n.d. | 63 | 4.1 | n.d. |
| 8 | 47 | 33 | 36 | 20 | n.d. | 64 | 1.1 | n.d. |
| 9 | 23 | 18 | 37 | 385 | n.d. | 65 | 2.7 | n.d. |
| 10 | 0.7 | 1.0 | 38 | 1.88 | n.d. | 66 | 35 | n.d. |
| 11 | 10 | n.d. | 39 | 9.5 | n.d. | 67 | 6.3 | n.d. |
| 12 | 1.32 | n.d. | 40 | 117 | n.d. | 68 | 16 | n.d. |
| 13 | 24 | n.d. | 41 | 4.7 | n.d. | 69 | 7.3 | n.d. |
| 14 | 0.42 | n.d. | 42 | 7.3 | n.d. | 70 | 1.7 | n.d. |
| 15 | 38 | n.d. | 43 | 10.8 | n.d. | 71 | 38 | n.d. |
| 16 | 0.67 | n.d. | 44 | 25 | n.d. | 72 | 1.4 | n.d. |
| 17 | 0.21 | n.d. | 45 | 26 | n.d. | 73 | 47 | 120 |
| 18 | 4.7 | n.d. | 46 | 4.3 | n.d. | 74 | 6.7 | 12.0 |
| 19 | 1.4 | n.d. | 47 | 346 | n.d. | 75 | 23 | 10 |
| 20 | 3.8 | n.d. | 48 | 667 | n.d. | 76 | 0.5 | n.d. |
| 21 | 51 | n.d. | 49 | 7.6 | n.d. | 77 | 140 | n.d. |
| 22 | 39 | n.d. | 50 | 15.7 | n.d. | 78 | 9.6 | n.d. |
| 23 | 86 | n.d. | 51 | 9.0 | n.d. | 79 | 0.01 | 0.07 |
| 24 | 3.7 | n.d. | 52 | 0.71 | n.d. | | | |
| 25 | 11.4 | n.d. | 53 | 29 | n.d. | | | |
| 26 | 28 | n.d. | 54 | 21 | n.d. | | | |
| 27 | 133 | n.d. | 55 | 2.5 | n.d. | | | |
| 28 | 63 | n.d. | 56 | 2.3 | n.d. | | | |

| | | | | | | | | |
|---|---|---|---|---|---|---|---|---|
| n.d. bedeutet: Nicht getestet (not determined) | | | | | | | | |

### Abortivtest an weiblichen Ratten

Die antagonistische Wirkung der erfindungsgemäßen Verbindungen wurde an trächtigen Ratten (6 Ratten pro Gruppe) an Tag 5 bis 7 post coitum unter herkömmlichen Haltungs- und Fütterungsbedingungen getestet.

Nach erfolgreicher Anpaarung, wurden die trächtigen Tiere (Vorhandensein von Spermien im Vaginalabstrich an Tag 1 der Schwangerschaft = d1 p.c.) randomisiert und auf die Behandlungs- und Kontrollgruppe aufgeteilt. Die Tiere erhielten dann subkutan oder oral je 0,15; 0,5; 1,5 oder 5 mg/kg der Testverbindung oder 1,0 ml/kg Vehikel (Benzylbenzoat/Rhizinusöl: 1 +4 [v/v]) täglich von Tag 5 bis Tag 7 (d5 - d7 p.c.).

Die Autopsie wurde an Tag 9 (d9 p.c.) durchgeführt. Als Kenngröße der Progesteronrezeptor-antagonistischen Wirkung wurde der Uterus auf das Vorhandensein von Nidationsstellen untersucht. Dabei wurde das völlige Fehlen, aber auch das Vorhandensein pathologischer, hämorrhagischer oder sonst abnormer Nidationsstellen an Tag 9 (d9 p.c.) als Abort gewertet. Die Ergebnisse der Tests sind in Tabelle 3 dargestellt. Die Testverbindung zeigt in allen Dosierung einen vollen Effekt.

**Tab. 2: Ergebnisse an der Ratte (Terminierung der frühen Schwangerschaft)**

| Testverbindung nach | Tagesdosis [mg/kg] p.o. | Abortrate [%] |
|---|---|---|
| Vehikel | | 0 |
| Beispiel 1 | 0,5 | 0 |
| 4-[(11β,17β)-17-Hydroxy-3-oxo-17-(pentafluorethyl)estra-4,9-dien-11-yl]benzamid | 1,5 | 50 |
| | 5,0 | 100 |
| Beispiel 79 | 0,5 | 100 |
| 4'-[(11β,17β)-17-Hydroxy-3-oxo-17-(pentafluor-ethyl)estra-4,9-dien-11-yl]biphenyl-4-carboxamid | 1,5 | 100 |
| | 5,0 | 100 |

### Metabolische Stabilität von 4-[(11β,17β)-17-Hydroxy-3-oxo-17-(pentafluor-ethyl)estra-4,9-dien-11-yl]benzamid und 4'-[(11β,17β)-17-Hydroxy-3-oxo-17-(pentafluor-ethyl)estra-4,9-dien-11-yl]biphenyl-4-carboxamid in humanen Lebermikrosomen (HLM)

Es wurden isolierte humane Lebermikrosomen (HLM) zur Beurteilung der metabolischen Stabilität von Verbindungen der allgemeinen Formel I eingesetzt.
Die Inkubationen wurden mit 2.4 ml HLM-lösung (0.5 mg/ml Proteingehalt), 30 µl der Testverbindung (finale Konzentration 1 µM) und 0.6 ml des Cofaktorgemisches (=NADPHgenerierendem System aus 3 IU Glucose-6-phosphatdehydrogenase, 14.6 mg Glucose-6-phosphat, 1.2 mg NADP) bei 37°C in 100 mM Phosphatpuffer bei pH 7.4 durchgeführt. Es werden zu 6 Zeitpunkten (2 - 60 min) Proben entnommen, mit gleichem Volumen Methanol gefällt und der Widerfund der eingesetzten Testsubstanzen im Überstand mittels LC-MS/MS-Analytik ermittelt. Aus der daraus ermittelten Halbwertzeit des Substanzabbaus kann man die intrinsische Clearance der Substanz im Lebermikrosomenansatz berechnen. Mit Hilfe dieser kann dann unter Zuhilfenahme verschiedener physiologischer Kenngrößen nach dem well-stirred Modell eine (metabolische) in vivo Clearance in Bezug auf Phase I Reaktionen vorhersagt werden. Die entsprechend für die Testverbindungen 4-[(11β,17β)-17-Hydroxy-3-oxo-17-(pentafluor-ethyl)estra-4,9-dien-11-yl]benzamid und 4'-[(11β,17β)-17-Hydroxy-3-oxo-17-(pentafluor-ethyl)estra-4,9-dien-11-yl]biphenyl-4-carboxamid vorhergesagte (metabolische) in vivo Clearance im Menschen war mit 0.33 L/h/kg bzw. 0,21 L/h/kg sehr niedrig.

### Bestimmung der Clearance und der Halbwertszeit nach intravenöser Applikation in Ratten

Die Bestimmung der in vivo Clearance und Halbwertzeit von Prüfsubstanzen wurde in weiblichen Ratten mit einem Körpergewicht von ca. 200-250 g durchgeführt. Hierzu wurden die Prüfsubstanzen (bei Cassettenversuch bis zu 3 verschiedene Substanzen pro Tier) in gelöster Form bei einer Dosis von 0.3-0.5 mg/kg als bolus im Volumen von 2 ml/kg intravenös (i.v.) in die Schwanzvene appliziert, wobei verträgliche Lösungsvermittler wie PEG400 und/oder Ethanol in verträglicher Menge verwendet wurden. Aus einem Polyurethan-Katheter in der Vena jugularis wurden zu den Zeitpunkten 2min, 8min, 15min, 30min, 45min, 1h, 2h, 4h, 6h, 8h, 24h ca. 0.2 mL Blutproben entnommen. Die Blutproben wurden ohne Schütteln in Lithium-Heparinröhrchen (Monovetten® von Sarstedt) aufbewahrt und für 15min bei 3000Upm zentrifugiert. Ein Aliquot von 100µL wurde dem Überstand (Plasma) entnommen und durch Zugabe von 400µL kaltem Methanol gefällt. Die gefällten Proben wurden über Nacht bei -20°C ausgefroren, danach wiederum für 15min bei 3000UpM zentrifugiert bevor 150µL des klaren Überstandes zur Konzentrationsbestimmung abgenommen wurde. Die Analytik erfolgte durch ein Agilent 1200 HPLC-System mit angeschlossener LCMS/MS Detektion.

### Berechnung der PK Parameter (via nicht linearer Regression durch PK Berechnungssoftware):

CLplasma: Gesamtplasmaclearance der Prüfsubstanz (in L*kg/h),
   wobei CLplasma = Dosis / AUCinf;
AUCinf: extrapolierte Fläche unter der Plasmakonzentration-Zeit-Kurve (in µg*h/L);
CLblood: Gesamtblutclearance der Prüfsubstanz (in L*kg/h),
   wobei (CLblood = CLplasma*Cp/Cb);
Cb/Cp: Verhältnis der Blut zu Plasma Konzentrationsverteilung der Prüfsubstanz;
T1/2: terminale Halbwertszeit der Prüfsubstanz (in h).

**Tabelle 3**

| Beispiel | CL_{blood} [L/h/kg] | t1/2 [h] |
|---|---|---|
| 79 | 0,4 | 9 |

## Patentansprüche

1. Verbindungen der Formel I 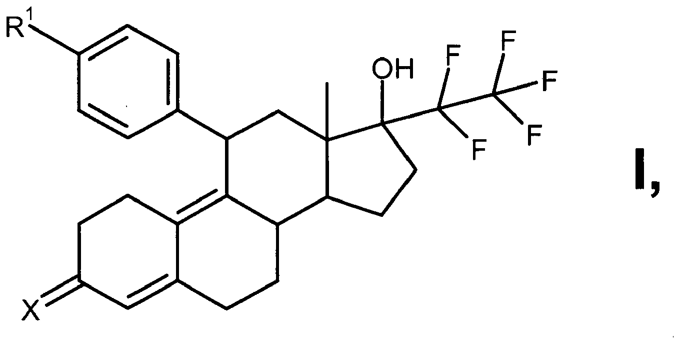 worin
R¹ entweder für einen Rest Y oder für einen mit Y substituierten Phenylring steht,
Y für eine Gruppe 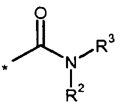 steht,
R² und R³ gleich oder verschieden sind und für Wasserstoff,
einen gegebenenfalls -N(CH₃)₂, 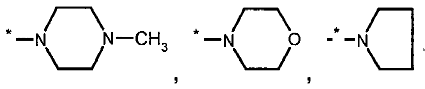 oder -NHC(O)CH₃ substituierten C₁-C₆-Alkylrest,
einen 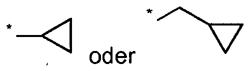 Rest
einen gegebenenfalls ein-, zwei- oder mehrfach mit Halogen (-F, -Cl, -Br, -I),-OH, -OAlkyl, -C(O)OH, -C(O)OAlkyl, -C(O)NH₂, -C(O)NHAlkyl, -C(O)NDialkyl, -C(O)NHAryl, -C(O)NHHeteroaryl, -NH₂, -NH(C₁-C₆-Alkyl), -N(C₁-C₆-Alkyl)₂, insbesondere -N(CH₃)₂, -NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂, -SO₂NHAlkyl oder-SO₂NDialkyl substituierten 6-10-gliedrigen Arylrest,
einen gegebenenfalls ein-, zwei- oder mehrfach mit den oben genannten Substituenten des 6-10-gliedrigen Arylrests substituierten 5-10-gliedrigen Heteroarylrest,
einen am Arylring gegebenenfalls ein-, zwei- oder mehrfach mit den oben genannten Substituenten des 6-10-gliedrigen Arylrests substituierten C₁-C₆-Arylalkylrest oder
einen am Heteroarylring gegebenenfalls ein-, zwei- oder mehrfach mit den oben genannten Substituenten des 6-10-gliedrigen Arylrests substituierten C₁-C₆-Heteroarylalkylrest stehen oder aber
R² und R³ gemeinsam Bestandteil eines gegebenenfalls am Kohlenstoff Alkyl-, Carboxyl-, Alkoxycarbonyl--, Alkylcarbonyl-, Aminocarbonyl-, Aryl-, insbesondere Phenyl-, Arylalkyl-, Heteroaryl-, Heteroarylalkyl-, Aminoalkyl- oder Dimethylaminoalkyl-substituierten bzw. am Stickstoff Alkyl-, Alkanoyl-, Carboxyl-, Alkoxycarbonyl-, Aryl-, insbesondere Phenyl-, Pyridinyl-, Pyrimidinyl-, Pyrazinyl-, Sulfonyl-, Benzoyl-, Alkylsulfonyl-, Arylsulfonyl-, Aminocarbonyl-, Dimethylaminocarbonyl-, Aminocarbonylalkyl, Alkylaminocarbonylalkyl, Arylalkyl-, insbesondere Phenylalkyl-, Heterocyclylalkyl- Heteroarylalkyl-, Aminoalkyl- oder 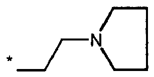 substituierten 3-10-gliedrigen Ringes sind, der gegebenenfalls Stickstoff-, Sauerstoff- oder Schwefelatome, die gegebenenfalls zum Sulfoxid oder Sulfon oxidiert sein können, enthält, wobei an den 3-10-gliedrigen Ring gegebenenfalls ein Aromat ankondensiert sein kann,
X ein Sauerstoffatom, NOR⁴ oder NNHSO₂R⁴ bedeutet und
R⁴ ausgewählt ist aus der Gruppe, umfassend Wasserstoff, C₁-C₆-Alkyl und Aryl
und ihre Salze, Solvate oder Solvate der Salze, einschließlich aller Kristallmodifikationen (* symbolisiert die Anknüpfungsstelle am Grundkörper).

2. Verbindung gemäß Anspruch 1 der allgemeinen Formel V: 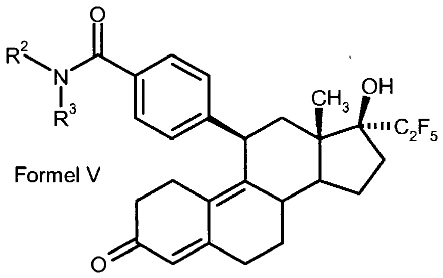 worin
R² Wasserstoff, C₁-C₄-Alkyl (insbesondere Methyl, Ethyl, Cyclopropyl oder Cyclopropylmethyl), -(CH₂)ₖ-N(CH₃)₂ mit k = 2 oder 3, -CH₂-CH₂-NH-CO-CH₃ und -(CH₂)ₖ-R⁵ mit k = 2 oder 3 und 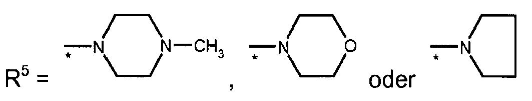 und
R³ Wasserstoff oder C₁-C₄-Alkyl (insbesondere Methyl oder Ethyl) bedeutet und ihre Salze, Solvate öder Solvate der Salze, einschließlich aller Kristallmodifikationen..

3. Verbindung gemäß Anspruch 1 der allgemeinen Formel VI 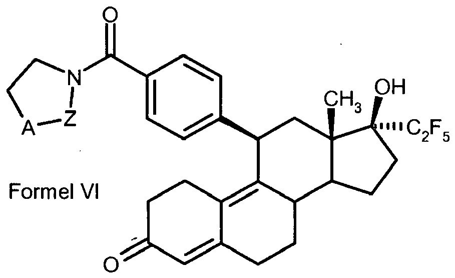 worin
Z -CH₂- oder -CH₂-CH₂-,
A Sauerstoff, -CHR⁶- oder -NR⁷- und
R⁶, R⁷ Wasserstoff,
C₁-C₄-Alkyl,
einen gegebenenfalls ein-, zwei- oder mehrfach mit Halogen (-F, -Cl, -Br, -I), -OH,-O-Alkyl, -CO₂H, -CO₂-Alkyl, -C(O)NH₂, -C(O)NHAlkyl, -C(O)NDialkyl, -C(O)NHAryl, -C(O)NHHeteroaryl, -NH₂, -NH(C₁-C₆-Alkyl), -N(C₁-C₆-Alkyl)₂, insbesondere-N(CH₃)₂, -NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂, -SO₂NHAlkyl oder -SO₂NDialkyl substituierten-(CH₂)_{I}-Arylrest mit I = 0, 1 oder 2,
einen gegebenenfalls ein-, zwei- oder mehrfach mit Halogen (-F, -Cl, -Br, -I), -OH, - O-Alkyl, -CO₂H, -CO₂-Alkyl, -C(O)NH₂, -C(O)NHAlkyl, -C(O)NDialkyl, -C(O)NHAryl, -C(O)NHHeteroaryl, -NH₂, -NH(C₁-C₆-Alkyl), -N(C₁-C₆-Alkyl)₂, insbesondere-N(CH₃)₂, -NHC(O)Alkyl, -NO₂, -N₃, -CN, -C₁-C₆-Alkyl, -C₁-C₆-Perfluor-Alkyl, -C₁-C₆-Acyl, -C₁-C₆-Acyloxy, -SO₂NH₂, -SO₂NHAlkyl oder -SO₂NDialkyl substituierten-(CH₂)_{I}-Heteroarylrest mit bis zu zwei Heteroatomen und I = 0, 1 oder 2 oder
-COR⁸ und
R⁸ -OH, -(C₁-C₄-Alkyl), -Aryl, -O-C₁-C₄-Alkyl, -NH-(C₁-C₄-Alkyl), -N(CH₃)₂, -SO₂-(C₁-C₄-Alkyl) bedeuten
und ihre Salze, Solvate oder Solvate der Salze, einschließlich aller Kristallmodifikationen..

4. Verbindung gemäß Anspruch 3 worin
R⁶ Wasserstoff, Phenyl, -CO₂H, -CO₂CH₃ und
R⁷ Wasserstoff, -CH₃, -(CH₂)_{I}-Phenyl mit I = 0, 1 oder 2, 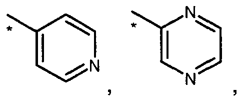 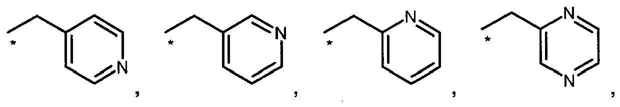 -CH₂-CH₂-N(CH₃)₂, 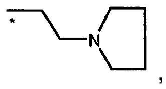 -CO-CH₃, -CO-Phenyl, -CO₂CH₃, -CO₂C(CH₃)₃, -CO-NH-CH₃, -CO-N(CH₃)₂; -SO₂-CH₃, -CH₂CO-NH-CH₃ bedeutet und ihre Salze, Solvate oder Solvate der Salze, einschließlich aller Kristallmodifikationen..

5. Verbindung gemäß Anspruch 1 der allgemeinen Formel VII: 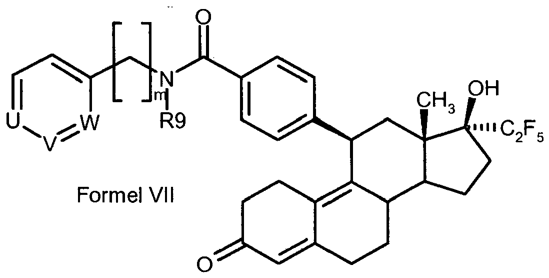 worin
m 0, 1 oder 2,
R⁹ Wasserstoff oder -C₁-C₄-Alkyl, insbesondere Methyl oder Ethyl,
U, V und W unabhängig voneinander -CH=, -CR¹⁰= oder -N= bedeuten und -CR¹⁰= oder -N= unabhängig von der Postition im aromatischen Ring jedoch höchstens einmal vorkommen und
R¹⁰ -O-(C₁-C₄-Alkyl), Halogen, -COR¹¹ mit R¹¹ = -OH, -NH₂ oder -O-(C₁-C₄-Alkyl), -SO₂-NH₂; -NH-CO-(C₁-C₄-Alkyl), oder -CO-NH-Aryl bedeutet und ihre Salze, Solvate oder Solvate der Salze, einschließlich aller Kristallmodifikationen..

6. Verbindung gemäß Anspruch 5 worin
R⁹ Wasserstoff, Methyl oder Ethyl und
R¹⁰ -O-CH₃, -Cl, -CO₂H, -CO₂CH₃, -CO-NH₂, -SO₂-NH₂; -NH-CO-CH₃ oder-CO-NH-Phenyl bedeutet und ihre Salze, Solvate oder Solvate der Salze, einschließlich aller Kristallmodifikationen..

7. Die Verbindungen gemäß einem der vorstehenden Ansprüche, nämlich
4-[((11β,17β)-17-Hydroxy-3-oxo-17-(pentafluorethyl)estra-4,9-dien-11-yl]benzamid
4-[((11β,17β)-17-Hydroxy-3-oxo-17-(pentafluorethyl)estra-4,9-dien-11-yl]-N,N-dimethylbenzamid
(11β,17β)-17-Hydroxy-11-{4-[(4-methylpiperazin-1-yl)carbonyl]phenyl}-17-(pentafluorethyl)estra-4,9-dien-3-on
tert-Butyl-4-{4-[(11β,17β)-17-hydroxy-3-oxo-17-(pentafluorethyl)estra-4,9-dien-11-yl]benzoyl}piperazin-1-carboxylat
(11β,17β)-17-Hydroxy-17-(pentafluorethyl)-11-[4-(piperidin-1-ylcarbonyl)phenyl]estra-4,9-dien-3-on
Methyl-1-{4-[(11β,17β)-17-hydroxy-3-oxo-17-(pentafluorethyl)estra-4,9-dien-11-yl]benzoyl}piperidin-4-carboxylat
N-[2-(Dimethylamino)ethyl]-4-[(11β,17β)-17-hydroxy-3-oxo-17-(pentafluorethyl)estra-4,9-dien-11-yl]benzamid
N-[3-(Dimethylamino)propyl]-4-[(11β,17β)-17-hydroxy-3-oxo-17-(pentafluorethyl)estra-4,9-dien-11-yl]benzamid
N-[2-(Dimethylamino)ethyl]-4-[(11β,17β)-17-hydroxy-3-oxo-17-(pentafluorethyl)estra-4,9-dien-11-yl]-N-methylbenzamid
Methyl-2-({4-[(11β,17β)-17-hydroxy-3-oxo-17-(pentafluorethyl)estra-4,9-dien-11-yl]benzoyl}amino)benzoat
4-[((11β,17β)-17-Hydroxy-3-oxo-17-(pentafluorethyl)estra-4,9-dien-11-yl]-N-(pyridin-2-yl)benzamid
4-[((11β,17β)-17-Hydroxy-3-oxo-17-(pentafluorethyl)estra-4,9-dien-11-yl]-N-(pyridin-3-yl)benzamid
4-[((11β,17β)-17-Hydroxy-3-oxo-17-(pentafluorethyl)estra-4,9-dien-11-yl]-N-[3-(morpholin-4-yl)propyl]benzamid
4-[((11β,17β)-17-Hydroxy-3-oxo-17-(pentafluorethyl)estra-4,9-dien-11-yl]-N-(pyridin-4-yl)benzamid
(11β,17β)-17-Hydroxy-17-(pentafluorethyl)-11-[4-(pyrrolidin-1-ylcarbonyl)phenyl]estra-4,9-dien-3-on
4-[((11β,17β)-17-Hydroxy-3-oxo-17-(pentafluorethyl)estra-4,9-dien-11-yl]-N-(2-phenylethyl)benzamid
4-[((11β,17β)-17-Hydroxy-3-oxo-17-(pentafluorethyl)estra-4,9-dien-11-yl]-N-(2-phenylpropan-2-yl)benzamid
4-[((11β,17β)-17-Hydroxy-3-oxo-17-(pentafluorethyl)estra-4,9-dien-11-yl]-N-(4-sulfamoylbenzyl)benzamid
N-Ethyl-4-[(11β,17β)-17-hydroxy-3-oxo-17-(pentafluorethyl)estra-4,9-dien-11-yl]benzamid
4-[((11β,17β)-17-Hydroxy-3-oxo-17-(pentafluorethyl)estra-4,9-dien-11-yl]-N-(pyridin-2-ylmethyl)benzamid
4-[((11β,17β)-17-Hydroxy-3-oxo-17-(pentafluorethyl)estra-4,9-dien-11-yl]-N-(pyridin-4-ylmethyl)benzamid
4-[((11β,17β)-17-Hydroxy-3-oxo-17-(pentafluorethyl)estra-4,9-dien-11-yl]-N-phenylbenzamid
4-[((11β,17β)-17-Hydroxy-3-oxo-17-(pentafluorethyl)estra-4,9-dien-11-yl]-N-(2-methoxyphenyl)benzamid
4-[((11β,17β)-17-Hydroxy-3-oxo-17-(pentafluorethyl)estra-4,9-dien-11-yl]-N-(3-methoxyphenyl)benzamid
4-[((11β,17β)-17-Hydroxy-3-oxo-17-(pentafluorethyl)estra-4,9-dien-11-yl]-N-(4-methoxyphenyl)benzamid
N-(4-Chlorphenyl)-4-[(11β,17β)-17-hydroxy-3-oxo-17-(pentafluorethyl)estra-4,9-dien-11-yl]benzamid
N,N-Diethyl-4-[(11β,17β)-17-hydroxy-3-oxo-17-(pentafluorethyl)estra-4,9-dien-11-yl]benzamid
4-[((11β,17β)-17-Hydroxy-3-oxo-17-(pentafluorethyl)estra-4,9-dien-11-yl]-N-methyl-N-phenylbenzamid
(11β,17β)-17-Hydroxy-11-[4-(morpholin-4-ylcarbonyl)phenyl]-17-(pentafluorethyl)estra-4,9-dien-3-on
(11β,17β)-17-Hydroxy-17-(pentafluorethyl)-11-{4-[(4-phenylpiperazin-1-yl)carbonyl]phenyl}estra-4,9-dien-3-on
4-[((11β,17β)-17-Hydroxy-3-oxo-17-(pentafluorethyl)estra-4,9-dien-11-yl]-N-methyl-N-(pyridin-2-ylmethyl)benzamid
4-[((11β,17β)-17-Hydroxy-3-oxo-17-(pentafluorethyl)estra-4,9-dien-11-yl]-N-methyl-N-(pyridin-3-ylmethyl)benzamid
4-[((11β,17β)-17-Hydroxy-3-oxo-17-(pentafluorethyl)estra-4,9-dien-11-yl]-N-methyl-N-(pyridin-4-ylmethyl)benzamid
N-Benzyl-4-[(11β,17β)-17-hydroxy-3-oxo-17-(pentafluorethyl)estra-4,9-dien-11-yl]benzamid
4-[((11β,17β)-17-Hydroxy-3-oxo-17-(pentafluorethyl)estra-4,9-dien-11-yl]-N-(4-methoxybenzyl)benzamid
N-(4-Chlorbenzyl)-4-[(11β,17β)-17-hydroxy-3-oxo-17-(pentafluorethyl)estra-4,9-dien-11-yl]benzamid
N-(2-Acetamidoethyl)-4-[(11β,17β)-17-hydroxy-3-oxo-17-(pentafluorethyl)estra-4,9-dien-11-yl]benzamid
Methyl-4-({4-[(11β,17β)-17-hydroxy-3-oxo-17-(pentafluorethyl)estra-4,9-dien-11-yl]benzoyl}amino)benzoat
(11β,17β)-11-[4-(3,4-Dihydroisochinolin-2(1H)-ylcarbonyl)phenyl]-17-hydroxy-17-(pentafluorethyl)estra-4,9-dien-3-on
(11β,17β)-11-{4-[(4-Benzylpiperazin-1-yl)carbonyl]phenyl}-17-hydroxy-17-(pentafluorethyl)estra-4,9-dien-3-on
N-(4-Carbamoylphenyl)-4-[(11β,17β)-17-hydroxy-3-oxo-17-(pentafluorethyl)estra-4,9-dien-11-yl]benzamid
N-Cyclopropyl-4-[(11β,17β)-17-hydroxy-3-oxo-17-(pentafluorethyl)estra-4,9-dien-11-yl]benzamid
4-[((11β,17β)-17-Hydroxy-3-oxo-17-(pentafluorethyl)estra-4,9-dien-11-yl]-N-methyl-N-(2-phenylethyl)benzamid
(11β,17β)-17-Hydroxy-17-(pentafluorethyl)-11-(4-{[4-(2-phenylethyl)piperazin-1-yl]carbonyl}phenyl)estra-4,9-dien-3-on
(11β,17β)-17-Hydroxy-17-(pentafluorethyl)-11-(4-{[4-(pyridin-4-yl)piperazin-1-yl]carbonyl}phenyl)estra-4,9-dien-3-on
(11β,17β)-17-Hydroxy-17-(pentafluorethyl)-11-(4-{[4-(pyrazin-2-yl)piperazin-1-yl]carbonyl}phenyl)estra-4,9-dien-3-on
(11β,17β)-11-[4-({4-[2-(Dimethylamino)ethyl]piperazin-1-yl}carbonyl)phenyl]-17-hydroxy-17-(pentafluorethyl)estra-4,9-dien-3-on
(11β,17β)-17-Hydroxy-17-(pentafluorethyl)-11-[4-({4-[2-(pyrrolidin-1-yl)ethyl]piperazin-1-yl}carbonyl)phenyl]estra-4,9-dien-3-on
(11β,17β)-17-Hydroxy-17-(pentafluorethyl)-11-(4-{[4-(pyridin-4-ylmethyl)piperazin-1-yl]carbonyl}phenyl)estra-4,9-dien-3-on
4-[((11β,17β)-17-Hydroxy-3-oxo-17-(pentafluorethyl)estra-4,9-dien-11-yl]-N-methyl-N-[2-(pyridin-2-yl)ethyl]benzamid
N-Benzyl-4-[(11β,17β)-17-hydroxy-3-oxo-17-(pentafluorethyl)estra-4,9-dien-11-yl]-N-methylbenzamid
N-(4-Acetamidophenyl)-4-[(11β,17β)-17-hydroxy-3-oxo-17-(pentafluorethyl)estra-4,9-dien-11-yl]benzamid
4-[((11β,17β)-17-Hydroxy-3-oxo-17-(pentafluorethyl)estra-4,9-dien-11-yl]-N-(3-methoxybenzyl)benzamid
4-[((11β,17β)-17-Hydroxy-3-oxo-17-(pentafluorethyl)estra-4,9-dien-11-yl]-N-[2-(pyrrolidin-1-yl)ethyl]benzamid
4-[((11β,17β)-17-Hydroxy-3-oxo-17-(pentafluorethyl)estra-4,9-dien-11-yl]-N-[2-(pyridin-3-yl)ethyl]benzamid
N-(3-Chlorbenzyl)-4-[(11β,17β)-17-hydroxy-3-oxo-17-(pentafluorethyl)estra-4,9-dien-11-yl]benzamid
Methyl-3-({4-[(11β,17β)-17-hydroxy-3-oxo-17-(pentafluorethyl)estra-4,9-dien-11-yl]benzoyl}amino)benzoat
4-[((11β,17β)-17-Hydroxy-3-oxo-17-(pentafluorethyl)estra-4,9-dien-11-yl]-N-methyl-N-[2-(pyridin-4-yl)ethyl]benzamid
N-(Cyclopropylmethyl)-4-[(11β,17β)-17-hydroxy-3-oxo-17-(pentafluorethyl)estra-4,9-d ien-11-yl]benzamid
4-[((11β,17β)-17-Hydroxy-3-oxo-17-(pentafluorethyl)estra-4,9-dien-11-yl]-N-[4-(phenylcarbamoyl)phenyl]benzamid
4-[((11β,17β)-17-Hydroxy-3-oxo-17-(pentafluorethyl)estra-4,9-dien-11-yl]-N-[2-(pyridin-2-yl)ethyl]benzamid
4-[((11β,17β)-17-Hydroxy-3-oxo-17-(pentafluorethyl)estra-4,9-dien-11-yl]-N-[2-(4-methylpiperazin-1-yl)ethyl]benzamid
(11β,17β)-17-Hydroxy-17-(pentafluorethyl)-11-{4-[(4-phenylpiperidin-1-yl)carbonyl]phenyl}estra-4,9-dien-3-on
4-[((11β,17β)-17-Hydroxy-3-oxo-17-(pentafluorethyl)estra-4,9-dien-11-yl]-N-[2-(pyridin-4-yl)ethyl]benzamid
(11β,17β)-11-{4-[(4-Benzoylpiperazin-1-yl)carbonyl]phenyl}-17-hydroxy-17-(pentafluorethyl)estra-4,9-dien-3-on
4-{4-[(11β,17β)-17-Hydroxy-3-oxo-17-(pentafluorethyl)estra-4,9-dien-11-yl]benzoyl}-N,N-dimethylpiperazin-1-carboxamid
(11β,17β)-17-Hydroxy-11-(4-{[4-(methylsulfonyl)piperazin-1-yl]carbonyl}phenyl)-17-(pentafluorethyl)estra-4,9-dien-3-on
(11β,17β)-17-Hydroxy-17-(pentafluorethyl)-11-(4-{[4-(pyridin-2-ylmethyl)piperazin-1-yl]carbonyl}phenyl)estra-4,9-dien-3-on
(11β,17β)-17-Hydroxy-17-(pentafluorethyl)-11-(4-{[4-(pyridin-3-ylmethyl)piperazin-1-yl]carbonyl}phenyl)estra-4,9-dien-3-on
Methyl-4-{4-[(11β,17β)-17-hydroxy-3-oxo-17-(pentafluorethyl)estra-4,9-dien-11-yl]benzoyl}piperazin-1-carboxylat
2-((4-{4-[(11β,17β)-17-Hydroxy-3-oxo-17-(pentafluorethyl)estra-4,9-dien-11-yl]benzoyl}piperazin-1-yl)-N-methylacetamid
4-[((11β,17β)-17-Hydroxy-3-oxo-17-(pentafluorethyl)estra-4,9-dien-11-yl]-N-methyl-N-[2-(pyridin-3-yl)ethyl]benzamid
(11β,17β)-17-Hydroxy-17-(pentafluorethyl)-11-[4-(piperazin-1-ylcarbonyl)phenyl]estra-4,9-dien-3-on
(11β,17β)-11-{4-[(4-Acetylpiperazin-1-yl)carbonyl]phenyl}-17-hydroxy-17-(pentafluorethyl)estra-4,9-dien-3-on
1-{4-[(11β,17β)-17-Hydroxy-3-oxo-17-(pentafluorethyl)estra-4,9-dien-11-yl]benzoyl}piperidin-4-carbonsäure
2-({4-[(11β,17β)-17-Hydroxy-3-oxo-17-(pentafluorethyl)estra-4,9-dien-11-yl]benzoyl}amino)benzoesäure
3-({4-[(11β,17β)-17-Hydroxy-3-oxo-17-(pentafluorethyl)estra-4,9-dien-11-yl]benzoyl}amino)benzoesäure
4-({4-[(11β,17β)-17-Hydroxy-3-oxo-17-(pentafluorethyl)estra-4,9-dien-11-yl]benzoyl}amino)benzoesäure
4'-[(11β,17β)-17-Hydroxy-3-oxo-17-(pentafluorethyl)estra-4,9-dien-11-yl]biphenyl-4-carboxamid
und ihre Salze, Solvate oder Solvate der Salze, einschließlich aller Kristallmodifikationen.

8. Eine Verbindungen gemäß einem der vorstehenden Ansprüche zur Behandlung und Prophylaxe von Krankheiten.

9. Eine Verbindung gemäß einem der Ansprüche 1 - 7 zur Behandlung und Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

10. Verwendung einer Verbindung nach einem der Ansprüche 1 - 7 zur Herstellung eines Arzneimittels zur Behandlung und/oder Prophylaxe von Krankheiten.

11. Verwendung einer Verbindung nach einem der Ansprüche 1 - 7 zur Herstellung eines Arzneimittels zur Behandlung und/oder Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

12. Arzneimittel enthaltend eine Verbindung, wie in einem der Ansprüche 1 - 7 definiert, in Kombination mit einem weiteren Wirkstoff.

13. Arzneimittel enthaltend eine Verbindung, wie in einem der Ansprüche 1 - 7 definiert, in Kombination mit einem inerten, nichttoxischen, pharmazeutisch geeigneten Hilfsstoff.

14. Arzneimittel nach Anspruch 13 zur Behandlung und/oder Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

## Claims

1. Compounds of formula I 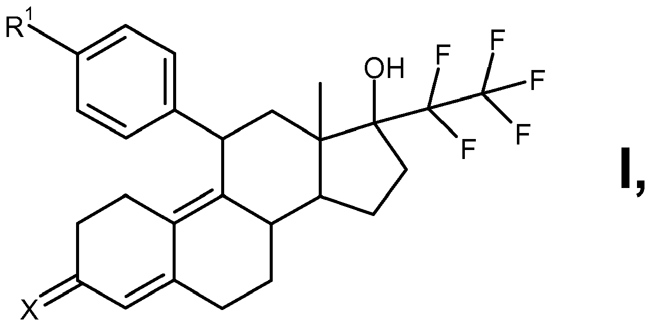 in which
R¹ stands either for a residue Y or for a phenyl ring substituted with Y,
Y stands for a group 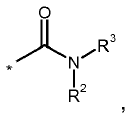
R² and R³ are identical or different and stand for
hydrogen,
an optionally -N(CH₃)₂, 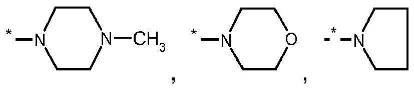 or -NHC(O)CH₃ substituted C₁-C₆-alkyl residue,
a 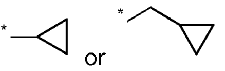 residue
a 6-10-membered aryl residue optionally substituted one, two or more times with halogen (-F, -Cl, -Br, -I), -OH, -Oalkyl, -C(O)OH, - C(O)O-alkyl, -C(O)NH₂, -C(O)NH-alkyl, -C(O)N-dialkyl, -C(O)NH-aryl, -C(O)NH-heteroaryl, -NH₂, -NH(C₁-C₆-alkyl), -N(C₁-C₆-alkyl)₂, in particular -N(CH₃)₂, -NHC(O)alkyl, -NO₂, -N₃, -CN, -C₁-C₆-alkyl,-C₁-C₆-perfluoro-alkyl, -C₁-C₆-acyl, -C₁-C₆-acyloxy, -SO₂NH₂,-SO₂NH-alkyl or -SO₂N-dialkyl,
a 5-10-membered heteroaryl residue optionally substituted one, two or more times with the aforementioned substituents of the 6-10-membered aryl residue,
a C₁-C₆-aralkyl residue optionally substituted on the aryl ring one, two or more times with the aforementioned substituents of the 6-10-membered aryl residue or
a C₁-C₆-heteroarylalkyl residue optionally substituted on the heteroaryl ring one, two or more times with the aforementioned substituents of the 6-10-membered aryl residue or else
R² and R³ are together a constituent of a 3-10-membered ring optionally alkyl-, carboxyl-, alkoxycarbonyl-, alkylcarbonyl-, aminocarbonyl-, aryl-, in particular phenyl-, aralkyl-, heteroaryl-, heteroarylalkyl-, aminoalkyl-or dimethylaminoalkyl-substituted on the carbon or alkyl-, alkanoyl-, carboxyl-, alkoxycarbonyl-, aryl-, in particular phenyl-, pyridinyl-, pyrimidinyl-, pyrazinyl-, sulphonyl-, benzoyl-, alkylsulphonyl-, arylsulphonyl-, aminocarbonyl-, dimethylaminocarbonyl-, aminocarbonylalkyl-, alkylaminocarbonylalkyl-, aralkyl-, in particular phenylalkyl-, heterocyclylalkyl- heteroarylalkyl-, aminoalkyl- or 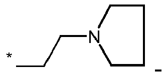 substituted on the nitrogen, which optionally contains nitrogen, oxygen or sulphur atoms, which can optionally be oxidized to the sulphoxide or sulphone, wherein optionally an aromatic can be condensed onto the 3-10-membered ring,
X denotes an oxygen atom, NOR⁴ or NNHSO₂R⁴ and
R⁴ is selected from the group comprising hydrogen, C₁-C₆-alkyl and aryl
and their salts, solvates or solvates of the salts, including all crystal modifications (* represents the site of attachment on the parent substance).

2. Compound according to Claim 1 of general formula V: 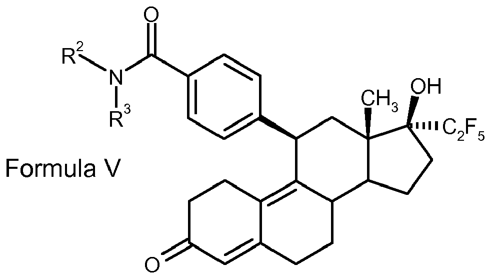 in which
R² denotes hydrogen, C₁-C₄-alkyl (in particular methyl, ethyl, cyclopropyl or cyclopropylmethyl), -(CH₂)ₖ-N(CH₃)₂ with k = 2 or 3, -CH₂-CH₂-NH-CO-CH₃ and -(CH₂)ₖ-R⁵ with k = 2 or 3 and 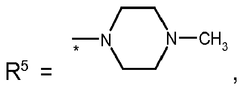 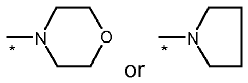 and
R³ denotes hydrogen or C₁-C₄-alkyl (in particular methyl or ethyl) and their salts, solvates or solvates of the salts, including all crystal modifications.

3. Compound according to Claim 1 of general formula VI 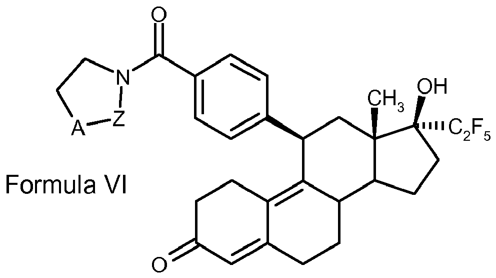 in which
Z denotes -CH₂- or -CH₂-CH₂-,
A denotes oxygen, -CHR⁶- or -NR⁷- and
R⁶, R⁷ denote hydrogen,
C₁-C₄-alkyl,
a -(CH₂)_{I}-aryl residue with I = 0, 1 or 2, optionally substituted one, two or more times with halogen (-F, -Cl, -Br, -I), -OH, -O-alkyl, -CO₂H, -CO₂-alkyl, -C(O)NH₂, -C(O)NH-alkyl, -C(O)N-dialkyl, -C(O)NH-aryl, -C(O)NH-heteroaryl, -NH₂, -NH(C₁-C₆-alkyl), -N(C₁-C₆-alkyl)₂, in particular - N(CH₃)₂, -NHC(O)alkyl, -NO₂, -N₃, -CN, -C₁-C₆-alkyl, -C₁-C₆-perfluoroalkyl, -C₁-C₆-acyl, -C₁-C₆-acyloxy, -SO₂NH₂, -SO₂NH-alkyl or -SO₂N-dialkyl,
a -(CH₂)ₗ-heteroaryl residue with up to two heteroatoms and l = 0, 1 or 2, optionally substituted one, two or more times with halogen (-F, -Cl, -Br, - I), -OH, -O-alkyl, -CO₂H, -CO₂-alkyl, -C(O)NH₂, -C(O)NH-alkyl, -C(O)N-dialkyl, -C(O)NH-aryl, -C(O)NH-heteroaryl, -NH₂, -NH(C₁-C₆-alkyl), -N(C₁-C₆-alkyl)₂, in particular -N(CH₃)₂, -NHC(O)alkyl, -NO₂, -N₃, -CN, -C₁-C₆-alkyl, -C₁-C₆-perfluoro-alkyl, -C₁-C₆-acyl, -C₁-C₆-acyloxy, -SO₂NH₂, - SO₂NH-alkyl or -SO₂N-dialkyl or
-COR⁸ and
R⁸ denotes -OH, -(C₁-C₄-alkyl), -aryl, -O-C₁-C₄-alkyl, -NH-(C₁-C₄-alkyl), -N(CH₃)₂, -SO₂-(C₁-C₄-alkyl)
and their salts, solvates or solvates of the salts, including all crystal modifications.

4. Compound according to Claim 3 in which
R⁶ denotes hydrogen, phenyl, -CO₂H, -CO₂CH₃ and
R⁷ denotes hydrogen, -CH₃, -(CH₂)ₗ-phenyl with l = 0, 1 or 2, 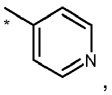 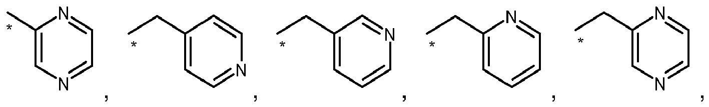 -CH₂-CH₂-N(CH₃)₂, 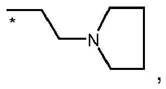 -CO-CH₃, -CO-phenyl, - CO₂CH₃, -CO₂C(CH₃)₃, -CO-NH-CH₃, -CO-N(CH₃)₂; -SO₂-CH₃, - CH₂CO-NH-CH₃ and their salts, solvates or solvates of the salts, including all crystal modifications.

5. Compound according to Claim 1 of general formula VII: 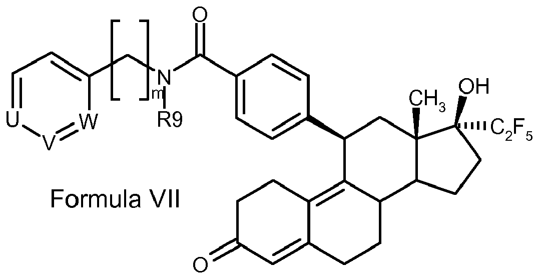 in which
m denotes 0, 1 or 2,
R⁹ denotes hydrogen or -C₁-C₄-alkyl, in particular methyl or ethyl,
U, V and W independently of one another denote -CH=, -CR¹⁰= or -N=,
and -CR¹⁰= or -N=, regardless of the position in the aromatic ring, are present at most once and
R¹⁰ denotes -O-(C₁-C₄-alkyl), halogen, -COR¹¹ with R¹¹ = -OH, -NH₂ or-O-(C₁-C₄-alkyl), -SO₂-NH₂; -NH-CO-(C₁-C₄-alkyl), or - CO-NH-aryl and their salts, solvates or solvates of the salts, including all crystal modifications.

6. Compound according to Claim 5 in which
R⁹ denotes hydrogen, methyl or ethyl and
R¹⁰ denotes -O-CH₃, -Cl, -CO₂H, -CO₂CH₃, -CO-NH₂, -SO₂-NH₂; -NH-CO-CH₃ or -CO-NH-phenyl and their salts, solvates or solvates of the salts, including all crystal modifications.

7. Compounds according to one of the preceding claims, namely
4-[((11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N,N-dimethylbenzamide
(11β,17β)-17-hydroxy-11-{4-[(4-methylpiperazin-1-yl)carbonyl]phenyl}-17-(pentafluoroethyl)estra-4,9-dien-3-one
tert-butyl-4-{4-[(11β,17β)-17-hydroxy-3-oxo-17-(pentafluoroethyl)estra-4,9-dien-11-yl]benzoyl}piperazine-1-carboxylate
(11β,17β)-17-hydroxy-17-(pentafluoroethyl)-11-[4-(piperidin-1-ylcarbonyl)phenyl]estra-4,9-dien-3-one
methyl-1-{4-[(11β,17β)-17-hydroxy-3-oxo-17-(pentafluoroethyl)estra-4,9-dien-11-yl]benzoyl}piperidine-4-carboxylate
N-[2-(dimethylamino)ethyl]-4-[(11β,17β)-17-hydroxy-3-oxo-17-(pentafluoroethyl)estra-4,9-dien-11-yl]benzamide
N-[3-(dimethylamino)propyl]-4-[(11β,17β)-17-hydroxy-3-oxo-17-(pentafluoroethyl)estra-4,9-dien-11-yl]benzamide
N-[2-(dimethylamino)ethyl]-4-[(11β,17β)-17-hydroxy-3-oxo-17-(pentafluoroethyl)estra-4,9-dien-11-yl]-N-methylbenzamide
methyl-2-({4-[(11β,17β)-17-hydroxy-3-oxo-17-(pentafluoroethyl)estra-4,9-dien-11-yl]benzoyl}amino)benzoate
4-[((11β,17β)-17-hydroxy-3-oxo-17-(pentafluoroethyl)estra-4,9-dien-11-yl]-N-(pyridin-2-yl)benzamide
4-[((11β,17β)-17-hydroxy-3-oxo-17-(pentafluoroethyl)estra-4,9-dien-11-yl]-N-(pyridin-3-yl)benzamide
4-[((11β,17β)-17-hydroxy-3-oxo-17-(pentafluoroethyl)estra-4,9-dien-11-yl]-N-[3-(morpholin-4-yl)propyl]benzamide
4-[((11β,17β)-17-hydroxy-3-oxo-17-(pentafluoroethyl)estra-4,9-dien-11-yl]-N-(pyridin-4-yl)benzamide
(11β,17β)-17-hydroxy-17-(pentafluoroethyl)-11-[4-(pyrrolidin-1-ylcarbonyl)phenyl]estra-4,9-dien-3-one
4-[((11β,17β)-17-hydroxy-3-oxo-17-(pentafluoroethyl)estra-4,9-dien-11-yl]-N-(2-phenylethyl)benzamide
4-[((11β,17β)-17-hydroxy-3-oxo-17-(pentafluoroethyl)estra-4,9-dien-11-yl]-N-(2-phenylpropan-2-yl)benzamide
4-[((11β,17β)-17-hydroxy-3-oxo-17-(pentafluoroethyl)estra-4,9-dien-11-yl]-N-(4-sulphamoylbenzyl)benzamide
N-ethyl-4-[(11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N-(pyridin-2-ylmethyl)benzamide
4-[((11β,17β)-17-hydroxy-3-oxo-17-(pentafluoroethyl)estra-4,9-dien-11-yl]-N-(pyridin-4-ylmethyl)benzamide
4-[((11β,17β)-17-hydroxy-3-oxo-17-(pentafluoroethyl)estra-4,9-dien-11-yl]-N-phenylbenzamide
4-[((11β,17β)-17-hydroxy-3-oxo-17-(pentafluoroethyl)estra-4,9-dien-11-yl]-N-(2-methoxyphenyl)benzamide
4-[((11β,17β)-17-hydroxy-3-oxo-17-(pentafluoroethyl)estra-4,9-dien-11-yl]-N-(3-methoxyphenyl)benzamide
4-[((11β,17β)-17-hydroxy-3-oxo-17-(pentafluoroethyl)estra-4,9-dien-11-yl]-N-(4-methoxyphenyl)benzamide
N-(4-chlorophenyl)-4-[(11β,17β)-17-hydroxy-3-oxo-17-(pentafluoroethyl)estra-4,9-dien-11-yl]benzamide
N,N-diethyl-4-[(11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N-methyl-N-phenylbenzamide
(11β,17β)-17-hydroxy-11-[4-(morpholin-4-ylcarbonyl)phenyl]-17-(pentafluoroethyl)estra-4,9-dien-3-one
(11β,17β)-17-hydroxy-17-(pentafluoroethyl)-11-{4-[(4-phenylpiperazin-1-yl)carbonyl]phenyl}estra-4,9-dien-3-one
4-[((11β,17β)-17-hydroxy-3-oxo-17-(pentafluoroethyl)estra-4,9-dien-11-yl]-N-methyl-N-(pyridin-2-ylmethyl)benzamide
4-[((11β,17β)-17-hydroxy-3-oxo-17-(pentafluoroethyl)estra-4,9-dien-11-yl]-N-methyl-N-(pyridin-3-ylmethyl)benzamide
4-[((11β,17β)-17-hydroxy-3-oxo-17-(pentafluoroethyl)estra-4,9-dien-11-yl]-N-methyl-N-(pyridin-4-ylmethyl)benzamide
N-benzyl-4-[(11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N-(4-methoxybenzyl)benzamide
N-(4-chlorobenzyl)-4-[(11β,17β)-17-hydroxy-3-oxo-17-(pentafluoroethyl)estra-4,9-dien-11-yl]benzamide
N-(2-acetamidoethyl)-4-[(11β,17β)-17-hydroxy-3-oxo-17-(pentafluoroethyl)estra-4,9-dien-11-yl]benzamide
methyl-4-({4-[(11β,17β)-17-hydroxy-3-oxo-17-(pentafluoroethyl)estra-4,9-dien-11-yl]benzoyl}amino)benzoate
(11β,17β)-11-[4-(3,4-dihydroisoquinolin-2(1H)-ylcarbonyl)phenyl]-17-hydroxy-17-(pentafluoroethyl)estra-4,9-dien-3-one
(11β,17β)-11-{4-[(4-benzylpiperazin-1-yl)carbonyl]phenyl}-17-hydroxy-17-(pentafluoroethyl)estra-4,9-dien-3-one
N-(4-carbamoylphenyl)-4-[(11β,17β)-17-hydroxy-3-oxo-17-(pentafluoroethyl)estra-4,9-dien-11-yl]benzamide
N-cyclopropyl-4-[(11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N-methyl-N-(2-phenylethyl)benzamide
(11β,17β)-17-hydroxy-17-(pentafluoroethyl)-11-(4-{[4-(2-phenylethyl)piperazin-1-yl]carbonyl}phenyl)estra-4,9-dien-3-one
(11β,17β)-17-hydroxy-17-(pentafluoroethyl)-11-(4-{[4-(pyridin-4-yl)piperazin-1-yl]carbonyl}phenyl)estra-4,9-dien-3-one
(11β,17β)-17-hydroxy-17-(pentafluoroethyl)-11-(4-{[4-(pyrazin-2-yl)piperazin-1-yl]carbonyl}phenyl)estra-4,9-dien-3-one
(11 β,17β)-11-[4-({4-[2-(dimethylamino)ethyl]piperazin-1-yl}carbonyl)phenyl]-17-hydroxy-17-(pentafluoroethyl)estra-4,9-dien-3-one
(11β,17β)-17-hydroxy-17-(pentafluoroethyl)-11-[4-({4-[2-(pyrrolidin-1-yl)ethyl]piperazin-1-yl}carbonyl)phenyl]estra-4,9-dien-3-one
(11β,17β)-17-hydroxy-17-(pentafluoroethyl)-11-(4-{[4-(pyridin-4-ylmethyl)piperazin-1-yl]carbonyl}phenyl)estra-4,9-dien-3-one
4-[((11β,17β)-17-hydroxy-3-oxo-17-(pentafluoroethyl)estra-4,9-dien-11-yl]-N-methyl-N-[2-(pyridin-2-yl)ethyl]benzamide
N-benzyl-4-[(11β,17β)-17-hydroxy-3-oxo-17-(pentafluoroethyl)estra-4,9-dien-11-yl]-N-methylbenzamide
N-(4-acetamidophenyl)-4-[(11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N-(3-methoxybenzyl)benzamide
4-[((11β,17β)-17-hydroxy-3-oxo-17-(pentafluoroethyl)estra-4,9-dien-11-yl]-N-[2-(pyrrolidin-1-yl)ethyl]benzamide
4-[((11β,17β)-17-hydroxy-3-oxo-17-(pentafluoroethyl)estra-4,9-dien-11-yl]-N-[2-(pyridin-3-yl)ethyl]benzamide
N-(3-chlorobenzyl)-4-[(11β,17β)-17-hydroxy-3-oxo-17-(pentafluoroethyl)estra-4,9-dien-11-yl]benzamide
methyl-3-({4-[(11β,17β)-17-hydroxy-3-oxo-17-(pentafluoroethyl)estra-4,9-dien-11-yl]benzoyl}amino)benzoate
4-[((11β,17β)-17-hydroxy-3-oxo-17-(pentafluoroethyl)estra-4,9-dien-11-yl]-N-methyl-N-[2-(pyridin-4-yl)ethyl]benzamide
N-(cyclopropylmethyl)-4-[(11β,17β)-17-hydroxy-3-oxo-17-(pentafluoroethyl)estra-4,9-dien-11-yl]benzamide
4-[((11β,17β)-17-hydroxy-3-oxo-17-(pentafluoroethyl)estra-4,9-dien-11-yl]-N-[4-(phenylcarbamoyl)phenyl]benzamide
4-[((11β,17β)-17-hydroxy-3-oxo-17-(pentafluoroethyl)estra-4,9-dien-11-yl]-N-[2-(pyridin-2-yl)ethyl]benzamide
4-[((11β,17β)-17-hydroxy-3-oxo-17-(pentafluoroethyl)estra-4,9-dien-11-yl]-N-[2-(4-methylpiperazin-1-yl)ethyl]benzamide
(11β,17β)-17-hydroxy-17-(pentafluoroethyl)-11-{4-[(4-phenylpiperidin-1-yl)carbonyl]phenyl}estra-4,9-dien-3-one
4-[((11β,17β)-17-hydroxy-3-oxo-17-(pentafluoroethyl)estra-4,9-dien-11-yl]-N-[2-(pyridin-4-yl)ethyl]benzamide
(11β,17β)-11-{4-[(4-benzoylpiperazin-1-yl)carbonyl]phenyl}-17-hydroxy-17-(pentafluoroethyl)estra-4,9-dien-3-one
4-{4-[(11β,17β)-17-hydroxy-3-oxo-17-(pentafluoroethyl)estra-4,9-dien-11-yl]benzoyl}-N,N-dimethylpiperazine-1-carboxamide
(11 β,17β)-17-hydroxy-11-(4-{[4-(methylsulphonyl)piperazin-1-yl]carbonyl}phenyl)-17-(pentafluoroethyl)estra-4,9-dien-3-one
(11β,17β)-17-hydroxy-17-(pentafluoroethyl)-11-(4-{[4-(pyridin-2-ylmethyl)piperazin-1-yl]carbonyl}phenyl)estra-4,9-dien-3-one
(11β,17β)-17-hydroxy-17-(pentafluoroethyl)-11-(4-{[4-(pyridin-3-ylmethyl)piperazin-1-yl]carbonyl}phenyl)estra-4,9-dien-3-one
methyl-4-{4-[(11β,17β)-17-hydroxy-3-oxo-17-(pentafluoroethyl)estra-4,9-dien-11-yl]benzoyl}piperazine-1-carboxylate
2-((4-{4-[(11β,17β)-17-hydroxy-3-oxo-17-(pentafluoroethyl)estra-4,9-dien-11-yl]benzoyl}piperazin-1-yl)-N-methylacetamide
4-[((11β,17β)-17-hydroxy-3-oxo-17-(pentafluoroethyl)estra-4,9-dien-11-yl]-N-methyl-N-[2-(pyridin-3-yl)ethyl]benzamide
(11β,17β)-17-hydroxy-17-(pentafluoroethyl)-11-[4-(piperazin-1-ylcarbonyl)phenyl]estra-4,9-dien-3-one
(11β,17β)-11-{4-[(4-acetylpiperazin-1-yl)carbonyl]phenyl}-17-hydroxy-17-(pentafluoroethyl)estra-4,9-dien-3-one
1-{4-[(11β,17β)-17-hydroxy-3-oxo-17-(pentafluoroethyl)estra-4,9-dien-11-yl]benzoyl}piperidine-4-carboxylicacid
2-({4-[(11β,17β)-17-hydroxy-3-oxo-17-(pentafluoroethyl)estra-4,9-dien-11-yl]benzoyl}amino)benzoic acid
3-({4-[(11β,17β)-17-hydroxy-3-oxo-17-(pentafluoroethyl)estra-4,9-dien-11-yl]benzoyl}amino)benzoic acid
4-({4-[(11β,17β)-17-hydroxy-3-oxo-17-(pentafluoroethyl)estra-4,9-dien-11-yl]benzoyl}amino)benzoic acid
4'-[(11β,17β)-17-hydroxy-3-oxo-17-(pentafluoroethyl)estra-4,9-dien-11-yl]biphenyl-4-carboxamide
and their salts, solvates or solvates of the salts, including all crystal modifications.

8. Compound according to one of the preceding claims for the treatment and prevention of diseases.

9. Compound according to one of Claims 1-7 for treating and preventing uterine fibroids, endometriosis, heavy menstrual bleeding, meningiomata, hormone-dependent breast cancers and menopause-associated complaints or for fertility control and emergency contraception.

10. Use of a compound according to one of Claims 1-7 for producing a medicinal product for the treatment and/or prevention of diseases.

11. Use of a compound according to one of Claims 1-7 for producing a medicinal product for treating and/or preventing uterine fibroids, endometriosis, heavy menstrual bleeding, meningiomata, hormone-dependent breast cancers and menopause-associated complaints or for fertility control and emergency contraception.

12. Medicinal product containing a compound as defined in one of Claims 1-7, in combination with another active substance.

13. Medicinal product containing a compound as defined in one of Claims 1-7, in combination with an inert, non-toxic, pharmaceutically suitable excipient.

14. Medicinal product according to Claim 13 for the treatment and/or prevention of uterine fibroids, endometriosis, heavy menstrual bleeding, meningiomata, hormone-dependent breast cancers and menopause-associated complaints or for fertility control and emergency contraception.

## Revendications

1. Composés de formule I 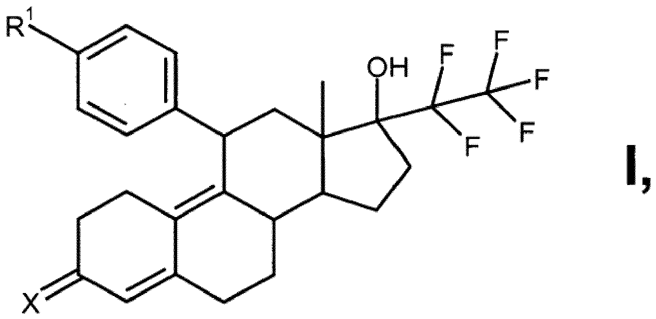 dans laquelle
R¹ représente un radical Y ou un cycle phényle substitué avec Y,
Y représente un groupe 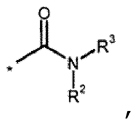
R² et R³ sont identiques ou différents et représentent l'hydrogène,
un radical alkyle en C₁-C₆ éventuellement substitué
par -N(CH₃)₂, 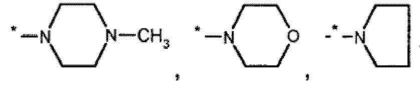 ou -NHC(O)CH₃, un radical 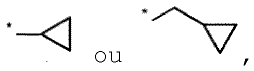 un radical aryle de 6 à 10 éléments éventuellement substitué une ou deux fois ou plus avec halogène (-F, - Cl, -Br, -I), -OH, -O-alkyle, -C(O)OH, -C(0)0-alkyle, - C(O)NH₂, -C(O)NH-alkyle, -C(O)N-dialkyle, -C(O)NH-aryle, -C(O)NH-hétéroaryle, -NH₂, -NH(alkyle en C₁-C₆), - N(alkyle en C₁-C₆)₂, notamment -N(CH₃)₂, -NHC(O)-alkyle, -NO₂, -N₃, -CN, -alkyle en C₁-C₆, -perfluoroalkyle en C₁-C₆, -acyle en C₁-C₆, -acyloxy en C₁-C₆, -SO₂NH₂, -SO₂NH-alkyle ou -SO₂N-dialkyle,
un radical hétéroaryle de 5 à 10 éléments éventuellement substitué une ou deux fois ou plus avec les substituants susmentionnés du radical aryle de 6 à 10 éléments,
un radical arylalkyle en C₁-C₆ éventuellement substitué une ou deux ou plus sur le cycle aryle avec les substituants susmentionnés du radical aryle de 6 à 10 éléments, ou
un radical hétéroarylalkyle en C₁-C₆ éventuellement substitué une ou deux ou plus sur le cycle hétéroaryle avec les substituants susmentionnés du radical aryle de 6 à 10 éléments, ou
R² et R³ font ensemble partie d'un cycle de 3 à 10 éléments éventuellement substitué sur le carbone par alkyle, carboxyle, alcoxycarbonyle, alkylcarbonyle, aminocarbonyle, aryle, notamment phényle, arylalkyle, hétéroaryle, hétéroarylalkyle, aminoalkyle ou diméthylaminoalkyle, ou sur l'azote par alkyle, alcanoyle, carboxyle, alcoxycarbonyle, aryle, notamment phényle, pyridinyle, pyrimidinyle, pyrazinyle, sulfonyle, benzoyle, alkylsulfonyle, arylsulfonyle, aminocarbonyle, diméthylaminocarbonyle, aminocarbonylalkyle, alkylaminocarbonylalkyle, arylalkyle, notamment phénylalkyle, hétérocyclylalkyle, hétéroarylalkyle, aminoalkyle ou 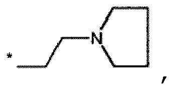 qui contient éventuellement des atomes d'azote, d'oxygène ou de soufre, qui peuvent éventuellement être oxydés en sulfoxyde ou sulfone, un aromatique pouvant éventuellement être condensé sur le cycle de 3 à 10 éléments,
X signifie un atome d'oxygène, NOR⁴ ou NNHSO₂R⁴, et
R⁴ est choisi dans le groupe comprenant hydrogène, alkyle en C₁-C₆ et aryle,
et leurs sels, solvates ou solvates des sels, y compris toutes leurs formes cristallines (* symbolise l'emplacement de liaison au corps de base).

2. Composé selon la revendication 1 de formule générale V : 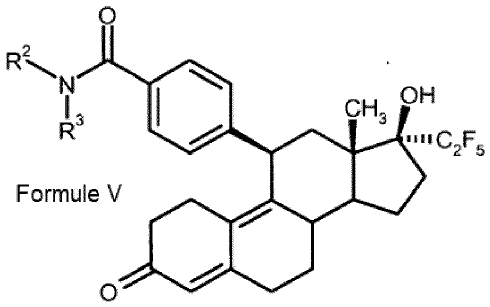 dans laquelle
R² signifie hydrogène, alkyle en C₁-C₄ (notamment méthyle, éthyle, cyclopropyle ou cyclopropylméthyle), - (CH₂)ₖ-N(CH₃)₂ avec k = 2 ou 3, -CH₂-CH₂-NH-CO-CH₃ et - (CH₂)ₖ-R⁵ avec k = 2 ou 3 et 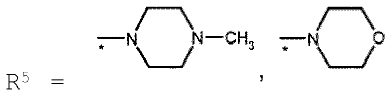 ou 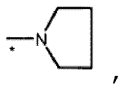 et
R³ signifie hydrogène ou alkyle en C₁-C₄ (notamment méthyle ou éthyle), et leurs sels, solvates ou solvates des sels, y compris toutes leurs formes cristallines.

3. Composé selon la revendication 1 de formule générale VI 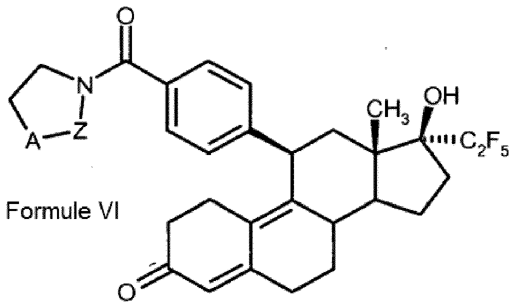 dans laquelle
Z représente -CH₂ ou -CH₂-CH₂,
A représente oxygène, -CHR⁶ ou -NR⁷- et
R⁶, R⁷ représentent
hydrogène,
alkyle en C₁-C₄,
un radical aryle - (CH₂)ₗ- avec l = 0, 1 ou 2, éventuellement substitué une ou deux fois ou plus avec halogène (-F, -Cl, -Br, -I), -OH, -O-alkyle, -CO₂H, - CO₂-alkyle, -C(O)NH₂, -C(O)NH-alkyle, -C(O)N-dialkyle, - C(O)NH-aryle, -C(O)NH-hétéroaryle, -NH₂, -NH(alkyle en C₁-C₆), -N (alkyle en C₁-C₆)₂, notamment -N(CH₃)₂, -NHC(O)-alkyle, -NO₂, -N₃, -CN, -alkyle en C₁-C₆, - perfluoroalkyle en C₁-C₆, -acyle en C₁-C₆, -acyloxy en C₁-C₆, -SO₂NH₂, -SO₂NH-alkyle ou -SO₂N-dialkyle,
un radical hétéroaryle -(CH₂)ₗ- contenant jusqu'à deux hétéroatomes avec l = 0, 1 ou 2, éventuellement substitué une ou deux fois ou plus avec halogène (-F, - Cl, -Br, -I), -OH, -O-alkyle, -CO₂H, -CO₂-alkyle, - C(O)NH₂, -C(O)NH-alkyle, -C(O)N-dialkyle, -C(O)NH-aryle, -C(O)NH-hétéroaryle, -NH₂, -NH (alkyle en C₁-C₆),-N(alkyle en C₁-C₆)₂, notamment -N(CH₃)₂, -NHC(O)-alkyle, -NO₂, -N₃, -CN, -alkyle en C₁-C₆, -perfluoroalkyle en C₁-C₆, -acyle en C₁-C₆, -acyloxy en C₁-C₆, -SO₂NH₂, -SO₂NH-alkyle ou -SO₂N-dialkyle, ou
-COR⁸ et
R⁸ signifie -OH, -(alkyle en C₁-C₄), -aryle, -O-alkyle en C₁-C₄, -NH-(alkyle en C₁-C₄), -N(CH₃)₂, -SO₂-(alkyle en C₁-C₄),
et leurs sels, solvates ou solvates des sels, y compris toutes leurs formes cristallines.

4. Composé selon la revendication 3, dans lequel
R⁶ signifie hydrogène, phényle, -CO₂H, -CO₂CH₃ et
R⁷ signifie hydrogène, -CH₃, -(CH₂)ₗ-phényle avec l = 0, 1 ou 2, 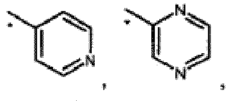 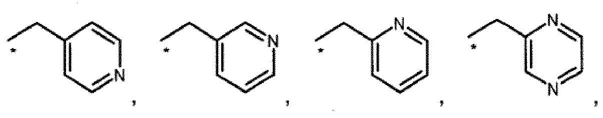 -CH₂-CH₂-N(CH₃)₂, 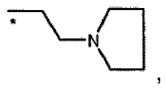 -CO-CH₃, -CO-phényle, -CO₂CH₃, - CO₂C(CH₃)₃, -CO-NH-CH₃, -CO-N(CH₃)₂, SO₂-CH₃, -CH₂CO-NH-CH₃, et leurs sels, solvates ou solvates des sels, y compris toutes leurs formes cristallines.

5. Composé selon la revendication 1 de formule générale VII : 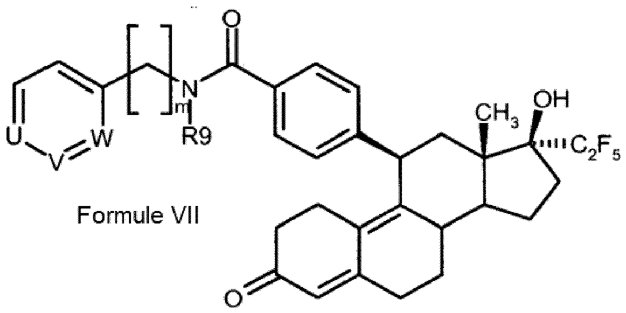 dans laquelle
m signifie 0, 1 ou 2,
R⁹ signifie hydrogène ou alkyle en C₁-C₄, notamment méthyle ou éthyle,
U, V et W signifient indépendamment les uns des autres -CH=, -CR¹⁰= ou -N=, -CR¹⁰= ou -N= apparaissant toutefois au plus une fois indépendamment de la position dans le cycle aromatique, et
R¹⁰ signifie -O-(alkyle en C₁-C₄), halogène, -COR¹¹ avec
R¹¹ = -OH, -NH₂ ou -O-(alkyle en C₁-C₄), -SO₂-NH₂ ; NH-CO-(alkyle en C₁-C₄) ou -CO-NH-aryle, et leurs sels, solvates ou solvates des sels, y compris toutes leurs formes cristallines.

6. Composé selon la revendication 5, dans lequel
R⁹ signifie hydrogène, méthyle ou éthyle, et
R¹⁰ signifie -O-CH₃, -Cl, -CO₂H, -CO₂CH₃, -CO-NH₂, -SO₂-NH₂ ; -NH-CO-CH₃ ou -CO-NH-phényle, et leurs sels, solvates ou solvates des sels, y compris toutes leurs formes cristallines.

7. Composés selon l'une quelconque des revendications précédentes, à savoir :
4-[((11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N,N-diméthylbenzamide
(11β,17β)-17-hydroxy-11-{4-[(4-méthylpipérazin-1-yl)carbonyl]phényl}-17-(pentafluoroéthyl)estra-4,9-dién-3-one
4-{4-[(11β,17β)-17-hydroxy-3-oxo-17-(pentafluoroéthyl)estra-4,9-dién-11-yl]benzoyl}pipérazine-1-carboxylate de tert-butyle
(11β,17β)-17-hydroxy-17-(pentafluoroéthyl)-11-[4-(pipéridin-1-ylcarbonyl)phényl]estra-4,9-dién-3-one
1-{4-[(11β,17β)-17-hydroxy-3-oxo-17-(pentafluoroéthyl)estra-4,9-dién-11-yl]benzoyl}pipéridine-4-carboxylate de méthyle
N-[2-(diméthylamino)éthyl]-4-[(11β,17β)-17-hydroxy-3-oxo-17-(pentafluoroéthyl)estra-4,9-dién-11-yl]benzamide
N-[3-(diméthylamino)propyl]-4-[(11β,17β)-17-hydroxy-3-oxo-17-(pentafluoroéthyl)estra-4,9-dién-11-yl]benzamide
N-[2-(diméthylamino)éthyl]-4-[(11β,17β)-17-hydroxy-3-oxo-17-(pentafluoroéthyl)estra-4,9-dién-11-yl]-N-méthylbenzamide
2-({4-[(11β,17β)-17-hydroxy-3-oxo-17-(pentafluoroéthyl)estra-4,9-dién-11-yl]benzoyl}amino)benzoate de méthyle
4-[((11β,17β)-17-hydroxy-3-oxo-17-(pentafluoroéthyl)estra-4,9-dién-11-yl]-N-(pyridin-2-yl)benzamide
4-[((11β,17β)-17-hydroxy-3-oxo-17-(pentafluoroéthyl)estra-4,9-dién-11-yl]-N-(pyridin-3-yl)benzamide
4-[((11β,17β)-17-hydroxy-3-oxo-17-(pentafluoroéthyl)estra-4,9-dién-11-yl]-N-[3-(morpholin-4-yl)propyl]benzamide
4-[((11β,17β)-17-hydroxy-3-oxo-17-(pentafluoroéthyl)estra-4,9-dién-11-yl]-N-(pyridin-4-yl)benzamide
(11β,17β)-17-hydroxy-17-(pentafluoroéthyl)-11-[4-(pyrrolidin-1-ylcarbonyl)phényl]estra-4,9-dién-3-one
4-[((11β,17β)-17-hydroxy-3-oxo-17-(pentafluoroéthyl)estra-4,9-dién-11-yl]-N-(2-phényléthyl)benzamide
4-[((11β,17β)-17-hydroxy-3-oxo-17-(pentafluoroéthyl)estra-4,9-dién-11-yl]-N-(2-phénylpropan-2-yl)benzamide
4-[((11β,17β)-17-hydroxy-3-oxo-17-(pentafluoroéthyl)estra-4,9-dién-11-yl]-N-(4-sulfamoylbenzyl)benzamide
N-éthyl-4-[(11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N-(pyridin-2-ylméthyl)benzamide
4-[((11β,17β)-17-hydroxy-3-oxo-17-(pentafluoroéthyl)estra-4,9-dién-11-yl]-N-(pyridin-4-ylméthyl)benzamide
4-[((11β,17β)-17-hydroxy-3-oxo-17-(pentafluoroéthyl)estra-4,9-dién-11-yl]-N-phénylbenzamide
4-[((11β,17β)-17-hydroxy-3-oxo-17-(pentafluoroéthyl)estra-4,9-dién-11-yl]-N-(2-méthoxyphényl)benzamide
4-[((11β,17β)-17-hydroxy-3-oxo-17-(pentafluoroéthyl)estra-4,9-dién-11-yl]-N-(3-méthoxyphényl)benzamide
4-[((11β,17β)-17-hydroxy-3-oxo-17-(pentafluoroéthyl)estra-4,9-dién-11-yl]-N-(4-méthoxyphényl)benzamide
N-(4-chlorophényl)-4-[(11β,17β)-17-hydroxy-3-oxo-17-(pentafluoroéthyl)estra-4,9-dién-11-yl]benzamide
N,N-diéthyl-4-[(11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N-méthyl-N-phénylbenzamide
(11β,17β)-17-hydroxy-11-[4-(morpholin-4-ylcarbonyl)phényl]-17-(pentafluoroéthyl)estra-4,9-dién-3-one
(11β,17β)-17-hydroxy-17-(pentafluoroéthyl)-11-{4-[(4-phénylpipérazin-1-yl)carbonyl]phényl}estra-4,9-dién-3-one
4-[((11β,17β)-17-hydroxy-3-oxo-17-(pentafluoroéthyl)estra-4,9-dién-11-yl]-N-méthyl-N-(pyridin-2-ylméthyl)benzamide
4-[((11β,17β)-17-hydroxy-3-oxo-17-(pentafluoroéthyl)estra-4,9-dién-11-yl]-N-méthyl-N-(pyridin-3-ylméthyl)benzamide
4-[((11β,17β)-17-hydroxy-3-oxo-17-(pentafluoroéthyl)estra-4,9-dién-11-yl]-N-méthyl-N-(pyridin-4-ylméthyl)benzamide
N-benzyl-4-[(11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N-(4-méthoxybenzyl)benzamide
N-(4-chlorobenzyl)-4-[(11β,17β)-17-hydroxy-3-oxo-17-(pentafluoroéthyl)estra-4,9-dién-11-yl]benzamide
N-(2-acétamidoéthyl)-4-[(11β,17β)-17-hydroxy-3-oxo-17-(pentafluoroéthyl)estra-4,9-dién-11-yl]benzamide
4-({4-[(11β,17β)-17-hydroxy-3-oxo-17-(pentafluoroéthyl)estra-4,9-dién-11-yl]benzoyl}amino)benzoate de méthyle
(11β,17β)-11-[4-(3,4-dihydroisoquinolin-2(1H)-ylcarbonyl)phényl]-17-hydroxy-17-(pentafluoroéthyl)estra-4,9-dién-3-one
(11β,17β)-11-{4-[(4-benzylpipérazin-1-yl)carbonyl]phényl}-17-hydroxy-17-(pentafluoroéthyl)estra-4,9-dién-3-one
N-(4-carbamoylphényl)-4-[(11β,17β)-17-hydroxy-3-oxo-17-(pentafluoroéthyl)estra-4,9-dién-11-yl]benzamide
N-cyclopropyl-4-[(11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N-méthyl-N-(2-phényléthyl)benzamide
(11β,17β)-17-hydroxy-17-(pentafluoroéthyl)-11-(4-{[4-(2-phényléthyl)pipérazin-1-yl]carbonyl}phényl)estra-4,9-dién-3-one
(11β,17β)-17-hydroxy-17-(pentafluoroéthyl)-11-(4-{[4-(pyridin-4-yl)pipérazin-1-yl]carbonyl}phényl)estra-4,9-dién-3-one
(11β,17β)-17-hydroxy-17-(pentafluoroéthyl)-11-(4-{[4-(pyrazin-2-yl)pipérazin-1-yl]carbonyl}phényl)estra-4,9-dién-3-one
(11β,17β)-11-[4-({4-[2-(diméthylamino)éthyl]pipérazin-1-yl}carbonyl)phényl]-17-hydroxy-17-(pentafluoroéthyl)estra-4,9-dién-3-one
(11β,17β)-17-hydroxy-17-(pentafluoroéthyl)-11-[4-({4-[2-(pyrrolidin-1-yl)éthyl]pipérazin-1-yl}carbonyl)phényl]estra-4,9-dién-3-one
(11β,17β)-17-hydroxy-17-(pentafluoroéthyl)-11-(4-{[4-(pyridin-4-ylméthyl)pipérazin-1-yl]carbonyl}phényl)estra-4,9-dién-3-one
4-[((11β,17β)-17-hydroxy-3-oxo-17-(pentafluoroéthyl)estra-4,9-dién-11-yl]-N-méthyl-N-[2-(pyridin-2-yl)éthyl]benzamide
N-benzyl-4-[(11β,17β)-17-hydroxy-3-oxo-17-(pentafluoroéthyl)estra-4,9-dién-11-yl]-N-méthylbenzamide
N-(4-acétamidophényl)-4-[(11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N-(3-méthoxybenzyl)benzamide
4-[((11β,17β)-17-hydroxy-3-oxo-17-(pentafluoroéthyl)estra-4,9-dién-11-yl]-N-[2-(pyrrolidin-1-yl)éthyl]benzamide
4-[((11β,17β)-17-hydroxy-3-oxo-17-(pentafluoroéthyl)estra-4,9-dién-11-yl]-N-[2-(pyridin-3-yl)éthyl]benzamide
N-(3-chlorobenzyl)-4-[(11β,17β)-17-hydroxy-3-oxo-17-(pentafluoroéthyl)estra-4,9-dién-11-yl]benzamide
3-({4-[(11β,17β)-17-hydroxy-3-oxo-17-(pentafluoroéthyl)estra-4,9-dién-11-yl]benzoyl}amino)benzoate de méthyle
4-[((11β,17β)-17-hydroxy-3-oxo-17-(pentafluoroéthyl)estra-4,9-dién-11-yl]-N-méthyl-N-[2-(pyridin-4-yl)éthyl]benzamide
N-(cyclopropylméthyl)-4-[(11β,17β)-17-hydroxy-3-oxo-17-(pentafluoroéthyl)estra-4,9-dién-11-yl]benzamide
4-[((11β,17β)-17-hydroxy-3-oxo-17-(pentafluoroéthyl)estra-4,9-dién-11-yl]-N-[4-(phénylcarbamoyl)phényl]benzamide
4-[((11β,17β)-17-hydroxy-3-oxo-17-(pentafluoroéthyl)estra-4,9-dién-11-yl]-N-[2-(pyridin-2-yl)éthyl]benzamide
4-[((11β,17β)-17-hydroxy-3-oxo-17-(pentafluoroéthyl)estra-4,9-dién-11-yl]-N-[2-(4-méthylpipérazin-1-yl)éthyl]benzamide
(11β,17β)-17-hydroxy-17-(pentafluoroéthyl)-11-{4-[(4-phénylpipéridin-1-yl)carbonyl]phényl}estra-4,9-dién-3-one
4-[((11β,17β)-17-hydroxy-3-oxo-17-(pentafluoroéthyl)estra-4,9-dién-11-yl]-N-[2-(pyridin-4-yl)éthyl]benzamide
(11β,17β)-11-{4-[(4-benzoylpipérazin-1-yl)carbonyl]phényl}-17-hydroxy-17-(pentafluoroéthyl)estra-4,9-dién-3-one
4-{4-[(11β,17β)-17-hydroxy-3-oxo-17-(pentafluoroéthyl)estra-4,9-dién-11-yl]benzoyl}-N,N-diméthylpipérazine-1-carboxamide
(11β,17β)-17-hydroxy-11-(4-{[4-(méthylsulfonyl)pipérazin-1-yl]carbonyl}phényl)-17-(pentafluoroéthyl)estra-4,9-dién-3-one
(11β,17β)-17-hydroxy-17-(pentafluoroéthyl)-11-(4-{[4-(pyridin-2-ylméthyl)pipérazin-1-yl]carbonyl}phényl)estra-4,9-dién-3-one
(11β,17β)-17-hydroxy-17-(pentafluoroéthyl)-11-(4-{[4-(pyridin-3-ylméthyl)pipérazin-1-yl]carbonyl}phényl)estra-4,9-dién-3-one
4-{4-[(11β,17β)-17-hydroxy-3-oxo-17-(pentafluoroéthyl)estra-4,9-dién-11-yl]benzoyl}pipérazine-1-carboxylate de méthyle
2-((4-{4-[(11β,17β)-17-hydroxy-3-oxo-17-(pentafluoroéthyl)estra-4,9-dién-11-yl]benzoyl}pipérazin-1-yl)-N-méthylacétamide
4-[((11β,17β)-17-hydroxy-3-oxo-17-(pentafluoroéthyl)estra-4,9-dién-11-yl]-N-méthyl-N-[2-(pyridin-3-yl)éthyl]benzamide
(11β,17β)-17-hydroxy-17-(pentafluoroéthyl)-11-[4-(pipérazin-1-ylcarbonyl)phényl]estra-4,9-dién-3-one
(11β,17β)-11-{4-[(4-acétylpipérazin-1-yl)carbonyl]phényl}-17-hydroxy-17-(pentafluoroéthyl)estra-4,9-dién-3-one
acide 1-{4-[(11β,17β)-17-hydroxy-3-oxo-17-(pentafluoroéthyl)estra-4,9-dién-11-yl]benzoyl}pipéridine-4-carboxylique
acide 2-({4-[(11β,17β)-17-hydroxy-3-oxo-17-(pentafluoroéthyl)estra-4,9-dién-11-yl]benzoyl}amino)benzoïque
acide 3-({4-[(11β,17β)-17-hydroxy-3-oxo-17-(pentafluoroéthyl)estra-4,9-dién-11-yl]benzoyl}amino)benzoïque
acide 4-({4-[(11β,17β)-17-hydroxy-3-oxo-17-(pentafluoroéthyl)estra-4,9-dién-11-yl]benzoyl}amino)benzoïque
4'-[(11β,17β)-17-hydroxy-3-oxo-17-(pentafluoroéthyl)estra-4,9-dién-11-yl]biphényl-4-carboxamide,
et leurs sels, solvates ou solvates des sels, y compris toutes leurs formes cristallines.

8. Composés selon l'une quelconque des revendications précédentes, pour le traitement et la prophylaxie de maladies.

9. Composés selon l'une quelconque des revendications 1 à 7, pour le traitement et la prophylaxie des fibromes utérins, de l'endométriose, des saignements menstruels abondants, des méningiomes, des cancers du sein hormonodépendants et des troubles associés avec la ménopause, ou pour le contrôle de la fertilité et la contraception d'urgence.

10. Utilisation d'un composé selon l'une quelconque des revendications 1 à 7 pour la fabrication d'un médicament pour le traitement et/ou la prophylaxie de maladies.

11. Utilisation d'un composé selon l'une quelconque des revendications 1 à 7 pour la fabrication d'un médicament pour le traitement et/ou la prophylaxie des fibromes utérins, de l'endométriose, des saignements menstruels abondants, des méningiomes, des cancers du sein hormonodépendants et des troubles associés avec la ménopause, ou pour le contrôle de la fertilité et la contraception d'urgence.

12. Médicament contenant un composé tel que défini dans l'une quelconque des revendications 1 à 7, en combinaison avec un agent actif supplémentaire.

13. Médicament contenant un composé tel que défini dans l'une quelconque des revendications 1 à 7, en combinaison avec un adjuvant inerte, non toxique, pharmaceutiquement approprié.

14. Médicament selon la revendication 13, pour le traitement et/ou la prophylaxie des fibromes utérins, de l'endométriose, des saignements menstruels abondants, des méningiomes, des cancers du sein hormonodépendants et des troubles associés avec la ménopause, ou pour le contrôle de la fertilité et la contraception d'urgence.
